# EUROPEAN PATENT SPECIFICATION

(11) **EP 4 471 002 B1**
(45) Date of publication and mention of the grant of the patent: **17.12.2025**
(21) Application number: 24178395.0
(22) Date of filing: 28.05.2024
(51) Int. Cl.: C07C 17/16, C07C 29/10, C07C 41/22, C07C 41/30, C07C 43/12, C07C 41/26, C07C 1/32

(54) **HALOALKYL ALKOXYMETHYL ETHER COMPOUND, AND PROCESS FOR PREPARING 4,6,8,10,16-PENTAMETHYLDOCOSANE THEREFROM AND FOR PREPARING SYNTHETIC INTERMEDIATE THEREFOR**
HALOALKYLALKOXYMETHYLETHERVERBINDUNG UND VERFAHREN ZUR HERSTELLUNG VON 4,6,8,10,16-PENTAMETHYLDOCOSAN DARAUS UND ZUR HERSTELLUNG EINES SYNTHETISCHEN ZWISCHENPRODUKTS DAFÜR
COMPOSÉ HALOALKYL ALCOXYMÉTHYL ÉTHER ET PROCÉDÉ DE PRÉPARATION DE 4,6,8,10,16-PENTAMÉTHYLDOCOSANE À PARTIR DE CELUI-CI ET DE PRÉPARATION D'UN INTERMÉDIAIRE SYNTHÉTIQUE POUR CELUI-CI

(30) Priority: 31.05.2023 JP 2023090373
(43) Date of publication of application: 04.12.2024
(73) Proprietor: Shin-Etsu Chemical Co., Ltd., Tokyo 100-0005 (JP)
(72) Inventor: MIYAKE, Yuki, Niigata, 942-8601 (JP); WATANABE, Takeru, Niigata, 942-8601 (JP); KINSHO, Takeshi, Niigata, 942-8601 (JP); KOMATSU, Ryo, Niigata, 942-8601 (JP)
(74) Representative: De Vries & Metman

(56) References cited:
- WILLIAM KITCHING ET AL., J. ORG. CHEM., vol. 70, 2005, pages 1808 - 1827, XP002812448

## Description

### TECHNICAL FIELD

The present invention relates to a haloalkyl alkoxymethyl ether compound, and also relates to a process for preparing 4,6,8,10,16-pentamethyldocosane from the haloalkyl alkoxymethyl ether compound and for preparing synthetic intermediate for preparing the haloalkyl alkoxymethyl ether compound.

### BACKGROUND ART

Compounds having a 1,3-dimethyl skeleton are widely known, such as the cuticular hydrocarbon of *Antitragus parvulus,* 4,6,8,10,16-pentamethyldocosane, and sex pheromones of *Margarodes prieskaensis,* 2,4,6,8-tetramethylundecanol, 2,4,6,8,10-pentamethyltridecane, and 2,4,6,8,10,12,14,16-octamethylheptadecane. In particular, *Antitragus parvulus* is a sugarcane pest of Australia that has been controlled with the widespread use of organophosphorus pesticides, causing problems due to residual agricultural chemicals. Therefore, efforts have been focused on biological control methods that minimize use of pesticides, with high expectations for the use of cuticular hydrocarbons (Non-Patent Literature 1 below).

Various compounds are known as cuticular hydrocarbons of *Antitragus parvulus,* such as 4,6,8,10,16-pentamethyldocosane and 4,6,8,10,16,18-hexamethyldocosane (Non-Patent Literature 1 below).

Processes for synthesizing 4,6,8,10,16-pentamethyldocosane include, for example, a process reported to have a yield of 3.28% for a total of 19 steps (Non-Patent Literature 1 below). For example, 2,4,6-trimethylphenol is subjected to a hydrogenation reaction to obtain 2,4,6-trimethylcyclohexanol. 2,4,6-Trimethylcyclohexanol thus obtained is subjected to a Jones oxidation reaction, then subjected to a Baeyer-Villiger oxidation reaction with 3-chloroperoxybenzoic acid (mCPBA), and then subjected to a ring-opening reaction with sodium methoxide to obtain methyl 6-hydroxy-2,4-dimethylheptanoate. The hydroxy group of methyl 6-hydroxy-2,4-dimethylheptanoate thus obtained is protected with a tetrahydropyranyl group by using 3,4-dihydro-2*H*-pyran, and then the ester of methyl 6-hydroxy-2,4-dimethylheptanoate is subjected to a reduction reaction with lithium aluminum hydride to obtain 2,4-dimethyl-6-[(tetrahydro-2*H*-pyran-2-yl)oxy]-1-heptanol. 2,4-Dimethyl-6-[(tetrahydro-2*H*-pyran-2-yl)oxy]-1-heptanol thus obtained is subjected to an oxidation reaction with pyridinium chlorochromate (PCC) in a dichloromethane solvent, and then subjected to a Wittig reaction with ethyl 2-(triphenylphosphoranylidene)propanoate. The carbon-carbon double bond is then subjected to a hydrogenation reaction with palladium on carbon, and the ester is reduced with lithium aluminum hydride, followed by iodinating the hydroxy group with triphenylphosphine, iodine, and imidazole. The reaction mixture is then subjected to a coupling reaction with ethylmagnesium bromide, followed by eliminating the tetrahydropyranyl group with methanol in an acidic condition to obtain 4,6,8-trimethyl-2-undecanol. The hydroxy group of 4,6,8-trimethyl-2-undecanol is then mesylated with triethylamine and methanesulfonyl chloride, followed by reacting with sodium cyanide in dimethyl sulfoxide (DMSO) to obtain 2,4,6,8-tetramethylundecanedecanenitrile. 2,4,6,8-Tetramethylundecanedecanenitrile is then reacted with acetyl chloride in methanol, followed by converting the nitrile group into a carboxylic acid. The reaction mixture is then subjected to a reduction reaction with lithium aluminum hydride to obtain 2,4,6,8-tetramethyl-1-undecanol. The hydroxy group of 2,4,6,8-tetramethyl-1-undecanol is then subjected to Swern oxidation with triethylamine and oxalyl chloride in DMSO to obtain 2,4,6,8-tetramethyl-1-undecanal. 2,4,6,8-Tetramethyl-1-undecanal is then subjected to a Wittig reaction with (5-methylundecylidene)triphenylphosphorane, and the carbon-carbon double bond is reduced in the presence of a palladium on carbon catalyst to obtain 4,6,8,10,16-pentamethyldocosane.

### PRIOR ART

### [Non-Patent Literatures]

[Non-Patent Literature 1] William Kitching et al., J. Org. Chem., 2005, 70, 1808-1827.

### OBJECT OF THE INVENTION

As mentioned above, various compounds, such as 4,6,8,10,16-pentamethyldocosane and 4,6,8,10,16,18-hexamethyldocosane, are known as cuticular hydrocarbons of *Antitragus parvulus.* Although the present disclosure focuses on 4,6,8,10,16-pentamethyldocosane, the present invention is not limited thereto. The process for preparing 4,6,8,10,16-pentamethyldocosane according to Non-Patent Literature 1 is unsuitable for industrially practical preparation due to the danger of explosion in multiple steps that use Jones oxidation, Baeyer-Villiger oxidation, PCC oxidation, and Swern oxidation reactions, the danger of fire in multiple steps that use lithium aluminum hydride and palladium on carbon, and the use of extremely toxic sodium cyanide. The high number of steps and low yield also are unsuitable for industrially practical preparation. Moreover, the preparation process according to Non-Patent Literature 1 can prepare the cuticular hydrocarbons of *Antitragus parvulus,* 4,6,8,10,16-pentamethyldocosane and 4,6,8,10,16,18-hexamethyldocosane, but cannot comprehensively synthesize other various compounds having 1,3-dimethyl skeletons.

The present invention has been made in view of the aforementioned circumstances, and aims to provide a comprehensive process for preparing compounds having 1,3-dimethyl skeletons. The present invention also aims to provide a novel synthetic intermediate that is useful for preparing compounds having 1,3-dimethyl skeletons, and particularly 4,6,8,10,16-pentamethyldocosane, and a process for preparing the synthetic intermediate. The present invention also aims to provide an efficient process for preparing 4,6,8,10,16-pentamethyldocosane from the aforesaid synthetic intermediate, and preferably a preparation process that is efficient and economic.

### SUMMARY OF THE INVENTION

As a result of intensive research to overcome the aforesaid problems of the prior art, the present inventors found that a haloalkyl alkoxymethyl ether compound is a key intermediate from which various compounds having 1,3-dimethyl skeletons can be comprehensively synthesized. The present inventors further found an inexpensive and efficient process for preparing the haloalkyl alkoxymethyl ether compound, and a process for preparing the cuticular hydrocarbon compound of *Antitragus parvulus, 4,6,8,10,16-*pentamethyldocosane, from the haloalkyl alkoxymethyl ether compound in fewer steps, and thus have completed the present invention. The aforesaid preparation process also was found to be suited to industrially practical preparation.

According to a first aspect of the present invention, there is provided a process for preparing a haloalkyl alkoxymethyl ether compound of the following general formula (1'): 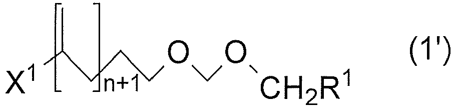 wherein X¹ represents a halogen atom, R¹ represents a hydrogen atom, an n-alkyl group having 1 to 9 carbon atoms, or a phenyl group, and *n* represents an integer of 2 to 7,
the process comprising the steps of
   converting a haloalkyl alkoxymethyl ether compound of the following general formula (1): 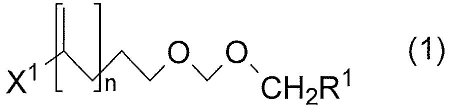
wherein X¹, R¹, and *n* are as defined above,
into a nucleophilic reagent, (2*n*+2)-alkoxymethoxyalkyl compound, of the following general formula (2): 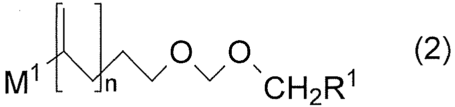
wherein M¹ represents Li, MgZ¹, CuZ¹, or CuLiZ¹, Z¹ represents a halogen atom or Z², and Z² represents: 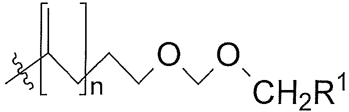
wherein R¹ and *n* are as defined above, and the wavy line indicates that the structures therebeyond are abbreviated,
subsequently subjecting the nucleophilic reagent, (2*n*+2)-alkoxymethoxyalkyl compound (2), to a nucleophilic addition reaction with propylene oxide of the following formula (3): 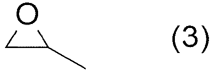
to obtain a hydroxyalkyl alkoxymethyl ether compound of the following general formula (4): 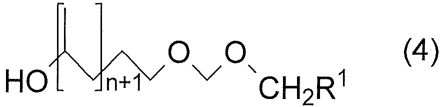
wherein R¹ and *n* are as defined above;
   and subjecting the hydroxyalkyl alkoxymethyl ether compound (4) to a halogenation reaction to obtain the aforesaid haloalkyl alkoxymethyl ether compound (1').

According to a second aspect of the present invention, there is provided a process for preparing a haloalkyl alkoxymethyl ether compound (1': *n+1* = 3) according to the aforesaid first aspect, wherein *n* represents 2 in the haloalkyl alkoxymethyl ether compounds (1) and (1').

According to a third aspect of the present invention, there is provided a process for preparing 4,6,8,10,16-pentamethyldocosane of the following general formula (11): 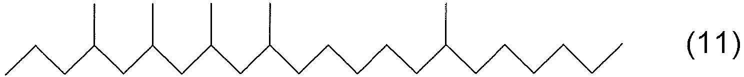
the process comprising the steps of
   a process for preparing a haloalkyl alkoxymethyl ether compound (1': *n+*1 = 3) such as, for example, the aforesaid process for preparing the haloalkyl alkoxymethyl ether compound (1': *n+*1 = 3) according to the first aspect, or another preparation process such as, for example, a process for preparing a haloalkyl alkoxymethyl ether compound (1': *n+*1 = 3) according to any one of Examples 5 and 6 of the present specification;
   converting the haloalkyl alkoxymethyl ether compound (1': *n+*1 = 3) into a nucleophilic reagent, 8-alkoxymethoxy-1,3,5-trimethyloctyl compound, of the following general formula (2: *n =* 3): 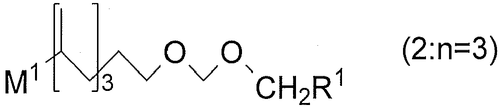
wherein M¹ represents Li, MgZ¹, CuZ¹, or CuLiZ¹, Z¹ represents a halogen atom or a 8-alkoxymethoxy-1,3,5-trimethyloctyl group, and R¹ is as defined above,
then subjecting the nucleophilic reagent, 8-alkoxymethoxy-1,3,5-trimethyloctyl compound (2: *n =* 3), to a coupling reaction with a 1-halo-2-methylpentane compound of the following general formula (5): 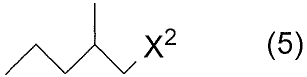
wherein X² represents a halogen atom,
to obtain a 4,6,8,10-tetramethyltridecyl alkoxymethyl ether compound of the following general formula (6): 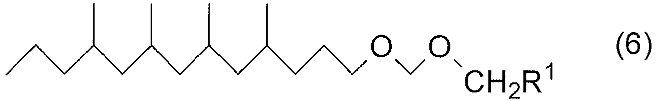
wherein R¹ is as defined above;
   subjecting the 4,6,8,10-tetramethyltridecyl alkoxymethyl ether compound (6) to a dealkoxymethylation reaction to obtain 4,6,8,10-tetramethyltridecanol of the following formula (7): 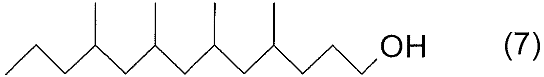
   subjecting the 4,6,8,10-tetramethyltridecanol (7) to a halogenation reaction to obtain a 1-halo-4,6,8,10-tetramethyltridecane compound of the following general formula (8): 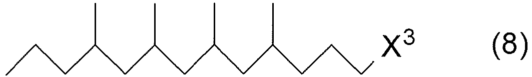
wherein X³ represents a halogen atom,
   converting the 1-halo-4,6,8,10-tetramethyltridecane compound (8) into a nucleophilic reagent, 4,6,8,10-tetramethyltridecyl compound, of the following general formula (9): 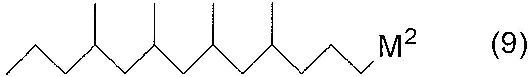
wherein M² represents Li, MgZ¹, CuZ¹, or CuLiZ¹, Z¹ represents a halogen atom or a 4,6,8,10-tetramethyltridecyl group,
and then subjecting the nucleophilic reagent, 4,6,8,10-tetramethyltridecyl compound (9), to a coupling reaction with a 1-halo-3-methylnonane compound of the following general formula (10): 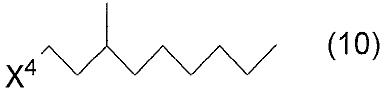
wherein X⁴ represents a halogen atom,
to obtain the aforesaid 4,6,8,10,16-pentamethyldocosane (11).

According to a fourth aspect of the present invention, there is provided a process for preparing 4,6,8,10,16-pentamethyldocosane of the following general formula (11): 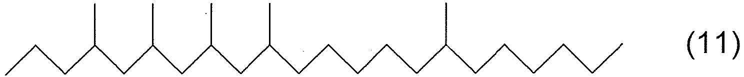
the process comprising the steps of
   converting a haloalkyl alkoxymethyl ether compound of the following general formula (1: *n =* 3): 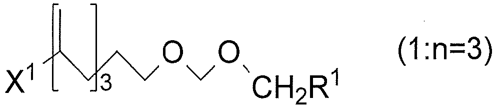
wherein X¹ represents a halogen atom, and R¹ represents a hydrogen atom, an n-alkyl group having 1 to 9 carbon atoms, or a phenyl group,
into a nucleophilic reagent, 8-alkoxymethoxy-1,3,5-trimethyloctyl compound, of the following general formula (2: *n =* 3): 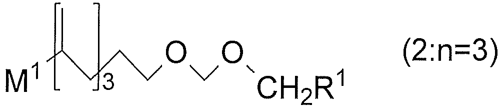
wherein M¹ represents Li, MgZ¹, CuZ¹, or CuLiZ¹, Z¹ represents a halogen atom or a 8-alkoxymethoxy-1,3,5-trimethyloctyl group, and R¹ is as defined above,
   subsequently subjecting the nucleophilic reagent, 8-alkoxymethoxy-1,3,5-trimethyloctyl compound (2: *n* = 3), to a coupling reaction with a 1-halo-2-methylpentane compound of the following general formula (5): 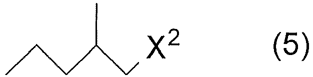
wherein X² represents a halogen atom,
to obtain a 4,6,8,10-tetramethyltridecyl alkoxymethyl ether compound of the following general formula (6): 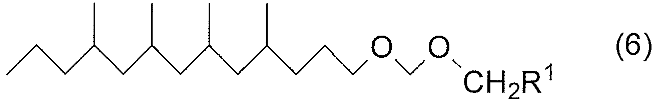
wherein R¹ is as defined above;
   subjecting the 4,6,8,10-tetramethyltridecyl alkoxymethyl ether compound (6) to a dealkoxymethylation reaction to obtain 4,6,8,10-tetramethyltridecanol of the following formula (7): 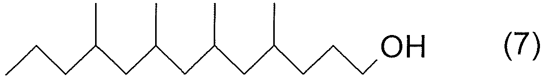 subjecting the 4,6,8,10-tetramethyltridecanol (7) to a halogenation reaction to obtain a 1-halo-4,6,8,10-tetramethyltridecane compound of the following general formula (8): 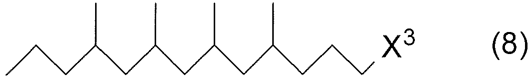
wherein X³ represents a halogen atom;
   converting the aforesaid 1-halo-4,6,8,10-tetramethyltridecane compound (8) into a nucleophilic reagent, 4,6,8,10-tetramethyltridecyl compound, of the following general formula (9): 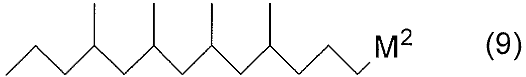
wherein M² represents Li, MgZ¹, CuZ¹, or CuLiZ¹, and Z¹ represents a halogen atom or a 4,6,8,10-tetramethyltridecyl group,
   and subsequently subjecting the nucleophilic reagent, 4,6,8,10-tetramethyltridecyl compound (9), to a coupling reaction with a 1-halo-3-methylnonane compound of the following general formula (10): 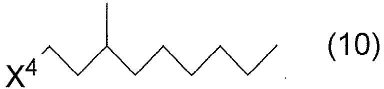
wherein X⁴ represents a halogen atom,
to form the aforesaid 4,6,8,10,16-pentamethyldocosane (11).

According to a fifth aspect of the present invention, there is provided a haloalkyl alkoxymethyl ether compound of the following general formula (1'): 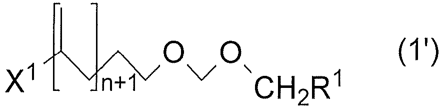 wherein X¹ represents a halogen atom, R¹ represents a hydrogen atom, an n-alkyl group having 1 to 9 carbon atoms, or a phenyl group, and *n* represents an integer of 2 to 7.

According to the present invention, it is possible to prepare a haloalkyl alkoxymethyl ether compound that is a building block for comprehensively synthesizing compounds having 1,3-dimethyl skeletons. The haloalkyl alkoxymethyl ether compound can be used as a key intermediate to efficiently prepare 4,6,8,10,16-pentamethyldocosane, a compound having a 1,3-dimethyl skeleton, with less environmental impact and in fewer steps. The preparation process is also economic.

### DETAILED DESCRIPTION OF THE INVENTION

### I. Haloalkyl alkoxymethyl ether compound of the following general formulas (1) and (1')

First, the haloalkyl alkoxymethyl ether compounds (1) and (1') will be explained in detail below. 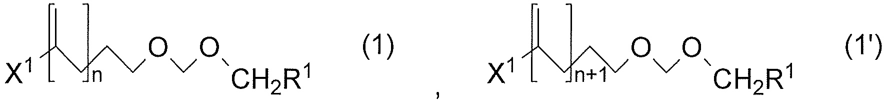 In each of the general formulas (1) and (1') above, X¹ represents a halogen atom. Examples of the halogen atom X¹ include a chlorine atom, a bromine atom, and an iodine atom. A chlorine atom and a bromine atom are preferred. By using said chlorine atom and bromine atom, a preferred reactivity may be ensured. A chlorine atom is particularly preferred. By using said chlorine atom, a particularly preferred reactivity may be ensured.

In each of the general formulas (1) and (1') above, R¹ represents a hydrogen atom, an n-alkyl group having 1 to 9 carbon atoms, and preferably 1 to 4 carbon atoms, or a phenyl group.

Examples of the n-alkyl group include a methyl group, an ethyl group, a propyl group, a butyl group, a pentyl group, a hexyl group, a heptyl group, an octyl group, and a nonyl group.

*n* represents an integer of 1 to 7. Depending on the value of *n* from 1 to 7, the compound (1) may be the compound (1: *n =* 1), the compound (1: *n* = 2), the compound (1: *n =* 3), the compound (1: *n* = 4), the compound (1: *n =* 5), the compound (1: *n =* 6), and the compound (1: *n* = 7) (hereinafter, the cases where *n* is 1 to 7 are collectively referred to as the compound (1: *n* = 1 to 7)). Similarly, depending on the value of *n* from 1 to 7, the compound (1') may be the compound (1': *n+*1 = 2), the compound (1': *n+*1 = 3), the compound (1': *n+*1 = 4), the compound (1': *n+*1 = 5), the compound (1': *n+*1 = 6), the compound (1': *n+*1 = 7), and the compound (1': *n+*1 = 8) (hereinafter, the cases where *n* is 1 to 7 are collectively referred to as the compound (1': *n+*1 = 2 to 8)). The present specification focuses on a haloalkyl alkoxymethyl ether compound wherein *n* is an integer of 2 to 7, and preferably 2 to 6, as the target compound of the present invention. Accordingly, the compound (1) wherein *n* is 2 to 7 is collectively referred to as the compound (1: *n* = 2 to 7), and the compound (1') wherein *n* is 2 to 7 is collectively referred to as the compound (1': *n+*1 = 3 to 8).

Specific examples of the haloalkyl alkoxymethyl ether compounds (1) and (1') include the following compounds:
4-halopentyl alkoxymethyl ether compounds (1: *n* = 1) such as 4-chloropentyl methoxymethyl ether, 4-bromopentyl methoxymethyl ether, 4-iodopentyl methoxymethyl ether, 4-chloropentyl ethoxymethyl ether, 4-bromopentyl ethoxymethyl ether, 4-iodopentyl ethoxymethyl ether, 4-chloropentyl propyloxymethyl ether, 4-bromopentyl propyloxymethyl ether, 4-iodopentyl propyloxymethyl ether, 4-chloropentyl butyloxymethyl ether, 4-bromopentyl butyloxymethyl ether, 4-iodopentyl butyloxymethyl ether, 4-chloropentyl pentyloxymethyl ether, 4-bromopentyl pentyloxymethyl ether, 4-iodopentyl pentyloxymethyl ether, 4-chloropentyl hexyloxymethyl ether, 4-bromopentyl hexyloxymethyl ether, 4-iodopentyl hexyloxymethyl ether, 4-chloropentyl heptyloxymethyl ether, 4-bromopentyl heptyloxymethyl ether, 4-iodopentyl heptyloxymethyl ether, 4-chloropentyl octyloxymethyl ether, 4-bromopentyl octyloxymethyl ether, 4-iodopentyl octyloxymethyl ether, 4-chloropentyl nonyloxymethyl ether, 4-bromopentyl nonyloxymethyl ether, 4-iodopentyl nonyloxymethyl ether, 4-chloropentyl decyloxymethyl ether, 4-bromopentyl decyloxymethyl ether, 4-iodopentyl decyloxymethyl ether, 4-chloropentyl benzyloxymethyl ether, 4-bromopentyl benzyloxymethyl ether, and 4-iodopentyl benzyloxymethyl ether;
6-halo-4-methylheptyl alkoxymethyl ether compounds ((1: *n* = 2) or (1': *n*+1 = 2)) such as 6-chloro-4-methylheptyl methoxymethyl ether, 6-bromo-4-methylheptyl methoxymethyl ether, 6-iodo-4-methylheptyl methoxymethyl ether, 6-chloro-4-methylheptyl ethoxymethyl ether, 6-bromo-4-methylheptyl ethoxymethyl ether, 6-iodo-4-methylheptyl ethoxymethyl ether, 6-chloro-4-methylheptyl propyloxymethyl ether, 6-bromo-4-methylheptyl propyloxymethyl ether, 6-iodo-4-methylheptyl propyloxymethyl ether, 6-chloro-4-methylheptyl butyloxymethyl ether, 6-bromo-4-methylheptyl butyloxymethyl ether, 6-iodo-4-methylheptyl butyloxymethyl ether, 6-chloro-4-methylheptyl pentyloxymethyl ether, 6-bromo-4-methylheptyl pentyloxymethyl ether, 6-iodo-4-methylheptyl pentyloxymethyl ether, 6-chloro-4-methylheptyl hexyloxymethyl ether, 6-bromo-4-methylheptyl hexyloxymethyl ether, 6-iodo-4-methylheptyl hexyloxymethyl ether, 6-chloro-4-methylheptyl heptyloxymethyl ether, 6-bromo-4-methylheptyl heptyloxymethyl ether, 6-iodo-4-methylheptyl heptyloxymethyl ether, 6-chloro-4-methylheptyl octyloxymethyl ether, 6-bromo-4-methylheptyl octyloxymethyl ether, 6-iodo-4-methylheptyl octyloxymethyl ether, 6-chloro-4-methylheptyl nonyloxymethyl ether, 6-bromo-4-methylheptyl nonyloxymethyl ether, 6-iodo-4-methylheptyl nonyloxymethyl ether, 6-chloro-4-methylheptyl decyloxymethyl ether, 6-bromo-4-methylheptyl decyloxymethyl ether, 6-iodo-4-methylheptyl decyloxymethyl ether, 6-chloro-4-methylheptyl benzyloxymethyl ether, 6-bromo-4-methylheptyl benzyloxymethyl ether, and 6-iodo-4-methylheptyl benzyloxymethyl ether;
8-halo-4,6-dimethylnonyl alkoxymethyl ether compounds ((1: *n =* 3) or (1': *n+*1 = 3)) such as 8-chloro-4,6-dimethylnonyl methoxymethyl ether, 8-bromo-4,6-dimethylnonyl methoxymethyl ether, 8-iodo-4,6-dimethylnonyl methoxymethyl ether, 8-chloro-4,6-dimethylnonyl ethoxymethyl ether, 8-bromo-4,6-dimethylnonyl ethoxymethyl ether, 8-iodo-4,6-dimethylnonyl ethoxymethyl ether, 8-chloro-4,6-dimethylnonyl propyloxymethyl ether, 8-bromo-4,6-dimethylnonyl propyloxymethyl ether, 8-iodo-4,6-dimethylnonyl propyloxymethyl ether, 8-chloro-4,6-dimethylnonyl butyloxymethyl ether, 8-bromo-4,6-dimethylnonyl butyloxymethyl ether, 8-iodo-4,6-dimethylnonyl butyloxymethyl ether, 8-chloro-4,6-dimethylnonyl pentyloxymethyl ether, 8-bromo-4,6-dimethylnonyl pentyloxymethyl ether, 8-iodo-4,6-dimethylnonyl pentyloxymethyl ether, 8-chloro-4,6-dimethylnonyl hexyloxymethyl ether, 8-bromo-4,6-dimethylnonyl hexyloxymethyl ether, 8-iodo-4,6-dimethylnonyl hexyloxymethyl ether, 8-chloro-4,6-dimethylnonyl heptyloxymethyl ether, 8-bromo-4,6-dimethylnonyl heptyloxymethyl ether, 8-iodo-4,6-dimethylnonyl heptyloxymethyl ether, 8-chloro-4,6-dimethylnonyl octyloxymethyl ether, 8-bromo-4,6-dimethylnonyl octyloxymethyl ether, 8-iodo-4,6-dimethylnonyl octyloxymethyl ether, 8-chloro-4,6-dimethylnonyl nonyloxymethyl ether, 8-bromo-4,6-dimethylnonyl nonyloxymethyl ether, 8-iodo-4,6-dimethylnonyl nonyloxymethyl ether, 8-chloro-4,6-dimethylnonyl decyloxymethyl ether, 8-bromo-4,6-dimethylnonyl decyloxymethyl ether, 8-iodo-4,6-dimethylnonyl decyloxymethyl ether, 8-chloro-4,6-dimethylnonyl benzyloxymethyl ether, 8-bromo-4,6-dimethylnonyl benzyloxymethyl ether, and 8-iodo-4,6-dimethylnonyl benzyloxymethyl ether;
10-halo-4,6,8-trimethylundecyl alkoxymethyl ether compounds ((1: *n* = 4) or (1': *n+*1 = 4)) such as 10-chloro-4,6,8-trimethylundecyl methoxymethyl ether, 10-bromo-4,6,8-trimethylundecyl methoxymethyl ether, 10-iodo-4,6,8-trimethylundecyl methoxymethyl ether, 10-chloro-4,6,8-trimethylundecyl ethoxymethyl ether, 10-bromo-4,6,8-trimethylundecyl ethoxymethyl ether, 10-iodo-4,6,8-trimethylundecyl ethoxymethyl ether, 10-chloro-4,6,8-trimethylundecyl propyloxymethyl ether, 10-bromo-4,6,8-trimethylundecyl propyloxymethyl ether, 10-iodo-4,6,8-trimethylundecyl propyloxymethyl ether, 10-chloro-4,6,8-trimethylundecyl buthyloxymethyl ether, 10-bromo-4,6,8-trimethylundecyl buthyloxymethyl ether, 10-iodo-4,6,8-trimethylundecyl buthylmethyl ether, 10-chloro-4,6,8-trimethylundecyl pentyloxymethyl ether, 10-bromo-4,6,8-trimethylundecyl pentyloxymethyl ether, 10-iodo-4,6,8-trimethylundecyl pentyloxymethyl ether, 10-chloro-4,6,8-trimethylundecyl hexyloxymethyl ether, 10-bromo-4,6,8-trimethylundecyl hexyloxymethyl ether, 10-iodo-4,6,8-trimethylundecyl hexyloxymethyl ether, 10-chloro-4,6,8-trimethylundecyl heptyloxymethyl ether, 10-bromo-4,6,8-trimethylundecyl heptyloxymethyl ether, 10-iodo-4,6,8-trimethylundecyl heptyloxymethyl ether, 10-chloro-4,6,8-trimethylundecyl octyloxymethyl ether, 10-bromo-4,6,8-trimethylundecyl octyloxymethyl ether, 10-iodo-4,6,8-trimethylundecyl octyloxymethyl ether, 10-chloro-4,6,8-trimethylundecyl nonyloxymethyl ether, 10-bromo-4,6,8-trimethylundecyl nonyloxymethyl ether, 10-iodo-4,6,8-trimethylundecyl nonyloxymethyl ether, 10-chloro-4,6,8-trimethylundecyl decyloxymethyl ether, 10-bromo-4,6,8-trimethylundecyl decyloxymethyl ether, 10-iodo-4,6,8-trimethylundecyl decyloxymethyl ether, 10-chloro-4,6,8-trimethylundecyl benzyloxymethyl ether, 10-bromo-4,6,8-trimethylundecyl benzyloxymethyl ether, and 10-iodo-4,6,8-trimethylundecyl benzyloxymethyl ether;
12-halo-4,6,8,10-tetramethyltridecyl alkoxymethyl ether compounds ((1: *n* = 5) or (1': *n*+1 = 5)) such as 12-chloro-4,6,8,10-tetramethyltridecyl methoxymethyl ether, 12-bromo-4,6,8,10-tetramethyltridecyl methoxymethyl ether, 12-iodo-4,6,8,10-tetramethyltridecyl methoxymethyl ether, 12-chloro-4,6,8,10-tetramethyltridecyl ethoxymethyl ether, 12-bromo-4,6,8,10-tetramethyltridecyl ethoxymethyl ether, 12-iodo-4,6,8,10-tetramethyltridecyl ethoxymethyl ether, 12-chloro-4,6,8,10-tetramethyltridecyl propyloxymethyl ether, 12-bromo-4,6,8,10-tetramethyltridecyl propyloxymethyl ether, 12-iodo-4,6,8,10-tetramethyltridecyl propyloxymethyl ether, 12-chloro-4,6,8,10-tetramethyltridecyl buthyloxymethyl ether, 12-bromo-4,6,8,10-tetramethyltridecyl buthyloxymethyl ether, 12-iodo-4,6,8,10-tetramethyltridecyl buthyloxymethyl ether, 12-chloro-4,6,8,10-tetramethyltridecyl pentyloxymethyl ether, 12-bromo-4,6,8,10-tetramethyltridecyl pentyloxymethyl ether, 12-iodo-4,6,8,10-tetramethyltridecyl pentyloxymethyl ether, 12-chloro-4,6,8,10-tetramethyltridecyl hexyloxymethyl ether, 12-bromo-4,6,8,10-tetramethyltridecyl hexyloxymethyl ether, 12-iodo-4,6,8,10-tetramethyltridecyl hexyloxymethyl ether, 12-chloro-4,6,8,10-tetramethyltridecyl heptyloxymethyl ether, 12-bromo-4,6,8,10-tetramethyltridecyl heptyloxymethyl ether, 12-iodo-4,6,8,10-tetramethyltridecyl heptyloxymethyl ether, 12-chloro-4,6,8,10-tetramethyltridecyl octyloxymethyl ether, 12-bromo-4,6,8,10-tetramethyltridecyl octyloxymethyl ether, 12-iodo-4,6,8,10-tetramethyltridecyl octyloxymethyl ether, 12-chloro-4,6,8,10-tetramethyltridecyl nonyloxymethyl ether, 12-bromo-4,6,8,10-tetramethyltridecyl nonyloxymethyl ether, 12-iodo-4,6,8,10-tetramethyltridecyl nonyloxymethyl ether, 12-chloro-4,6,8,10-tetramethyltridecyl decyloxymethyl ether, 12-bromo-4,6,8,10-tetramethyltridecyl decyloxymethyl ether, 12-iodo-4,6,8,10-tetramethyltridecyl decyloxymethyl ether, 12-chloro-4,6,8,10-tetramethyltridecyl benzyloxymethyl ether, 12-bromo-4,6,8,10-tetramethyltridecyl benzyloxymethyl ether, and 12-iodo-4,6,8,10-tetramethyltridecyl benzyloxymethyl ether;
14-halo-4,6,8,10,12-pentamethylpentadecyl alkoxymethyl ether compounds ((1: *n* = 6) or (1': *n*+1 = 6)) such as 14-chloro-4,6,8,10,12-pentamethylpentadecyl methoxymethyl ether, 14-bromo-4,6,8,10,12-pentamethylpentadecyl methoxymethyl ether, 14-iodo-4,6,8,10,12-pentamethylpentadecyl methoxymethyl ether, 14-chloro-4,6,8,10,12-pentamethylpentadecyl ethoxymethyl ether, 14-bromo-4,6,8,10,12-pentamethylpentadecyl ethoxymethyl ether, 14-iodo-4,6,8,10,12-pentamethylpentadecyl ethoxymethyl ether, 14-chloro-4,6,8,10,12-pentamethylpentadecyl propyloxymethyl ether, 14-bromo-4,6,8,10,12-pentamethylpentadecyl propyloxymethyl ether, 14-iodo-4,6,8,10,12-pentamethylpentadecyl propyloxymethyl ether, 14-chloro-4,6,8,10,12-pentamethylpentadecyl butyloxymethyl ether, 14-bromo-4,6,8,10,12-pentamethylpentadecyl butyloxymethyl ether, 14-iodo-4,6,8,10,12-pentamethylpentadecyl butyloxymethyl ether, 14-chloro-4,6,8,10,12-pentamethylpentadecyl pentyloxymethyl ether, 14-bromo-4,6,8,10,12-pentamethylpentadecyl pentyloxymethyl ether, 14-iodo-4,6,8,10,12-pentamethylpentadecyl pentyloxymethyl ether, 14-chloro-4,6,8,10,12-pentamethylpentadecyl hexyloxymethyl ether, 14-bromo-4,6,8,10,12-pentamethylpentadecyl hexyloxymethyl ether, 14-iodo-4,6,8,10,12-pentamethylpentadecyl hexyloxymethyl ether, 14-chloro-4,6,8,10,12-pentamethylpentadecyl heptyloxymethyl ether, 14-bromo-4,6,8,10,12-pentamethylpentadecyl heptyloxymethyl ether, 14-iodo-4,6,8,10,12-pentamethylpentadecyl heptyloxymethyl ether, 14-chloro-4,6,8,10,12-pentamethylpentadecyl octyloxymethyl ether, 14-bromo-4,6,8,10,12-pentamethylpentadecyl octyloxymethyl ether, 14-iodo-4,6,8,10,12-pentamethylpentadecyl octyloxymethyl ether, 14-chloro-4,6,8,10,12-pentamethylpentadecyl nonyloxymethyl ether, 14-bromo-4,6,8,10,12-pentamethylpentadecyl nonyloxymethyl ether, 14-iodo-4,6,8,10,12-pentamethylpentadecyl nonyloxymethyl ether, 14-chloro-4,6,8,10,12-pentamethylpentadecyl decyloxymethyl ether, 14-bromo-4,6,8,10,12-pentamethylpentadecyl decyloxymethyl ether, 14-iodo-4,6,8,10,12-pentamethylpentadecyl decyloxymethyl ether, 14-chloro-4,6,8,10,12-pentamethylpentadecyl benzyloxymethyl ether, 14-bromo-4,6,8,10,12-pentamethylpentadecyl benzyloxymethyl ether, and 14-iodo-4,6,8,10,12-pentamethylpentadecyl benzyloxymethyl ether;
17-halo-4,6,8,10,12,14-hexamethylheptadecyl alkoxymethyl ether compounds ((1: *n* = 7) or (1': *n*+1 = 7)) such as 17-chloro-4,6,8,10,12,14-hexamethylheptadecyl methoxymethyl ether, 17-bromo-4,6,8,10,12,14-hexamethylheptadecyl methoxymethyl ether, 17-iodo-4,6,8,10,12,14-hexamethylheptadecyl methoxymethyl ether, 17-chloro-4,6,8,10,12,14-hexamethylheptadecyl ethoxymethyl ether, 17-bromo-4,6,8,10,12,14-hexamethylheptadecyl ethoxymethyl ether, 17-iodo-4,6,8,10,12,14-hexamethylheptadecyl ethoxymethyl ether, 17-chloro-4,6,8,10,12,14-hexamethylheptadecyl propyloxymethyl ether, 17-bromo-4,6,8,10,12,14-hexamethylheptadecyl propyloxymethyl ether, 17-iodo-4,6,8,10,12,14-hexamethylheptadecyl propyloxymethyl ether, 17-chloro-4,6,8,10,12,14-hexamethylheptadecyl butyloxymethyl ether, 17-bromo-4,6,8,10,12,14-hexamethylheptadecyl butyloxymethyl ether, 17-iodo-4,6,8,10,12,14-hexamethylheptadecyl butyloxymethyl ether, 17-chloro-4,6,8,10,12,14-hexamethylheptadecyl pentyloxymethyl ether, 17-bromo-4,6,8,10,12,14-hexamethylheptadecyl pentyloxymethyl ether, 17-iodo-4,6,8,10,12,14-hexamethylheptadecyl pentyloxymethyl ether, 17-chloro-4,6,8,10,12,14-hexamethylheptadecyl hexyloxymethyl ether, 17-bromo-4,6,8,10,12,14-hexamethylheptadecyl hexyloxymethyl ether, 17-iodo-4,6,8,10,12,14-hexamethylheptadecyl hexyloxymethyl ether, 17-chloro-4,6,8,10,12,14-hexamethylheptadecyl heptyloxymethyl ether, 17-bromo-4,6,8,10,12,14-hexamethylheptadecyl heptyloxymethyl ether, 17-iodo-4,6,8,10,12,14-hexamethylheptadecyl heptyloxymethyl ether, 17-chloro-4,6,8,10,12,14-hexamethylheptadecyl octyloxymethyl ether, 17-bromo-4,6,8,10,12,14-hexamethylheptadecyl octyloxymethyl ether, 17-iodo-4,6,8,10,12,14-hexamethylheptadecyl octyloxymethyl ether, 17-chloro-4,6,8,10,12,14-hexamethylheptadecyl nonyloxymethyl ether, 17-bromo-4,6,8,10,12,14-hexamethylheptadecyl nonyloxymethyl ether, 17-iodo-4,6,8,10,12,14-hexamethylheptadecyl nonyloxymethyl ether, 17-chloro-4,6,8,10,12,14-hexamethylheptadecyl decyloxymethyl ether, 17-bromo-4,6,8,10,12,14-hexamethylheptadecyl decyloxymethyl ether, 17-iodo-4,6,8,10,12,14-hexamethylheptadecyl decyloxymethyl ether, 17-chloro-4,6,8,10,12,14-hexamethylheptadecyl benzyloxymethyl ether, 17-bromo-4,6,8,10,12,14-hexamethylheptadecyl benzyloxymethyl ether, and 17-iodo-4,6,8,10,12,14-hexamethylheptadecyl benzyloxymethyl ether; and
19-halo-4,6,8,10,12,14,16-heptamethylnonadecyl alkoxymethyl ether compounds (1': *n*+1 = 8) such as 19-chloro-4,6,8,10,12,14,16-heptamethylnonadecyl methoxymethyl ether, 19-bromo-4,6,8,10,12,14,16-heptamethylnonadecyl methoxymethyl ether, 19-iodo-4,6,8,10,12,14,16-heptamethylnonadecyl methoxymethyl ether, 19-chloro-4,6,8,10,12,14,16-heptamethylnonadecyl ethoxymethyl ether, 19-bromo-4,6,8,10,12,14,16-heptamethylnonadecyl ethoxymethyl ether, 19-iodo-4,6,8,10,12,14,16-heptamethylnonadecyl ethoxymethyl ether, 19-chloro-4,6,8,10,12,14,16-heptamethylnonadecyl propyloxymethyl ether, 19-bromo-4,6,8,10,12,14,16-heptamethylnonadecyl propyloxymethyl ether, 19-iodo-4,6,8,10,12,14,16-heptamethylnonadecyl propyloxymethyl ether, 19-chloro-4,6,8,10,12,14,16-heptamethylnonadecyl butyloxymethyl ether, 19-bromo-4,6,8,10,12,14,16-heptamethylnonadecyl butyloxymethyl ether, 19-iodo-4,6,8,10,12,14,16-heptamethylnonadecyl butyloxymethyl ether, 19-chloro-4,6,8,10,12,14,16-heptamethylnonadecyl pentyloxymethyl ether, 19-bromo-4,6,8,10,12,14,16-heptamethylnonadecyl pentyloxymethyl ether, 19-iodo-4,6,8,10,12,14,16-heptamethylnonadecyl pentyloxymethyl ether, 19-chloro-4,6,8,10,12,14,16-heptamethylnonadecyl hexyloxymethyl ether, 19-bromo-4,6,8,10,12,14,16-heptamethylnonadecyl hexyloxymethyl ether, 19-iodo-4,6,8,10,12,14,16-heptamethylnonadecyl hexyloxymethyl ether, 19-chloro-4,6,8,10,12,14,16-heptamethylnonadecyl heptyloxymethyl ether, 19-bromo-4,6,8,10,12,14,16-heptamethylnonadecyl heptyloxymethyl ether, 19-iodo-4,6,8,10,12,14,16-heptamethylnonadecyl heptyloxymethyl ether, 19-chloro-4,6,8,10,12,14,16-heptamethylnonadecyl octyloxymethyl ether, 19-bromo-4,6,8,10,12,14,16-heptamethylnonadecyl octyloxymethyl ether, 19-iodo-4,6,8,10,12,14,16-heptamethylnonadecyl octyloxymethyl ether, 19-chloro-4,6,8,10,12,14,16-heptamethylnonadecyl nonyloxymethyl ether, 19-bromo-4,6,8,10,12,14,16-heptamethylnonadecyl nonyloxymethyl ether, 19-iodo-4,6,8,10,12,14,16-heptamethylnonadecyl nonyloxymethyl ether, 19-chloro-4,6,8,10,12,14,16-heptamethylnonadecyl decyloxymethyl ether, 19-bromo-4,6,8,10,12,14,16-heptamethylnonadecyl decyloxymethyl ether, 19-iodo-4,6,8,10,12,14,16-heptamethylnonadecyl decyloxymethyl ether, 19-chloro-4,6,8,10,12,14,16-heptamethylnonadecyl benzyloxymethyl ether, 19-bromo-4,6,8,10,12,14,16-heptamethylnonadecyl benzyloxymethyl ether, and 19-iodo-4,6,8,10,12,14,16-heptamethylnonadecyl benzyloxymethyl ether.

Although the processes for preparing the haloalkyl alkoxymethyl ether compounds (1) and (1') are not particularly limited, the processes according to, for example, Section II, Section III, and Examples 2, 4, 5, 6, and 11 below may be used.

### II. Process for preparing haloalkyl alkoxymethyl ether compound (1': n+1 = 2 to 8)

The haloalkyl alkoxymethyl ether compound of the following general formula *(1': n+1 = 2* to 8) is prepared from the haloalkyl alkoxymethyl ether compound (1: *n* = 1 to 7) according to the preparation process of the following chemical reaction formula:

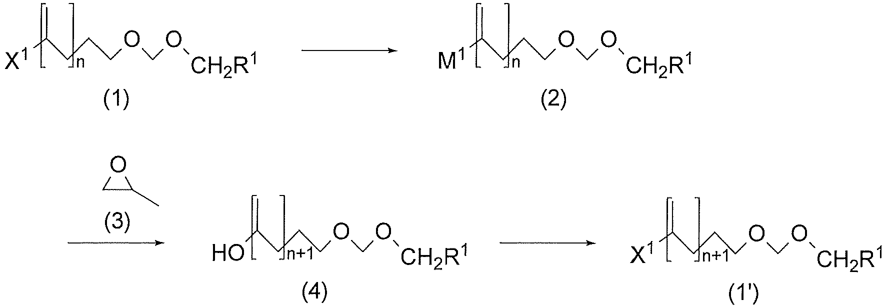

Specifically, to prepare the haloalkyl alkoxymethyl ether compound (1': *n+*1 = 2 to 8), a haloalkyl alkoxymethyl ether compound (1: *n* = 1 to 7) is converted into a nucleophilic reagent, (2*n*+2)-alkoxymethoxyalkyl compound (2). The nucleophilic reagent, (2*n*+2)-alkoxymethoxyalkyl compound (2), is then subjected to a nucleophilic addition reaction with propylene oxide (3) to prepare a hydroxyalkyl alkoxymethyl ether compound (4). The hydroxyalkyl alkoxymethyl ether compound (4) is then subjected to a halogenation reaction to form the haloalkyl alkoxymethyl ether compound (1': *n+*1 = 2 to 8).

### (i) Nucleophilic reagent, (2n+2)-alkoxymethoxyalkyl compound (2), and process for preparing nucleophilic reagent, (2n+2)-alkoxymethoxyalkyl compound (2)

(a) The aforesaid nucleophilic reagent, (2*n+*2)-alkoxymethoxyalkyl compound (2), will be described in detail below.

The nucleophilic reagent, (2*n*+2)-alkoxymethoxyalkyl compound (2), is represented by the following general formula (2):

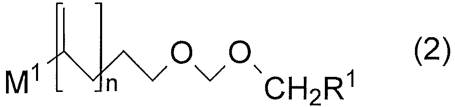

In the general formula (2) above, M¹ represents Li, MgZ¹, CuZ¹, or CuLiZ¹, Z¹ represents a halogen atom or Z², Z² is represented by: 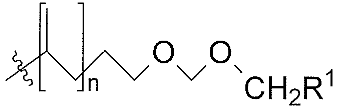 wherein R¹ and *n* are as defined for the general formulas (1) and (1') above, and the wavy line indicates that the structures therebeyond are abbreviated. *n* is as defined above, and represents an integer of 1 to 7. Examples of the halogen atom Z¹ include a chlorine atom, a bromine atom, and an iodine atom.

The nucleophilic reagent, (2*n*+2)-alkoxymethoxyalkyl compound (2: M¹ = MgZ¹), is a Grignard reagent.

Specific examples of the nucleophilic reagent, (2*n*+2)-alkoxymethoxyalkyl compound (2), include (2*n*+2)-alkoxymethoxyalkyllithium; (2*n*+2)-alkoxymethoxyalkylmagnesium halide reagents (when M¹ = MgZ¹) such as (2*n*+2)-alkoxymethoxyalkylmagnesium chloride, (2*n*+2)-alkoxymethoxyalkylmagnesium bromide, and (2*n*+2)-alkoxymethoxyalkylmagnesium iodide (i.e., Grignard reagents); bis[(2*n*+2)-alkoxymethoxyalkyl]cuprate (when M¹ = CuZ¹); and Gilman reagents (when M¹ = CuLiZ¹) such as lithium bis[(2*n*+2)-alkoxymethoxyalkyl]cuprate (i.e., Gilman reagents). Grignard reagents such as (2*n*+2)-alkoxymethoxyalkylmagnesium halide reagents are preferred. By using said Grignard reagents such as (2*n*+2)-alkoxymethoxyalkylmagnesium halide reagents, a preferred reactivity may be ensured.

The nucleophilic reagent, (2*n*+2)-alkoxymethoxyalkyl compound (2), may be used to prepare not only the haloalkyl alkoxymethyl ether compound (1'), but also to prepare 4,6,8,10,16-pentamethyldocosane as shown in Section IV below for the nucleophilic reagent, 8-alkoxymethoxy-1,3,5-trimethyloctyl compound (2: *n* = 3), when *n* is 3.

(b) A process for preparing the aforesaid nucleophilic reagent, (2*n*+2)-alkoxymethoxyalkyl compound (2), will be described in detail below.

The nucleophilic reagent, (2*n*+2)-alkoxymethoxyalkyl compound (2), may be prepared from the aforesaid haloalkyl alkoxymethyl ether compound (1).

A process for preparing the nucleophilic reagent, (2*n*+2)-alkoxymethoxyalkyl compound (2), wherein M¹ is MgZ¹, that is, the (2*n*+2)-alkoxymethoxyalkylmagnesium halide reagent (2: M¹ = MgZ¹), will be described in detail as an example.

The (2*n*+2)-alkoxymethoxyalkylmagnesium halide reagent (2: M¹ = MgZ¹) may be prepared, for example, by reacting the aforesaid haloalkyl alkoxymethyl ether compound (1) with magnesium in a solvent, as shown in the following chemical reaction formula:

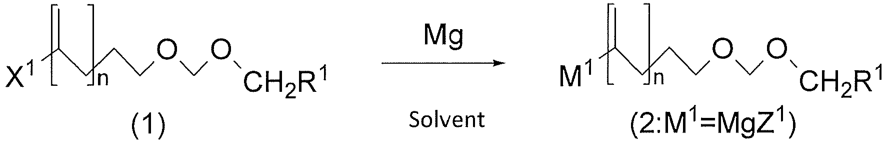

The amount of magnesium used, per mol of the haloalkyl alkoxymethyl ether compound (1), is preferably 1.0 to 2.0 gram atoms. By using said preferred amount, a preferred completion of the reaction may be ensured.

Examples of the solvent include general solvents such as, for example, ether solvents such as tetrahydrofuran (THF), 2-methyltetrahydrofuran (2-MeTHF), diethyl ether, dibutyl ether, 4-methyltetrahydropyran (MTHP), cyclopentylmethylether, and 1,4-dioxane; and hydrocarbon solvents such as hexane, heptane, benzene, toluene, xylene, and cumene. Hydrocarbon solvents such as toluene and xylene; and ether solvents such as tetrahydrofuran, 2-methyltetrahydrofuran, and 4-methyltetrahydropyran are preferred. By using said hydrocarbon solvents such as toluene and xylene; and ether solvents such as tetrahydrofuran, 2-methyltetrahydrofuran, and 4-methyltetrahydropyran, a preferred reaction rate of forming the aforesaid Grignard reagent may be ensured. Tetrahydrofuran, 2-methyltetrahydrofuran, and 4-methyltetrahydropyran are more preferred. By using said tetrahydrofuran, 2-methyltetrahydrofuran, and 4-methyltetrahydropyran, a more preferred reaction rate of forming the aforesaid Grignard reagent may be ensured.

The solvent may be used alone or in combination thereof, if necessary. The solvent may be a commercially available one.

The amount of the solvent used, per mol of the haloalkyl alkoxymethyl ether compound (1), is preferably 30 to 5,000 g, and more preferably 50 to 3,000 g. By using said preferred amount and said more preferred amount, a preferred reactivity and a more preferred reactivity may be ensured.

The reaction temperature varies, depending on the solvent used, and is preferably 30 to 120°C. By using said preferred reaction temperature, a preferred reactivity may be ensured.

The reaction time varies, depending on the solvent used and/or production scale, and is preferably 0.5 to 100 hours. By using said preferred reaction time, a preferred reactivity may be ensured.

### (ii) Hydroxyalkyl alkoxymethyl ether compound (4) and process for preparing hydroxyalkyl alkoxymethyl ether compound (4)

(a) The hydroxyalkyl alkoxymethyl ether compound (4) will be described in detail below.

The hydroxyalkyl alkoxymethyl ether compound (4) is represented by the following general formula (4):

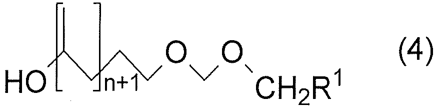

In the general formula (4) above, R¹ and *n* are as defined for general formulas (1) and (1').

Specific examples of the hydroxyalkyl alkoxymethyl ether compound (4) include the following compounds:
6-hydroxy-4-methylheptyl alkoxymethyl ether compounds (*n* = 1) such as 6-hydroxy-4-methylheptyl methoxymethyl ether, 6-hydroxy-4-methylheptyl ethoxymethyl ether, 6-hydroxy-4-methylheptyl propyloxymethyl ether, 6-hydroxy-4-methylheptyl butyloxymethyl ether, 6-hydroxy-4-methylheptyl pentyloxymethyl ether, 6-hydroxy-4-methylheptyl hexyloxymethyl ether, 6-hydroxy-4-methylheptyl heptyloxymethyl ether, 6-hydroxy-4-methylheptyl octyloxymethyl ether, 6-hydroxy-4-methylheptyl nonyloxymethyl ether, 6-hydroxy-4-methylheptyl decyloxymethyl ether, and 6-hydroxy-4-methylheptyl benzyloxymethyl ether;
8-hydroxy-4,6-dimethylnonyl alkoxymethyl ether compounds (*n* = 2) such as 8-hydroxy-4,6-dimethylnonyl methoxymethyl ether, 8-hydroxy-4,6-dimethylnonyl ethoxymethyl ether, 8-hydroxy-4,6-dimethylnonyl propyloxymethyl ether, 8-hydroxy-4,6-dimethylnonyl butyloxymethyl ether, 8-hydroxy-4,6-dimethylnonyl pentyloxymethyl ether, 8-hydroxy-4,6-dimethylnonyl hexyloxymethyl ether, 8-hydroxy-4,6-dimethylnonyl heptyloxymethyl ether, 8-hydroxy-4,6-dimethylnonyl octyloxymethyl ether, 8-hydroxy-4,6-dimethylnonyl nonyloxymethyl ether, 8-hydroxy-4,6-dimethylnonyl decyloxymethyl ether, and 8-hydroxy-4,6-dimethylnonyl benzyloxymethyl ether;
10-hydroxy-4,6,8-trimethylundecyl alkoxymethyl ether compounds (n = 3) such as 10-hydroxy-4,6,8-trimethylundecyl methoxymethyl ether, 10-hydroxy-4,6,8-trimethylundecyl ethoxymethyl ether, 10-hydroxy-4,6,8-trimethylundecyl propyloxymethyl ether, 10-hydroxy-4,6,8-trimethylundecyl butyloxymethyl ether, 10-hydroxy-4,6,8-trimethylundecyl pentyloxymethyl ether, 10-hydroxy-4,6,8-trimethylundecyl hexyloxymethyl ether, 10-hydroxy-4,6,8-trimethylundecyl heptyloxymethyl ether, 10-hydroxy-4,6,8-trimethylundecyl octyloxymethyl ether, 10-hydroxy-4,6,8-trimethylundecyl nonyloxymethyl ether, 10-hydroxy-4,6,8-trimethylundecyl decyloxymethyl ether, and 10-hydroxy-4,6,8-trimethylundecyl benzyloxymethyl ether;
12-hydroxy-4,6,8,10-tetramethyltridecyl alkoxymethyl ether compounds (n = 4) such as 12-hydroxy-4,6,8,10-tetramethyltridecyl methoxymethyl ether, 12-hydroxy-4,6,8,10-tetramethyltridecyl ethoxymethyl ether, 12-hydroxy-4,6,8,10-tetramethyltridecyl propyloxymethyl ether, 12-hydroxy-4,6,8,10-tetramethyltridecyl butyloxymethyl ether, 12-hydroxy-4,6,8,10-tetramethyltridecyl pentyloxymethyl ether, 12-hydroxy-4,6,8,10-tetramethyltridecyl hexyloxymethyl ether, 12-hydroxy-4,6,8,10-tetramethyltridecyl heptyloxymethyl ether, 12-hydroxy-4,6,8,10-tetramethyltridecyl octyloxymethyl ether, 12-hydroxy-4,6,8,10-tetramethyltridecyl nonyloxymethyl ether, 12-hydroxy-4,6,8,10-tetramethyltridecyl decyloxymethyl ether, and 12-hydroxy-4,6,8,10-tetramethyltridecyl benzyloxymethyl ether;
14-hydroxy-4,6,8,10,12-pentamethylpentadecyl alkoxymethyl ether compounds (*n* = 5) such as 14-hydroxy-4,6,8,10,12-pentamethylpentadecyl methoxymethyl ether, 14-hydroxy-4,6,8,10,12-pentamethylpentadecyl ethoxymethyl ether, 14-hydroxy-4,6,8,10,12-pentamethylpentadecyl propyloxymethyl ether, 14-hydroxy-4,6,8,10,12-pentamethylpentadecyl butyloxymethyl ether, 14-hydroxy-4,6,8,10,12-pentamethylpentadecyl pentyloxymethyl ether, 14-hydroxy-4,6,8,10,12-pentamethylpentadecyl hexyloxymethyl ether, 14-hydroxy-4,6,8,10,12-pentamethylpentadecyl heptyloxymethyl ether, 14-hydroxy-4,6,8,10,12-pentamethylpentadecyl octyloxymethyl ether, 14-hydroxy-4,6,8,10,12-pentamethylpentadecyl nonyloxymethyl ether, 14-hydroxy-4,6,8,10,12-pentamethylpentadecyl decyloxymethyl ether, and 14-hydroxy-4,6,8,10,12-pentamethylpentadecyl benzyloxymethyl ether;
16-hydroxy-4,6,8,10,12,14-hexamethylheptadecyl alkoxymethyl ether compounds (*n* = 6) such as 16-hydroxy-4,6,8,10,12,14-hexamethylheptadecyl methoxymethyl ether, 16-hydroxy-4,6,8,10,12,14-hexamethylheptadecyl ethoxymethyl ether, 16-hydroxy-4,6,8,10,12,14-hexamethylheptadecyl propyloxymethyl ether, 16-hydroxy-4,6,8,10,12,14-hexamethylheptadecyl butyloxymethyl ether, 16-hydroxy-4,6,8,10,12,14-hexamethylheptadecyl pentyloxymethyl ether, 16-hydroxy-4,6,8,10,12,14-hexamethylheptadecyl hexyloxymethyl ether, 16-hydroxy-4,6,8,10,12,14-hexamethylheptadecyl heptyloxymethyl ether, 16-hydroxy-4,6,8,10,12,14-hexamethylheptadecyl octyloxymethyl ether, 16-hydroxy-4,6,8,10,12,14-hexamethylheptadecyl nonyloxymethyl ether, 16-hydroxy-4,6,8,10,12,14-hexamethylheptadecyl decyloxymethyl ether, and 16-hydroxy-4,6,8,10,12,14-hexamethylheptadecyl benzyloxymethyl ether; and
18-hydroxy-4,6,8,10,12,14,16-heptamethylnonadecyl alkoxymethyl ether compounds (*n* = 7) such as 18-hydroxy-4,6,8,10,12,14,16-heptamethylnonadecyl methoxymethyl ether, 18-hydroxy-4,6,8,10,12,14,16-heptamethylnonadecyl ethoxymethyl ether, 18-hydroxy-4,6,8,10,12,14,16-heptamethylnonadecyl propyloxymethyl ether, 18-hydroxy-4,6,8,10,12,14,16-heptamethylnonadecyl butyloxymethyl ether, 18-hydroxy-4,6,8,10,12,14,16-heptamethylnonadecyl pentyloxymethyl ether, 18-hydroxy-4,6,8,10,12,14,16-heptamethylnonadecyl hexyloxymethyl ether, 18-hydroxy-4,6,8,10,12,14,16-heptamethylnonadecyl heptyloxymethyl ether, 18-hydroxy-4,6,8,10,12,14,16-heptamethylnonadecyl octyloxymethyl ether, 18-hydroxy-4,6,8,10,12,14,16-heptamethylnonadecyl nonyloxymethyl ether, 18-hydroxy-4,6,8,10,12,14,16-heptamethylnonadecyl decyloxymethyl ether, and 18-hydroxy-4,6,8,10,12,14,16-heptamethylnonadecyl benzyloxymethyl ether.

(b) A process for preparing a hydroxyalkyl alkoxymethyl ether compound (4) from the aforesaid nucleophilic reagent, (2*n*+2)-alkoxymethoxyalkyl compound (2), will be described in detail below.

The hydroxyalkyl alkoxymethyl ether compound (4) may be prepared from the aforesaid nucleophilic reagent, (2*n*+2)-alkoxymethoxyalkyl compound (2).

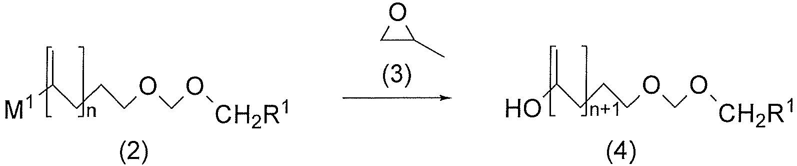

The preparation process includes at least the step of subjecting the nucleophilic reagent, (2*n*+2)-alkoxymethoxyalkyl compound (2), to a nucleophilic addition reaction with propylene oxide of the general formula (3) above to obtain the hydroxyalkyl alkoxymethyl ether compound (4).

In the aforesaid nucleophilic addition reaction, the nucleophilic reagent, (2*n*+2)-alkoxymethoxyalkyl compound (2), may be used alone or in combination thereof, if necessary. The nucleophilic reagent, (2*n*+2)-alkoxymethoxyalkyl compound (2), may be synthesized in house as in (i) above.

Propylene oxide (3) may be a commercially available one, or may be synthesized in house.

The amount of the nucleophilic reagent, (2*n*+2)-alkoxymethoxyalkyl compound (2), per mol of propylene oxide (3), used in the nucleophilic addition reaction is preferably 0.6 to 1.3 mol. By using said preferred amount, preferred economy may be ensured.

A solvent may be incorporated in the nucleophilic addition reaction, if necessary.

Examples of the solvent include general solvents such as, for example, ether solvents such as tetrahydrofuran (THF), 2-methyltetrahydrofuran (2-MeTHF), diethyl ether, dibutyl ether, 4-methyltetrahydropyran (MTHP), cyclopentylmethylether, and 1,4-dioxane; hydrocarbon solvents such as hexane, heptane, benzene, toluene, xylene, and cumene; and polar solvents such as *N,N*-dimethylformamide (DMF), *N,N-*dimethylacetamide (DMAC), *N*-methylpyrrolidone (NMP), dimethyl sulfoxide (DMSO), γ-butyrolactone (GBL), acetonitrile, *N,N*'-dimethylpropylene urea (DMPU), hexamethylphosphoric triamide (HMPA), dichloromethane, and chloroform. Hydrocarbon solvents such as toluene and xylene; ether solvents such as tetrahydrofuran, 2-methyltetrahydrofuran, and 4-methyltetrahydropyran; and acetonitrile are preferred. By using said hydrocarbon solvents such as toluene and xylene; ether solvents such as tetrahydrofuran, 2-methyltetrahydrofuran, and 4-methyltetrahydropyran; and acetonitrile, a preferred reactivity may be ensured. Tetrahydrofuran, 2-methyltetrahydrofuran, toluene, and xylene are more preferred. By using said tetrahydrofuran, 2-methyltetrahydrofuran, toluene, and xylene, a more preferred reactivity may be ensured.

The solvent may be used alone or in combination thereof, if necessary. The solvent may be a commercially available one.

The amount of the solvent used, per mol of the nucleophilic reagent, (2n+2)-alkoxymethoxyalkyl compound (2), is preferably 30 to 5,000 g, and more preferably 50 to 3,000 g. By using said preferred amount and said more preferred amount, a preferred reactivity and a more preferred reactivity may be ensured.

The nucleophilic addition reaction may be carried out in the presence of a catalyst, if necessary. Examples of the catalyst include cuprous halides such as cuprous chloride, cuprous bromide, and cuprous iodide; and cupric halides such as cupric chloride, cupric bromide, and cupric iodide. Cuprous halides are preferred. By using said cuprous halides, a preferred reactivity may be ensured. Cuprous chloride is more preferred. By using said cuprous chloride, a more preferred reactivity may be ensured.

The catalyst may be used alone or in combination thereof, if necessary. The catalyst may be a commercially available one.

The amount of the catalyst used, per mol of the nucleophilic reagent, (2n+2)-alkoxymethoxyalkyl compound (2), is preferably 0.0001 to 0.300 mol, and more preferably 0.0003 to 0.100 mol. By using said preferred amount and said more preferred amount, a preferred reaction rate and/or post-treatment and a more preferred reaction rate and/or post-treatment may be ensured.

When the nucleophilic addition reaction is carried out in the presence of the catalyst, a co-catalyst may be used, if necessary. Examples of the co-catalyst include phosphorus compounds such as trialkyl phosphite compounds having 3 to 9 carbon atoms such as triethyl phosphite; and triarylphosphine compounds having 18 to 21 carbon atoms such as triphenylphosphine. Trialkyl phosphite compounds having 3 to 9 carbon atoms, which are liquids at a room temperature, are preferred. By using said trialkyl phosphite compounds having 3 to 9 carbon atoms, a preferred handling may be ensured.

The co-catalyst may be used alone or in combination thereof, if necessary. The co-catalyst may be a commercially available one.

When the co-catalyst is used, the amount of the co-catalyst used, per mol of the nucleophilic reagent, (2n+2)-alkoxymethoxyalkyl compound (2), is preferably more than 0 up to 0.500 mol, and more preferably more than 0 up to 0.200 mol. By using said preferred amount and said more preferred amount, a preferred reactivity and a more preferred reactivity may be ensured.

When the nucleophilic addition reaction is carried out in the presence of the catalyst, a lithium halide may be added, if necessary.

Examples of the lithium halide include lithium chloride, lithium bromide, and lithium iodide. Lithium chloride is preferred. By using said lithium chloride, a preferred reactivity may be ensured.

When the lithium halide is used, the amount of the lithium halide used, per mol of the nucleophilic reagent, (2n+2)-alkoxymethoxyalkyl compound (2), is preferably more than 0 up to 0.250 mol. By using said preferred amount, a preferred reactivity may be ensured.

The reaction temperature of the nucleophilic addition reaction varies, depending on the nucleophilic reagent, (2n+2)-alkoxymethoxyalkyl compound (2), used, and is preferably -78 to 70°C, more preferably -20 to 50°C, and most preferably 5 to 35°C. By using said preferred reaction temperature, said more preferred reaction temperature, and said most preferred reaction temperature, a preferred reactivity, a more preferred reactivity, and a most preferred reactivity may be ensured.

The reaction time of the nucleophilic addition reaction varies, depending on the solvent used and/or production scale, and is preferably 0.5 to 100 hours. By using said preferred reaction time, a preferred reactivity may be ensured.

### (iii) Process for preparing haloalkyl alkoxymethyl ether compound (1': n+1 = 2 to 8)

(a) The haloalkyl alkoxymethyl ether compound (1': *n+1* = 2 to 8) is as mentioned above.
(b) A process for preparing the haloalkyl alkoxymethyl ether compound (1': *n+1* = 2 to 8) will be described in detail below.

The haloalkyl alkoxymethyl ether compound (1': *n+1* = 2 to 8) may be prepared from the aforesaid hydroxyalkyl alkoxymethyl ether compound (4).

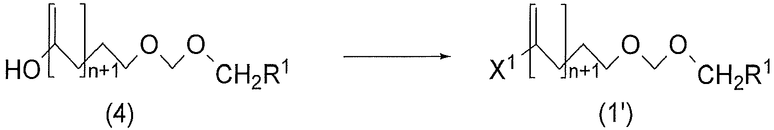

The preparation process includes a step of subjecting the hydroxyalkyl alkoxymethyl ether compound (4) to a halogenation reaction.

The halogenation reaction may be carried out by, for example, a process of tosylating a hydroxy group with a p-toluenesulfonyl halide compound and then halogenating with the metal salt, lithium halide compound; or a process of directly halogenating a hydroxy group with a halogenating agent.

Examples of the halogenating agent include halogens such as chlorine, bromine, and iodine; hydrogen halide compounds such as hydrogen chloride, hydrogen bromide, and hydrogen iodide; methanesulfonyl halide compounds such as methanesulfonyl chloride, methanesulfonyl bromide, and methanesulfonyl iodide; benzenesulfonyl halide compounds such as benzenesulfonyl chloride, benzenesulfonyl bromide, and benzenesulfonyl iodide; p-toluenesulfonyl halide compounds such as p-toluenesulfonyl chloride, p-toluenesulfonyl bromide, and p-toluenesulfonyl iodide; thionyl halide compounds such as thionyl chloride, thionyl bromide, and thionyl iodide; phosphorus halide compounds such as phosphorus trichloride, phosphorus pentachloride, and phosphorus tribromide; carbon tetrahalide compounds such as carbon tetrachloride, carbon tetrabromide, and carbon tetraiodide; alkylsilyl halide compounds such as trimethylsilyl chloride, trimethylsilyl bromide, trimethylsilyl iodide, triethylsilyl chloride, triethylsilyl bromide, triethylsilyl iodide, triisopropylsilyl chloride, triisopropylsilyl bromide, triisopropylsilyl iodide, *tert*-butyldimethylsilyl chloride, *tert*-butyldimethylsilyl bromide, and *tert*-butyldimethylsilyl iodide; oxalyl halide compounds such as oxalyl chloride, oxalyl bromide, and oxalyl iodide; and *N*-halosuccinimide compounds such as *N*-chlorosuccinimide, *N*-bromosuccinimide, and *N*-iodosuccinimide. Methanesulfonyl halide compounds, benzenesulfonyl halide compounds, *p*-toluenesulfonyl halide compounds, and thionyl halide compounds are preferred. By using said methanesulfonyl halide compounds, benzenesulfonyl halide compounds, *p*-toluenesulfonyl halide compounds, and thionyl halide compounds, a preferred suppression of side reactions may be ensured. Methanesulfonyl halide compounds, benzenesulfonyl halide compounds, and thionyl halide compounds are particularly preferred. By using said methanesulfonyl halide compounds, benzenesulfonyl halide compounds, and thionyl halide compounds, a particularly preferred suppression of side reactions may be ensured.

The halogenating agent may be used alone or in combination thereof, if necessary. The halogenating agent may be a commercially available one.

The amount of the halogenating agent used, per mol of the hydroxyalkyl alkoxymethyl ether compound (4), is preferably 0.8 to 5.0 mol, and more preferably 1.0 to 2.5 mol. By using said preferred amount and said more preferred amount, a preferred reactivity and a more preferred reactivity may be ensured.

A base may be incorporated in the halogenation reaction, if necessary.

Examples of the base include hydroxides such as sodium hydroxide, potassium hydroxide, calcium hydroxide, and magnesium hydroxide; carbonates such as sodium carbonate, potassium carbonate, calcium carbonate, and magnesium carbonate; and amines such as triethylamine, *N,N*-diisopropylethylamine, piperidine, pyrrolidine, pyridine, lutidine, 4-dimethylaminopyridine, *N,N*-dimethylaniline, *N,N-*diethylaniline, and 1,8-diazabicyclo[5.4.0]-7-undecene (DBU).

When a methanesulfonyl halide compound, a benzenesulfonyl halide compound, a p-toluenesulfonyl halide compound, or the like is used as the aforesaid halogenating agent, the base is preferably amines, and more preferably pyridines such as pyridine, lutidine, and 4-dimethylaminopyridine.

When a thionyl halide compound is used as the halogenating agent, the base is preferably amines, and more preferably trialkylamines such as triethylamine.

The base may be used alone or in combination thereof, if necessary. The base may be a commercially available one.

When the base is used, the amount of the base used, per mol of the hydroxyalkyl alkoxymethyl ether compound (4), is preferably more than 0 up to 8.0 mol, and more preferably more than 0 up to 3.0 mol. By using said preferred amount and said more preferred amount, a preferred yield and/or economy and a more preferred yield and/or economy may be ensured.

A metal salt may be incorporated in the halogenation reaction, if necessary.

Examples of the metal salt include lithium salts such as lithium chloride, lithium bromide, and lithium iodide; sodium salts such as sodium chloride, sodium bromide, and sodium iodide; potassium salts such as potassium chloride, potassium bromide, and potassium iodide; calcium salts such as calcium chloride, calcium bromide, and calcium iodide; and magnesium salts such as magnesium chloride, magnesium bromide, and magnesium iodide.

When the halogenation with the metal salt, lithium halide compound, is carried out after the tosylation, the reaction is carried out with, for example, lithium salts such as lithium chloride, lithium bromide, and lithium iodide.

The metal salt may be used alone or in combination thereof, if necessary. The metal salt may be a commercially available one.

When the metal salt is used, the amount of the metal salt used, per mol of the hydroxyalkyl alkoxymethyl ether compound (4), is preferably more than 0 up to 30.0 mol, and more preferably more than 0 up to 5.0 mol. By using said preferred amount and said more preferred amount, a preferred reactivity and a more preferred reactivity may be ensured.

Although the metal salt increases the concentration of halide ions in the reaction system to thereby enhance the reactivity, it is preferred not to incorporate the metal salt in the reaction. By not incorporating the metal salt, preferred economy and/or environmental protection may be ensured.

A solvent may be incorporated in the halogenation reaction, if necessary.

Examples of the solvent include general solvents such as, for example, ether solvents such as tetrahydrofuran (THF), 2-methyltetrahydrofuran (2-MeTHF), diethyl ether, dibutyl ether, 4-methyltetrahydropyran (MTHP), cyclopentylmethylether, and 1,4-dioxane; hydrocarbon solvents such as hexane, heptane, benzene, toluene, xylene, and cumene; polar solvents such as *N,N-*dimethylformamide (DMF), *N,N*-dimethylacetamide (DMAC), *N*-methylpyrrolidone (NMP), dimethyl sulfoxide (DMSO), γ-butyrolactone (GBL), acetonitrile, acetone, *N,N*'-dimethylpropylene urea (DMPU), hexamethylphosphoric triamide (HMPA), dichloromethane, and chloroform; and ester solvents such as methyl acetate, ethyl acetate, n-propyl acetate, and n-butyl acetate. 2-Methyltetrahydrofuran, 4-methyltetrahydropyran, dichloromethane, chloroform, γ-butyrolactone, *N*-methylpyrrolidone, *N,N*-dimethylformamide, *N,N*-dimethylacetamide, and acetonitrile are preferred. By using said 2-methyltetrahydrofuran, 4-methyltetrahydropyran, dichloromethane, chloroform, γ-butyrolactone, *N-*methylpyrrolidone, *N,N*-dimethylformamide, *N,N*-dimethylacetamide, and acetonitrile, a preferred reactivity may be ensured. 2-Methyltetrahydrofuran, γ-butyrolactone, and acetonitrile are particularly preferred. By using said 2-methyltetrahydrofuran, γ-butyrolactone, and acetonitrile, particularly preferred safety may be ensured.

The solvent may be used alone or in combination thereof, if necessary. The solvent may be a commercially available one.

When a solvent is incorporated in the halogenation reaction, the amount of the solvent used, per mol of the hydroxyalkyl alkoxymethyl ether compound (4), is preferably more than 0 up to 3,000 g, and more preferably more than 0 up to 800 g.

The solvent may occupy part of the reactor space, which reduces the space for the starting materials, and reduces productivity. Therefore, the reaction may be carried out without a solvent, or with the base as the solvent.

The reaction temperature of the halogenation reaction varies, depending on the halogenating agent used, and is preferably 5 to 180°C, and more preferably 20 to 120°C. By using said preferred reaction temperature and said more preferred reaction temperature, a preferred reactivity and a more preferred reactivity may be ensured.

The reaction time of the halogenation reaction varies, depending on the halogenating agent used and/or production scale, and is preferably 0.5 to 100 hours. By using said preferred reaction time, a preferred reactivity may be ensured.

Thus, the haloalkyl alkoxymethyl ether compound (1': *n*+1) may be prepared by three-carbon homologation from the haloalkyl alkoxymethyl ether compound (1: *n).* Then, *n* of the haloalkyl alkoxymethyl ether compound (1) may be increased unrestrictedly by repeatedly using the haloalkyl alkoxymethyl ether compound (1') thus prepared as the haloalkyl alkoxymethyl ether compound (1: *n*), converting the haloalkyl alkoxymethyl ether compound (1: *n*) into a nucleophilic reagent, and then subjecting the nucleophilic reagent to a nucleophilic addition reaction with propylene oxide and to a halogenation reaction. Specifically, the methyl branch may be unrestrictedly increased to comprehensively prepare various compounds having 1,3-dimethyl skeletons such as 2,4,6,8-tetramethyl-1-undecanol, 2,4,6,8,10,12,14,16-octamethylheptadecane, 4,6,8,10,16-pentamethyldocosane, and 4,6,8,10,16,18-hexamethyldocosane as shown in the following reaction formula:

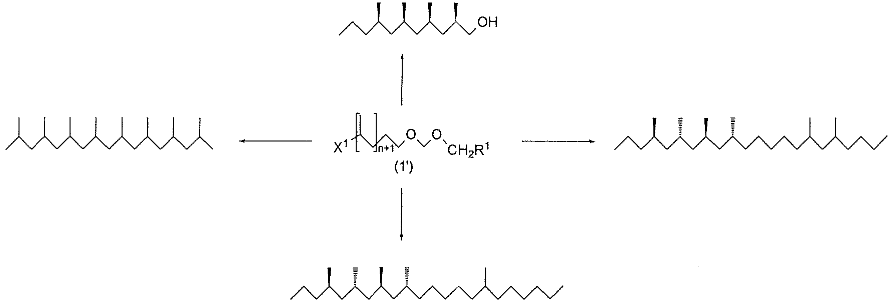

### III. Process for preparing haloalkyl alkoxymethyl ether compound (1: n = 1)

When *n* is 1 in the general formula (1) above, the haloalkyl alkoxymethyl ether compound (1: *n =* 1) (also referred to as 4-halopentyl alkoxymethyl ether compound) is prepared according to, for example, the preparation process of the following chemical reaction formula:

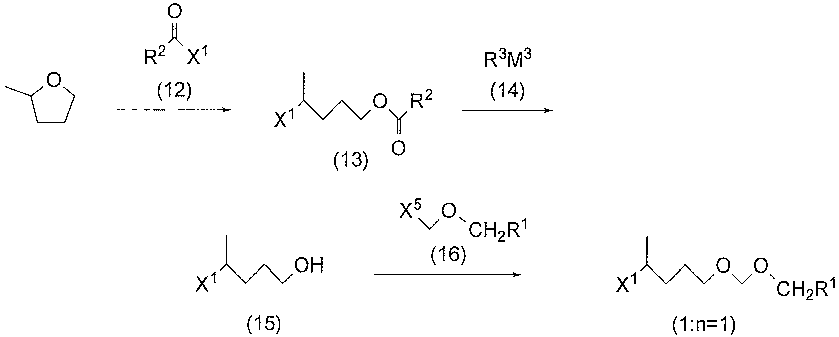

Specifically, the aforesaid 4-haloalkyl alkoxymethyl ether compound (1: *n =* 1), i.e., 4-halopentyl alkoxymethyl ether compound, may be prepared by opening the tetrahydrofuran ring of 2-methyltetrahydrofuran with an acid halide, followed by elimination of the acyl group, and then alkoxymethylation of the hydroxy group.

### (i) 4-Halopentyl acylate compound (13) and process for preparing 4-halopentyl acylate compound (13)

(a) The 4-halopentyl acylate compound (13) will be described in detail below.

The 4-halopentyl acylate compound (13) is represented by the following general formula (13):

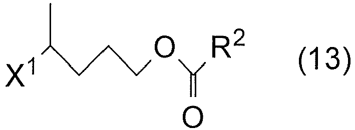

In the general formula (13) above, R² represents an alkyl group having 1 to 9 carbon atoms or a phenyl group, and X¹ is as defined for the general formulas (1) and (1').

Examples of the alkyl group R² include a methyl group, an ethyl group, a propyl group, a butyl group, a pentyl group, a hexyl group, a heptyl group, an octyl group, a nonyl group, and a *tert*-butyl group.

Examples of the halogen atom X¹ include a chlorine atom, a bromine atom, and an iodine atom. A chlorine atom and a bromine atom are preferred. By using said chlorine atom and bromine atom, a preferred reactivity may be ensured. A chlorine atom is particularly preferred. By using said chlorine atom, a particularly preferred reactivity may be ensured.

Specific examples of the 4-halopentyl acylate compound (13) include 4-chloropentyl acetate, 4-bromopentyl acetate, 4-iodopentyl acetate, 4-chloropentyl propionate, 4-bromopentyl propionate, 4-iodopentyl propionate, 4-chloropentyl butyrate, 4-bromopentyl butyrate, 4-iodopentyl butyrate, 4-chloropentyl valerate, 4-bromopentyl valerate, 4-iodopentyl valerate, 4-chloropentyl pivaloate, 4-bromopentyl pivaloate, 4-iodopentyl pivaloate, 4-chloropentyl benzoate, 4-bromopentyl benzoate, and 4-iodopentyl benzoate.

(b) A process for preparing the aforesaid 4-halopentyl acylate compound (13) will be described in detail below.

The 4-halopentyl acylate compound (13) may be prepared from 2-methyltetrahydrofuran and the acid halide (12), as shown in the following chemical reaction formula:

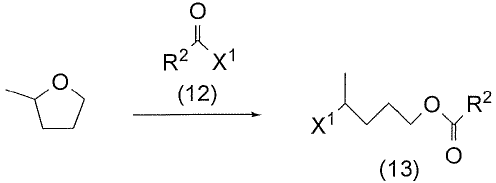

The preparation process includes a step of opening the tetrahydrofuran ring of 2-methyltetrahydrofuran with the acid halide (12).

The acid halide (12) will be described in detail below.

The acid halide (12) is represented by the following general formula (12):

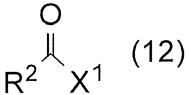

In the general formula (12), R² and X¹ are as defined for the general formula (13).

Specific examples of the acid halide (12) include acetyl halide compounds such as acetyl chloride, acetyl bromide, and acetyl iodide; propionyl halide compounds such as propionyl chloride, propionyl bromide, and propionyl iodide; butyryl halide compounds such as butyryl chloride, butyryl bromide, and butyryl iodide; valeryl halide compounds such as valeryl chloride, valeryl bromide, and valeryl iodide; pivaloyl halide compounds such as pivaloyl chloride, pivaloyl bromide, and pivaloyl iodide; and benzoyl halide compounds such as benzoyl chloride, benzoyl bromide, and benzoyl iodide.

The amount of the acid halide (12) used, per mol of 2-methyltetrahydrofuran, is preferably 0.7 to 1.5 mol, and more preferably 0.8 to 1.1 mol. By using said preferred amount and said more preferred amount, a preferred completion of the reaction and a more preferred completion of the reaction may be ensured.

The ring-opening reaction may be carried out in the presence of a catalyst, if necessary. Examples of the catalyst include zinc halide compounds such as zinc chloride, zinc bromide, and zinc iodide; titanium compounds such as titanium tetrachloride, titanium tetrabromide, titanium(IV) methoxide, titanium(IV) ethoxide, titanium(IV) isopropoxide, and titanium(IV) oxide; and zirconium compounds such as zirconium oxide. Zinc halide compounds are preferred. By using said zinc halide compounds, a preferred reactivity may be ensured.

The catalyst may be used alone or in combination thereof, if necessary. The catalyst may be a commercially available one.

The amount of the catalyst used, per mol of 2-methyltetrahydrofuran, is preferably 0.0001 to 0.3 mol, and more preferably 0.001 to 0.1 mol. By using said preferred amount and said more preferred amount, a preferred completion of the reaction and a more preferred completion of the reaction may be ensured.

A solvent may be incorporated in the ring-opening reaction, if necessary.

Examples of the solvent include those that do not affect the reaction such as, for example, hydrocarbon solvents such as hexane, heptane, benzene, toluene, xylene, and cumene. In view of easy preparation, 2-methyltetrahydrofuran may be used as both a solvent and a substrate without using another solvent.

The solvent may be used alone or in combination thereof, if necessary. The solvent may be a commercially available one.

When the solvent is used, the amount of the solvent used, per mol of 2-methyltetrahydrofuran, is preferably more than 0 up to 3,000 g, and more preferably more than 0 up to 500 g. By using said preferred amount and said more preferred amount, a preferred reactivity and a more preferred reactivity may be ensured.

The reaction temperature varies, depending on the production scale, and is preferably -15 to 85°C, and more preferably -10 to 45°C. By using said preferred reaction temperature and said more preferred reaction temperature, a preferred reactivity and a more preferred reactivity may be ensured.

The reaction time varies, depending on the solvent used and/or production scale, and is preferably 0.5 to 100 hours. By using said preferred reaction time, a preferred reactivity may be ensured.

### (ii) 4-Halo-1-pentanol compound (15) and process for preparing 4-halo-1-pentanol compound (15)

(a) The 4-halo-1-pentanol compound (15) will be described in detail below.

The 4-halo-1-pentanol compound (15) is represented by the following general formula (15):

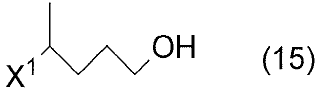

In the general formula (15) above, X¹ is as defined for the general formula (12).

Specific examples of the 4-halo-1-pentanol compound (15) include 4-chloro-1-pentanol, 4-bromo-1-pentanol, and 4-iodo-1-pentanol.

(b) A process for preparing the aforesaid 4-halo-1-pentanol compound (15) will be described in detail below.

The 4-halo-1-pentanol compound (15) may be prepared by deacylating the acyl group of the aforesaid 4-halopentyl acylate compound (13) with the organometallic reagent R³M³ (14).

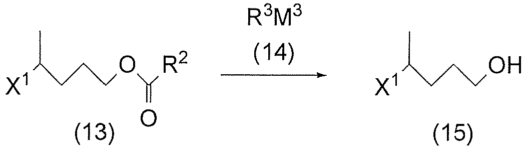

In the general formula (14) above, R³ represents an alkyl group having 1 to 14 carbon atoms, and preferably 1 to 8 carbon atoms, or an ethynyl group.

Examples of the alkyl group R³ include a methyl group, an ethyl group, a propyl group, a butyl group, a pentyl group, a hexyl group, a heptyl group, an octyl group, a nonyl group, a decyl group, an undecyl group, a dodecyl group, a tridecyl group, and a tetradecyl group.

In the general formula (14) above, M³ represents Li, Na, K, Ag, MgZ¹, or CaZ¹, and Z¹ represents a halogen atom or R³. Examples of the halogen atom Z¹ include a chlorine atom, a bromine atom, and an iodine atom.

Specific examples of the organometallic reagent R³M³ (14) include Grignard reagents such as alkyllithiums such as methyllithium, ethyllithium, propyllithium, n-butyllithium, *sec*-butyllithium, *tert*-butyllithium, pentyllithium, hexyllithium, heptyllithium, octyllithium, nonyllithium, and decyllithium; methylmagnesium halide compounds such as methylmagnesium chloride, methylmagnesium bromide, and methylmagnesium iodide; ethylmagnesium halide compounds such as ethylmagnesium chloride, ethylmagnesium bromide, and ethylmagnesium iodide; propylmagnesium halide compounds such as propylmagnesium chloride, propylmagnesium bromide, and propylmagnesium iodide; butylmagnesium halide compounds such as butylmagnesium chloride, butylmagnesium bromide, and butylmagnesium iodide; pentylmagnesium halide compounds such as pentylmagnesium chloride, pentylmagnesium bromide, and pentylmagnesium iodide; hexylmagnesium halide compounds such as hexylmagnesium chloride, hexylmagnesium bromide, and hexylmagnesium iodide; heptylmagnesium halide compounds such as heptylmagnesium chloride, heptylmagnesium bromide, and heptylmagnesium iodide; octylmagnesium halide compounds such as octylmagnesium chloride, octylmagnesium bromide, and octylmagnesium iodide; nonylmagnesium halide compounds such as nonylmagnesium chloride, nonylmagnesium bromide, and nonylmagnesium iodide; decylmagnesium halide compounds such as decylmagnesium chloride, decylmagnesium bromide, and decylmagnesium iodide; and metal acetylides such as lithium acetylide, sodium acetylide, potassium acetylide, calcium acetylide, and silver acetylide. Grignard reagents are preferred. By using said Grignard reagents, a preferred reactivity may be ensured.

The amount of the organometallic reagent R³M³ (14) used, per mol of the 4-halopentyl acylate compound (13), is preferably 1.5 to 5.0 mol, and more preferably 2.0 to 3.5 mol. By using said preferred amount and said more preferred amount, a preferred completion of the reaction and a more preferred completion of the reaction may be ensured.

A solvent may be incorporated in the deacylation, if necessary.

Examples of the solvent include general solvents such as, for example, ether solvents such as tetrahydrofuran (THF), 2-methyltetrahydrofuran (2-MeTHF), diethyl ether, dibutyl ether, 4-methyltetrahydropyran (MTHP), cyclopentylmethylether, and 1,4-dioxane; hydrocarbon solvents such as hexane, heptane, benzene, toluene, xylene, and cumene; and polar solvents such as *N,N-*dimethylformamide (DMF), *N,N-*dimethylacetamide (DMAC), *N*-methylpyrrolidone (NMP), dimethyl sulfoxide (DMSO), γ-butyrolactone (GBL), acetonitrile, *N,N*'-dimethylpropylene urea (DMPU), hexamethylphosphoric triamide (HMPA), dichloromethane, and chloroform. Hydrocarbon solvents such as toluene and xylene; ether solvents such as tetrahydrofuran, 2-methyltetrahydrofuran, and 4-methyltetrahydropyran; and acetonitrile are preferred. By using said hydrocarbon solvents such as toluene and xylene; ether solvents such as tetrahydrofuran, 2-methyltetrahydrofuran, and 4-methyltetrahydropyran; and acetonitrile, a preferred reactivity may be ensured. Tetrahydrofuran, 2-methyltetrahydrofuran, toluene, and xylene are more preferred. By using said tetrahydrofuran, 2-methyltetrahydrofuran, toluene, and xylene, a more preferred reactivity may be ensured.

The solvent may be used alone or in combination thereof, if necessary. The solvent may be a commercially available one.

When the solvent is incorporated in the deacylation, the amount of the solvent used, per mol of the 4-halopentyl acylate compound (13), is preferably more than 0 up to 5,000 g, and more preferably more than 0 up to 3,000 g. By using said preferred amount and said more preferred amount, a preferred reactivity and a more preferred reactivity may be ensured.

The reaction temperature of the deacylation varies, depending on the production scale, and is preferably -15 to 90°C, and more preferably 10 to 50°C. By using said preferred reaction temperature and said more preferred reaction temperature, a preferred reactivity and a more preferred reactivity may be ensured.

The reaction time of the deacylation varies, depending on the solvent used and/or production scale, and is preferably 0.5 to 100 hours. By using said preferred reaction time, a preferred reactivity may be ensured.

### (iii) 4-Halopentyl alkoxymethyl ether compound (1: n = 1) and process for preparing 4-halopentyl alkoxymethyl ether compound (1: n = 1)

(a) 4-Halopentyl alkoxymethyl ether compound (1: *n =* 1) is as mentioned above.
(b) A process for preparing the 4-halopentyl alkoxymethyl ether compound (1: *n* = 1) will be described in detail below.

The 4-halopentyl alkoxymethyl ether compound (1: *n* = 1) may be prepared from the aforesaid 4-halo-1-pentanol compound (15).

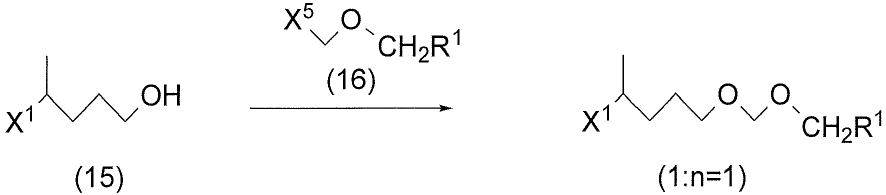

The preparation process includes a step of alkoxymethylating the 4-halo-1-pentanol compound (15) with the halomethyl alkyl ether compound (16).

The halomethyl alkyl ether compound (16) will be described in detail below.

The halomethyl alkyl ether compound (16) is represented by the following general formula (16):

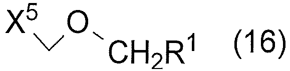

In the general formula (16) above, X⁵ represents a halogen atom. Examples of the halogen atom X⁵ include a chlorine atom, a bromine atom, and an iodine atom. A chlorine atom and a bromine atom are preferred. By using said chlorine atom and bromine atom, a preferred reactivity may be ensured. A chlorine atom is particularly preferred. By using said chlorine atom, a particularly preferred reactivity may be ensured.

In the general formula (16) above, R¹ is as defined for the general formulas (1) and (1') above.

Specific examples of the halomethyl alkyl ether compound (16) include the following compounds:
chloromethyl alkyl ether compounds such as chloromethyl methyl ether, chloromethyl ethyl ether, chloromethyl propyl ether, chloromethyl butyl ether, chloromethyl pentyl ether, chloromethyl hexyl ether, chloromethyl heptyl ether, chloromethyl octyl ether, chloromethyl nonyl ether, and chloromethyl decyl ether;
chloromethyl benzyl ether;
bromomethyl alkyl ether compounds such as bromomethyl methyl ether, bromomethyl ethyl ether, bromomethyl propyl ether, bromomethyl butyl ether, bromomethyl pentyl ether, bromomethyl hexyl ether, bromomethyl heptyl ether, bromomethyl octyl ether, bromomethyl nonyl ether, and bromomethyl decyl ether;
bromomethyl benzyl ether;
iodomethyl alkyl ether compounds such as iodomethyl methyl ether, iodomethyl ethyl ether, iodomethyl propyl ether, iodomethyl butyl ether, iodomethyl pentyl ether, iodomethyl hexyl ether, iodomethyl heptyl ether, iodomethyl octyl ether, iodomethyl nonyl ether, and iodomethyl decyl ether; and
iodomethyl benzyl ether.

Chloromethyl methyl ether, chloromethyl ethyl ether, chloromethyl propyl ether, chloromethyl butyl ether, and chloromethyl benzyl ether are preferred. By using said chloromethyl methyl ether, chloromethyl ethyl ether, chloromethyl propyl ether, chloromethyl butyl ether, and chloromethyl benzyl ether, a preferred availability may be ensured. Chloromethyl methyl ether and chloromethyl ethyl ether are more preferred. By using said chloromethyl methyl ether and chloromethyl ethyl ether, a more preferred availability may be ensured.

The halomethyl alkyl ether compound (16) may be a commercially available one, or may be synthesized in house (see, for example, Example 5 below).

The amount of the halomethyl alkyl ether compound (16) used, per mol of the 4-halo-1-pentanol compound (15), is preferably 1.0 to 3.0 mol, and more preferably 1.0 to 1.8 mol. By using said preferred amount and said more preferred amount, a preferred completion of the reaction and a more preferred completion of the reaction may be ensured.

A base may be incorporated in the alkoxymethylation reaction, if necessary.

Examples of the base include amines such as triethylamine, *N,N-*diisopropylethylamine, piperidine, pyrrolidine, pyridine, lutidine, 4-dimethylaminopyridine, *N,N*-dimethylaniline, *N,N*-diethylaniline, *N,N-*dipropylaniline, N,N-dibutylaniline, and 1,8-diazabicyclo[5.4.0]-7-undecene (DBU).

The base may be used alone or in combination thereof, if necessary. The base may be a commercially available one.

The amount of the base used, per mol of the 4-halo-1-pentanol compound (15), is preferably 0.1 to 4.0 mol, and more preferably 1.0 to 2.5 mol. By using said preferred amount and said more preferred amount, a preferred yield and economy and a more preferred yield and economy may be ensured.

A solvent may be incorporated in the alkoxymethylation, if necessary.

Examples of the solvent include general solvents such as, for example, ether solvents such as tetrahydrofuran (THF), 2-methyltetrahydrofuran (2-MeTHF), diethyl ether, dibutyl ether, 4-methyltetrahydropyran (MTHP), cyclopentylmethylether, and 1,4-dioxane; hydrocarbon solvents such as hexane, heptane, benzene, toluene, xylene, and cumene; polar solvents such as *N,N-*dimethylformamide (DMF), *N,N*-dimethylacetamide (DMAC), *N*-methylpyrrolidone (NMP), dimethyl sulfoxide (DMSO), γ-butyrolactone (GBL), acetonitrile, *N,N'*-dimethylpropylene urea (DMPU), hexamethylphosphoric triamide (HMPA), dichloromethane, and chloroform; and ester solvents such as methyl acetate, ethyl acetate, n-propyl acetate, and n-butyl acetate. Hydrocarbon solvents such as toluene and xylene; and ester solvents such as methyl acetate and ethyl acetate are preferred. By using said hydrocarbon solvents such as toluene and xylene; and ester solvents such as methyl acetate and ethyl acetate, a preferred reactivity may be ensured. The aforesaid alkoxymethylation may be carried out without a solvent to avoid reduced ease of preparation.

The solvent may be used alone or in combination thereof, if necessary. The solvent may be a commercially available one.

When a solvent is incorporated in the alkoxymethylation, the amount of the solvent used, per mol of the 4-halo-1-pentanol compound (15), is preferably more than 0 up to 1,500 g.

The reaction temperature of the alkoxymethylation varies, depending on the production scale, and is preferably -15 to 60°C, and more preferably 10 to 40°C. By using said preferred reaction temperature and said more preferred reaction temperature, a preferred reactivity and a more preferred reactivity may be ensured.

The reaction time of the alkoxymethylation varies, depending on the solvent used and/or production scale, and is preferably 0.5 to 100 hours. By using said preferred reaction time, a preferred reactivity may be ensured.

### IV. Process for preparing 4,6,8,10,16-pentamethyldocosane (11)

One target compound of the present invention, 4,6,8,10,16-pentamethyldocosane of the following general formula (11), is prepared from the aforesaid nucleophilic reagent, 8-alkoxymethoxy-1,3,5-trimethyloctyl compound (2: *n* = 3), according to the preparation process of the following chemical reaction formula:

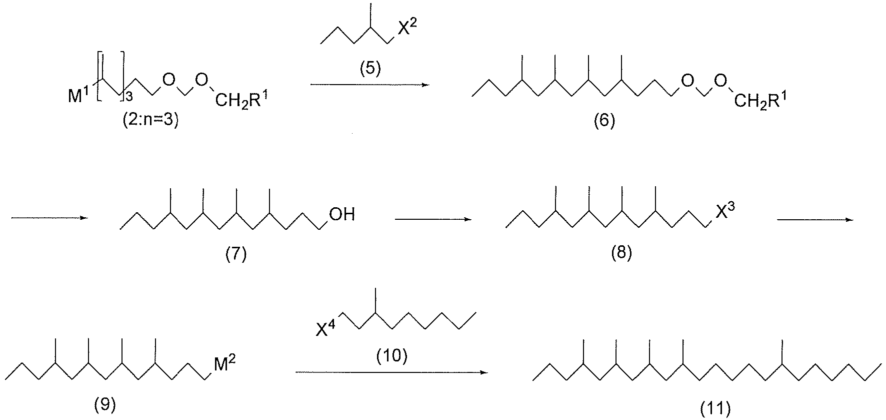

Specifically, the nucleophilic reagent, 8-alkoxymethoxy-1,3,5-trimethyloctyl compound (2: *n =* 3), is subjected to a coupling reaction with the 1-halo-2-methylpentane compound (5) to form the 4,6,8,10-tetramethyltridecyl alkoxymethyl ether compound (6). The 4,6,8,10-tetramethyltridecyl alkoxymethyl ether compound (6) is subjected to a dealkoxymethylation reaction to form 4,6,8,10-tetramethyltridecanol (7). The 4,6,8,10-tetramethyltridecanol (7) is then subjected to a halogenation reaction to form the 1-halo-4,6,8,10-tetramethyltridecane compound (8). The 1-halo-4,6,8,10-tetramethyltridecane compound (8) is then converted into the nucleophilic reagent, 4,6,8,10-tetramethyltridecyl compound (9). The nucleophilic reagent, 4,6,8,10-tetramethyltridecyl compound (9), is then subjected to a coupling reaction with the 1-halo-3-methylnonane compound (10) to obtain 4,6,8,10,16-pentamethyldocosane (11).

### (i) Process for preparing 4,6,8,10-tetramethyltridecyl alkoxymethyl ether compound (6)

(a) The aforesaid 4,6,8,10-tetramethyltridecyl alkoxymethyl ether compound (6) will be described in detail below.

The 4,6,8,10-tetramethyltridecyl alkoxymethyl ether compound (6) is represented by the following general formula (6):

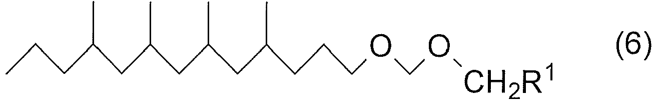

In the general formula (6) above, R¹ is as defined for the general formulas (1) and (1') above. When R¹ is an n-alkyl group, R¹ has 1 to 9 carbon atoms, and preferably 1 to 4 carbon atoms.

Specific examples of the 4,6,8,10-tetramethyltridecyl alkoxymethyl ether compound (6) include 4,6,8,10-tetramethyltridecyl methoxymethyl ether, 4,6,8,10-tetramethyltridecyl ethoxymethyl ether, 4,6,8,10-tetramethyltridecyl propyloxymethyl ether, 4,6,8,10-tetramethyltridecyl butyloxymethyl ether, 4,6,8,10-tetramethyltridecyl pentyloxymethyl ether, 4,6,8,10-tetramethyltridecyl hexyloxymethyl ether, 4,6,8,10-tetramethyltridecyl heptyloxymethyl ether, 4,6,8,10-tetramethyltridecyl octyloxymethyl ether, 4,6,8,10-tetramethyltridecyl nonyloxymethyl ether, 4,6,8,10-tetramethyltridecyl decyloxymethyl ether, and 4,6,8,10-tetramethyltridecyl benzyloxymethyl ether.

(b) A process for preparing the aforesaid 4,6,8,10-tetramethyltridecyl alkoxymethyl ether compound (6) will be described in detail below.

The 4,6,8,10-tetramethyltridecyl alkoxymethyl ether compound (6) may be prepared from, for example, a nucleophilic reagent, 8-alkoxymethoxy-1,3,5-trimethyloctyl compound, of the following general formula (2: *n* = 3), and a 1-halo-2-methylpentane compound of the following general formula (5), as shown in the following chemical reaction formula:

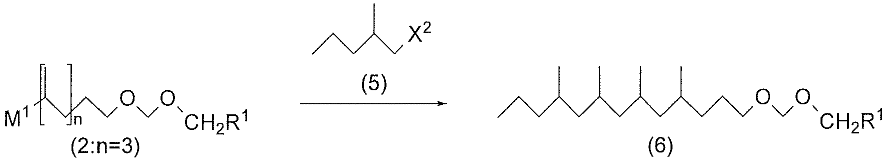

The preparation process includes a step of subjecting the nucleophilic reagent, 8-alkoxymethoxy-1,3,5-trimethyloctyl compound (2: *n* = 3), to a coupling reaction with the 1-halo-2-methylpentane compound (5).

The aforesaid nucleophilic reagent, 8-alkoxymethoxy-1,3,5-trimethyloctyl compound (2: *n =* 3), will be described in detail below.

The nucleophilic reagent, 8-alkoxymethoxy-1,3,5-trimethyloctyl compound (2: *n =* 3), is represented by the following general formula (2: *n =* 3):

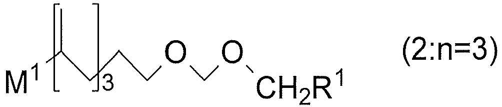

In the general formula (2: *n* = 3) above, M¹ represents Li, MgZ¹, CuZ¹, or CuLiZ¹, and Z¹ represents a halogen atom or a 8-alkoxymethoxy-1,3,5-trimethyloctyl group. Examples of the halogen atom Z¹ include a chlorine atom, a bromine atom, and an iodine atom. A chlorine atom and a bromine atom are preferred. By using said chlorine atom and bromine atom, a preferred reactivity may be ensured. A chlorine atom is particularly preferred. By using said chlorine atom, a particularly preferred reactivity may be ensured.

When M¹ is MgZ¹ in the general formula (2: *n* = 3) above, the nucleophilic reagent, 8-alkoxymethoxy-1,3,5-trimethyloctyl compound (2: *n* = 3, M^{1B} = MgZ^{1B}), is a Grignard reagent.

Specific examples of the nucleophilic reagent, 8-alkoxymethoxy-1,3,5-trimethyloctyl compound (2: *n* = 3), include the following examples:
8-alkoxymethoxy-1,3,5-trimethyloctyllithium reagents such as 8-methoxymethoxy-1,3,5-trimethyloctyllithium, 8-ethoxymethoxy-1,3,5-trimethyloctyllithium, 8-propyloxymethoxy-1,3,5-trimethyloctyllithium, and 8-butyloxymethoxy-1,3,5-trimethyloctyllithium;
8-alkoxymethoxy-1,3,5-trimethyloctylmagnesium halide reagents (when M¹ = MgZ¹) such as 8-methoxymethoxy-1,3,5-trimethyloctylmagnesium chloride, 8-ethoxymethoxy-1,3,5-trimethyloctylmagnesium chloride, 8-propyloxymethoxy-1,3,5-trimethyloctylmagnesium chloride, 8-butyloxymethoxy-1,3,5-trimethyloctylmagnesium chloride, 8-pentyloxymethoxy-1,3,5-trimethyloctylmagnesium chloride, 8-hexyloxymethoxy-1,3,5-trimethyloctylmagnesium chloride, 8-heptyloxymethoxy-1,3,5-trimethyloctylmagnesium chloride, 8-octyloxymethoxy-1,3,5-trimethyloctylmagnesium chloride, 8-nonyloxymethoxy-1,3,5-trimethyloctylmagnesium chloride, 8-benzyloxymethoxy-1,3,5-trimethyloctylmagnesium chloride, 8-methoxymethoxy-1,3,5-trimethyloctylmagnesium bromide, 8-ethoxymethoxy-1,3,5-trimethyloctylmagnesium bromide, 8-propyloxymethoxy-1,3,5-trimethyloctylmagnesium bromide, 8-butyloxymethoxy-1,3,5-trimethyloctylmagnesium bromide, 8-pentyloxymethoxy-1,3,5-trimethyloctylmagnesium bromide, 8-hexyloxymethoxy-1,3,5-trimethyloctylmagnesium bromide, 8-heptyloxymethoxy-1,3,5-trimethyloctylmagnesium bromide, 8-octyloxymethoxy-1,3,5-trimethyloctylmagnesium bromide, 8-nonyloxymethoxy-1,3,5-trimethyloctylmagnesium bromide, 8-benzyloxymethoxy-1,3,5-trimethyloctylmagnesium bromide, 8-methoxymethoxy-1,3,5-trimethyloctylmagnesium iodide, 8-ethoxymethoxy-1,3,5-trimethyloctylmagnesium iodide, 8-propyloxymethoxy-1,3,5-trimethyloctylmagnesium iodide, 8-butyloxymethoxy-1,3,5-trimethyloctylmagnesium iodide, 8-pentyloxymethoxy-1,3,5-trimethyloctylmagnesium iodide, 8-hexyloxymethoxy-1,3,5-trimethyloctylmagnesium iodide, 8-heptyloxymethoxy-1,3,5-trimethyloctylmagnesium iodide, 8-octyloxymethoxy-1,3,5-trimethyloctylmagnesium iodide, 8-nonyloxymethoxy-1,3,5-trimethyloctylmagnesium iodide, and 8-benzyloxymethoxy-1,3,5-trimethyloctylmagnesium iodide (i.e., Grignard reagents);
bis[8-alkoxymethoxy-1,3,5-trimethyloctyl]cuprates (when M¹ = CuZ¹) such as bis[8-methoxymethoxy-1,3,5-trimethyloctyl]cuprate, bis[8-ethoxymethoxy-1,3,5-trimethyloctyl]cuprate, bis[8-propyloxymethoxy-1,3,5-trimethyloctyl]cuprate, and bis[8-butyloxymethoxy-1,3,5-trimethyloctyl]cuprate; and
Gilman reagents (when M¹ = CuLiZ¹) such as lithium bis[8-alkoxymethoxy-1,3,5-trimethyloctyl]cuprates such as lithium bis[8-methoxymethoxy-1,3,5-trimethyloctyl]cuprate, lithium bis[8-ethoxymethoxy-1,3,5-trimethyloctyl]cuprate, lithium bis[8-propyloxymethoxy-1,3,5-trimethyloctyl]cuprate, and lithium bis[8-butyloxymethoxy-1,3,5-trimethyloctyl]cuprate (i.e., Gilman reagents).

Grignard reagents such as 8-alkoxymethoxy-1,3,5-trimethyloctylmagnesium halide reagents are preferred. By using said Grignard reagents such as 8-alkoxymethoxy-1,3,5-trimethyloctylmagnesium halide reagents, a preferred reactivity may be ensured.

The aforesaid 1-halo-2-methylpentane compound (5) will be described in detail below.

The 1-halo-2-methylpentane compound (5) is represented by the following general formula (5):

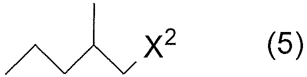

In the general formula (5) above, X² represents a halogen atom. Examples of the halogen atom X² include a chlorine atom, a bromine atom, and an iodine atom. A bromine atom and an iodine atom are preferred. By using said bromine atom and iodine atom, a preferred reactivity may be ensured. A bromine atom is particularly preferred. By using said bromine atom, a particularly preferred reactivity may be ensured.

Specific examples of the 1-halo-2-methylpentane compound (5) include 1-chloro-2-methylpentane, 1-bromo-2-methylpentane, and 1-iodo-2-methylpentane. 1-Bromo-2-methylpentane and 1-iodo-2-methylpentane are preferred. By using said 1-bromo-2-methylpentane and 1-iodo-2-methylpentane, a preferred yield may be ensured.

The 1-halo-2-methylpentane compound (5) may be used alone or in combination thereof, if necessary. The 1-halo-2-methylpentane compound (5) may be a commercially available one, or may be synthesized in house.

The 1-halo-2-methylpentane compound (5) may be prepared by, for example, subjecting 2-methylpentanol to halogenation.

The coupling reaction of the nucleophilic reagent, 8-alkoxymethoxy-1,3,5-trimethyloctyl compound (2: *n* = 3), with the 1-halo-2-methylpentane compound (5) will be described in detail below.

In the coupling reaction, the amount of the nucleophilic reagent, 8-alkoxymethoxy-1,3,5-trimethyloctyl compound (2: *n* = 3), used, per mol of the 1-halo-2-methylpentane compound (5), is preferably 0.8 to 1.4 mol. By using said preferred amount, preferred economy may be ensured.

A solvent may be incorporated in the coupling reaction, if necessary.

Examples of the solvent include general solvents such as, for example, ether solvents such as tetrahydrofuran (THF), 2-methyltetrahydrofuran (2-MeTHF), diethyl ether, dibutyl ether, 4-methyltetrahydropyran (MTHP), cyclopentylmethylether, and 1,4-dioxane; hydrocarbon solvents such as hexane, heptane, benzene, toluene, xylene, and cumene; and polar solvents such as *N,N*-dimethylformamide (DMF), *N,N-*dimethylacetamide (DMAC), *N*-methylpyrrolidone (NMP), dimethyl sulfoxide (DMSO), γ-butyrolactone (GBL), acetonitrile, *N,N*'-dimethylpropylene urea (DMPU), hexamethylphosphoric triamide (HMPA), dichloromethane, and chloroform. Hydrocarbon solvents such as toluene and xylene; ether solvents such as tetrahydrofuran, 2-methyltetrahydrofuran, and 4-methyltetrahydropyran; and acetonitrile are preferred. By using said hydrocarbon solvents such as toluene and xylene; ether solvents such as tetrahydrofuran, 2-methyltetrahydrofuran, and 4-methyltetrahydropyran; and acetonitrile, a preferred reactivity may be ensured. Tetrahydrofuran, 2-methyltetrahydrofuran, toluene, and xylene are more preferred. By using said tetrahydrofuran, 2-methyltetrahydrofuran, toluene, and xylene, a more preferred reactivity may be ensured.

The solvent may be used alone or in combination thereof, if necessary. The solvent may be a commercially available one.

The amount of the solvent used, per mol of the 1-halo-2-methylpentane compound (5), is preferably 30 to 5,000 g, and more preferably 50 to 3,000 g. By using said preferred amount and said more preferred amount, a preferred reactivity and a more preferred reactivity may be ensured.

The coupling reaction may be carried out in the presence of a catalyst, if necessary. Examples of the catalyst include cuprous halides such as cuprous chloride, cuprous bromide, and cuprous iodide; and cupric halides such as cupric chloride, cupric bromide, and cupric iodide. Cuprous halides are preferred. By using said cuprous halides, a preferred reactivity may be ensured. Cuprous iodide is more preferred. By using said cuprous iodide, a more preferred reactivity may be ensured.

The catalyst may be used alone or in combination thereof, if necessary. The catalyst may be a commercially available one.

The amount of the catalyst used, per mol of the 1-halo-2-methylpentane compound (5), is preferably 0.0003 to 0.3 mol, and more preferably 0.001 to 0.1 mol. By using said preferred amount and said more preferred amount, a preferred reaction rate and/or post-treatment and a more preferred reaction rate and/or post-treatment may be ensured.

When the coupling reaction is carried out in the presence of the catalyst, a co-catalyst may be added, if necessary. Examples of the co-catalyst include phosphorus compounds such as trialkyl phosphite compounds having 3 to 9 carbon atoms such as triethyl phosphite; and triarylphosphine compounds having 18 to 21 carbon atoms such as triphenylphosphine. Trialkyl phosphite compounds are preferred. By using said trialkyl phosphite compounds, a preferred reactivity may be ensured.

The co-catalyst may be used alone or in combination thereof, if necessary. The co-catalyst may be a commercially available one.

The amount of the co-catalyst used, per mol of the 1-halo-2-methylpentane compound (5), is preferably 0.001 to 0.500 mol, and more preferably 0.005 to 0.200 mol. By using said preferred amount and said more preferred amount, a preferred reactivity and a more preferred reactivity may be ensured.

When the coupling reaction is carried out in the presence of the catalyst, a lithium halide may be added, if necessary.

Examples of the lithium halide include lithium chloride, lithium bromide, and lithium iodide. Lithium chloride is preferred. By using said lithium chloride, a preferred reactivity may be ensured.

The amount of the lithium halide used in the coupling reaction, per mol of the 1-halo-2-methylpentane compound (5), is preferably 0.005 to 0.250 mol. By using said preferred amount, a preferred reactivity may be ensured.

The reaction temperature of the coupling reaction varies, depending on the nucleophilic reagent, 8-alkoxymethoxy-1,3,5-trimethyloctyl compound (2: *n* = 3), used, and is preferably -78 to 70°C, more preferably -20 to 50°C, and most preferably 5 to 35°C. By using said preferred reaction temperature, said more preferred reaction temperature, and said most preferred reaction temperature, a preferred reactivity, a more preferred reactivity, and a most preferred reactivity may be ensured.

The reaction time of the coupling reaction varies, depending on the solvent used and/or production scale, and is preferably 0.5 to 100 hours. By using said preferred reaction time, a preferred reactivity may be ensured.

### (ii) Process for preparing 4,6,8,10-tetramethyltridecanol (7)

A process for preparing the 4,6,8,10-tetramethyltridecanol (7) will be explained in detail below.

The 4,6,8,10-tetramethyltridecanol (7) may be prepared, for example, from the aforesaid 4,6,8,10-tetramethyltridecyl alkoxymethyl ether compound (6), as shown in the following chemical reaction formula:

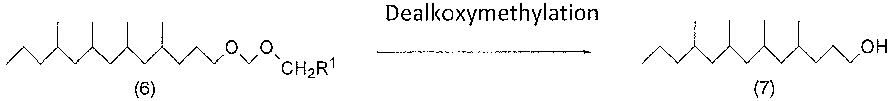

The preparation process includes a step of subjecting the 4,6,8,10-tetramethyltridecyl alkoxymethyl ether compound (6) to a dealkoxymethylation reaction.

Optimal conditions of the dealkoxymethylation reaction vary, depending on R¹ of the general formula (6) above. When R¹ is a phenyl group, the dealkoxymethylation may be carried out, for example, under Birch reduction conditions such that sodium is used in liquid ammonia. When R¹ is a hydrogen atom or an n-alkyl group such as a methyl group, an ethyl group, a propyl group, or a butyl group, the dealkoxymethylation may be carried out using an acid, water, or an alcohol compound (17).

Examples of the aforesaid acid include inorganic acids such as hydrochloric acid and hydrobromic acid; sulfonic acids such as p-toluenesulfonic acid and benzenesulfonic acid; organic acids such as trifluoroacetic acid, acetic acid, formic acid, and oxalic acid; and Lewis acids such as iodotrimethylsilane and titanium tetrachloride. *P-*toluenesulfonic acid, benzenesulfonic acid, hydrochloric acid, and hydrobromic acid are preferred. By using said *p*-toluenesulfonic acid, benzenesulfonic acid, hydrochloric acid, and hydrobromic acid, a preferred suppression of side reactions may be ensured. *P-*toluenesulfonic acid, hydrochloric acid, and hydrobromic acid are particularly preferred. By using said p-toluenesulfonic acid, hydrochloric acid, and hydrobromic acid, a particularly preferred suppression of side reactions may be ensured.

The acid may be used alone or in combination thereof, if necessary. The acid may be a commercially available one.

The amount of the acid used, per mol of the 4,6,8,10-tetramethyltridecyl alkoxymethyl ether compound (6), is preferably 0.0001 to 10.0 mol, and more preferably 0.001 to 1.0 mol.

The alcohol compound (17) is represented by the following general formula (17):

R⁴OH (17)

R⁴ represents a monovalent hydrocarbon group having 1 to 15 carbon atoms, and preferably 1 to 6 carbon atoms. A monovalent hydrocarbon group having 1 to 6 carbon atoms is preferred. By using said monovalent hydrocarbon group having 1 to 6 carbon atoms, a preferred price or availability may be ensured. Examples of the monovalent hydrocarbon group include linear saturated hydrocarbon groups such as a methyl group, an ethyl group, an n-propyl group, an n-butyl group, an n-pentyl group, an n-hexyl group, an n-heptyl group, an n-octyl group, an n-nonyl group, an n-decyl group, an n-undecyl group, and an n-dodecyl group; branched saturated hydrocarbon groups such as an isopropyl group, a *sec*-butyl group, and a 2-methylbutyl group; linear unsaturated hydrocarbon groups such as a 2-propenyl group; branched unsaturated hydrocarbon groups such as a 2-methyl-2-propenyl group; cyclic saturated hydrocarbon groups such as a cyclopropyl group; and isomers thereof. A part of the hydrogen atoms in the hydrocarbon groups may be substituted with a methyl group, an ethyl group, or a hydroxy group.

The monovalent hydrocarbon group is preferably a methyl group, an ethyl group, an n-propyl group, and an n-butyl group. By using said methyl group, ethyl group, n-propyl group, and n-butyl group, a preferred ease of handling of the alcohol compound (17) (encompassing, for example, the price and ease of removability due to the low boiling point) and/or quantity of starting materials due to the alcohol compound use may be ensured.

Examples of the alcohol compound (17) include linear alcohols such as methanol, ethanol, n-propanol, n-butanol, n-pentanol, n-hexanol, n-heptanol, n-octanol, n-nonanol, n-decanol, n-undecanol, n-dodecanol, n-tridecanol, n-tetradecanol, and n-pentadecanol; branched alcohols such as isopropanol and 2-butanol; and diols such as ethylene glycol, propylene glycol, 2-methyl-1,3-propanediol, 2,2-dimethyl-1,3-propanediol, 1,2-dimethyl-1,3-propanediol, 1,3-dimethyl-1,3-propanediol, and 2-methyl-1,4-butanediol. Methanol and ethanol are preferred. By using said methanol and ethanol, a preferred reactivity may be ensured. Methanol is particularly preferred. By using said methanol, a particularly preferred reactivity may be ensured.

The alcohol compound (17) may be used alone or in combination thereof, if necessary. The alcohol compound (17) may be a commercially available one.

The amount of the alcohol compound (17) used, per mol of the 4,6,8,10-tetramethyltridecyl alkoxymethyl ether compound (6), is preferably 1 to 1,000 mol, and more preferably 1 to 100 mol. By using said preferred amount and said more preferred amount, a preferred reactivity and a more preferred reactivity may be ensured.

When water is used, the amount of the water used, per mol of the 4,6,8,10-tetramethyltridecyl alkoxymethyl ether compound (6), is preferably more than 0 up to 1,000 mol, and more preferably more than 0 up to 100 mol. By using said preferred amount and said more preferred amount, a preferred reactivity and a more preferred reactivity may be ensured.

A solvent other than the alcohol compound (17) may be incorporated in the dealkoxymethylation reaction, if necessary.

Examples of the solvent include general solvents such as, for example, ether solvents such as tetrahydrofuran (THF), 2-methyltetrahydrofuran (2-MeTHF), diethyl ether, dibutyl ether, 4-methyltetrahydropyran (MTHP), cyclopentylmethylether, and 1,4-dioxane; hydrocarbon solvents such as hexane, heptane, benzene, toluene, xylene, and cumene; polar solvents such as *N,N-*dimethylformamide (DMF), *N,N*-dimethylacetamide (DMAC), *N*-methylpyrrolidone (NMP), dimethyl sulfoxide (DMSO), γ-butyrolactone (GBL), acetonitrile, *N,N'*-dimethylpropylene urea (DMPU), hexamethylphosphoric triamide (HMPA), dichloromethane, and chloroform; ester solvents such as methyl acetate, ethyl acetate, n-propyl acetate, and n-butyl acetate; and water.

The solvent may be used alone or in combination thereof, if necessary. The solvent may be a commercially available one.

When a solvent is incorporated in the dealkoxymethylation reaction, the amount of the solvent used, per mol of the 4,6,8,10-tetramethyltridecyl alkoxymethyl ether compound (6), is preferably more than 0 up to 2,000 g, and more preferably more than 0 up to 500 g.

The solvent may occupy part of the reactor space, which reduces the space for the starting materials, and reduces productivity. Therefore, the dealkoxymethylation may be carried out without a solvent.

The reaction temperature of the dealkoxymethylation reaction varies, depending on the 4,6,8,10-tetramethyltridecyl alkoxymethyl ether compound (6) used, and is preferably -5 to 180°C, and more preferably 10 to 130°C. By using said preferred reaction temperature and said more preferred reaction temperature, a preferred reactivity and a more preferred reactivity may be ensured.

The reaction time of the dealkoxymethylation reaction varies, depending on the 4,6,8,10-tetramethyltridecyl alkoxymethyl ether compound (6) used or production scale, and is preferably 0.5 to 100 hours. By using said preferred reaction time, a preferred reactivity may be ensured.

In the dealkoxymethylation reaction, by-produced alkoxymethoxymethane may be distilled off from the reaction system, if necessary. By removing the alkoxymethoxymethane from the reaction system, the equilibrium may be shifted to the product side to reduce the reaction time.

### (iii) 1-Halo-4,6,8,10-tetramethyltridecane compound (8) and process for preparing 1-halo-4,6,8,10-tetramethyltridecane compound (8)

(a) The aforesaid 1-halo-4,6,8,10-tetramethyltridecane compound (8) will be described in detail below.

The 1-halo-4,6,8,10-tetramethyltridecane compound (8) is represented by the following general formula (8): 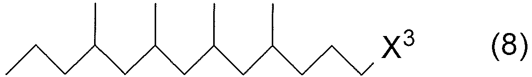 wherein X³ represents a halogen atom.

In the general formula (8) above, X³ represents a halogen atom. Examples of the halogen atom X³ include a chlorine atom, a bromine atom, and an iodine atom. A chlorine atom and a bromine atom are preferred. By using said chlorine atom and bromine atom, a preferred reactivity may be ensured. A chlorine atom is particularly preferred. By using said chlorine atom, a particularly preferred reactivity may be ensured.

(b) A process for preparing the aforesaid 1-halo-4,6,8,10-tetramethyltridecane compound (8) will be explained in detail below.

The 1-halo-4,6,8,10-tetramethyltridecane compound (8) may be prepared, for example, from 4,6,8,10-tetramethyltridecanol of the following general formula (7), as shown in the following chemical reaction formula:

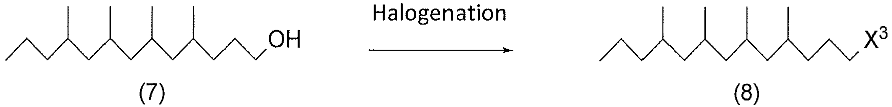

The preparation process includes a step of subjecting the 4,6,8,10-tetramethyltridecanol (7) to a halogenation reaction.

The halogenation reaction may be carried out by, for example, a process of tosylating the hydroxy group with a p-toluenesulfonyl halide compound and then halogenating with the metal salt, lithium halide compound; or a process of directly halogenating the hydroxy group with a halogenating agent.

Examples of the halogenating agent include halogens such as chlorine, bromine, and iodine; hydrogen halide compounds such as hydrogen chloride, hydrogen bromide, and hydrogen iodide; methanesulfonyl halide compounds such as methanesulfonyl chloride, methanesulfonyl bromide, and methanesulfonyl iodide; benzenesulfonyl halide compounds such as benzenesulfonyl chloride, benzenesulfonyl bromide, and benzenesulfonyl iodide; *p*-toluenesulfonyl halide compounds such as *p*-toluenesulfonyl chloride, *p*-toluenesulfonyl bromide, and *p*-toluenesulfonyl iodide; thionyl halide compounds such as thionyl chloride, thionyl bromide, and thionyl iodide; phosphorus halide compounds such as phosphorus trichloride, phosphorus pentachloride, and phosphorus tribromide; carbon tetrahalide compounds such as carbon tetrachloride, carbon tetrabromide, and carbon tetraiodide; alkylsilyl halide compounds such as trimethylsilyl chloride, trimethylsilyl bromide, trimethylsilyl iodide, triethylsilyl chloride, triethylsilyl bromide, triethylsilyl iodide, triisopropylsilyl chloride, triisopropylsilyl bromide, triisopropylsilyl iodide, tert-butyldimethylsilyl chloride, *tert*-butyldimethylsilyl bromide, and tert-butyldimethylsilyl iodide; oxalyl halide compounds such as oxalyl chloride, oxalyl bromide, and oxalyl iodide; and *N*-halosuccinimide compounds such as *N*-chlorosuccinimide, *N-*bromosuccinimide, and *N*-iodosuccinimide. Methanesulfonyl halide compounds, benzenesulfonyl halide compounds, *p*-toluenesulfonyl halide compounds, and thionyl halide compounds are preferred. By using said methanesulfonyl halide compounds, benzenesulfonyl halide compounds, *p*-toluenesulfonyl halide compounds, and thionyl halide compounds, a preferred suppression of side reactions may be ensured. Methanesulfonyl halide compounds, benzenesulfonyl halide compounds, and thionyl halide compounds are particularly preferred. By using said methanesulfonyl halide compounds, benzenesulfonyl halide compounds, and thionyl halide compounds, a particularly preferred suppression of side reactions may be ensured.

The halogenating agent may be used alone or in combination thereof, if necessary. The halogenating agent may be a commercially available one.

The amount of the halogenating agent used, per mol of the 4,6,8,10-tetramethyltridecanol (7), is preferably 0.8 to 5.0 mol, and more preferably 1.0 to 2.5 mol. By using said preferred amount and said more preferred amount, a preferred reactivity and a more preferred reactivity may be ensured.

A base may be incorporated in the halogenation reaction, if necessary.

Examples of the base include hydroxides such as sodium hydroxide, potassium hydroxide, calcium hydroxide, and magnesium hydroxide; carbonates such as sodium carbonate, potassium carbonate, calcium carbonate, and magnesium carbonate; and amines such as triethylamine, *N,N*-diisopropylethylamine, piperidine, pyrrolidine, pyridine, lutidine, 4-dimethylaminopyridine, *N,N*-dimethylaniline, *N,N*-diethylaniline, and 1,8-diazabicyclo[5.4.0]-7-undecene (DBU).

When a methanesulfonyl halide compound, a benzenesulfonyl halide compound, a *p*-toluenesulfonyl halide compound, or the like is used as the halogenating agent, the base is preferably amines, and more preferably pyridines such as pyridine, lutidine, and 4-dimethylaminopyridine.

When a thionyl halide compound is used as the halogenating agent, the base is preferably amines, and more preferably trialkylamines such as triethylamine.

The base may be used alone or in combination thereof, if necessary. The base may be a commercially available one.

When the base is used, the amount of the base used, per mol of the 4,6,8,10-tetramethyltridecanol (7), is preferably more than 0 up to 8.0 mol, and more preferably more than 0 up to 3.0 mol. By using said preferred amount and said more preferred amount, a preferred yield and/or economy and a more preferred yield and/or economy may be ensured.

A metal salt may be incorporated in the halogenation reaction, if necessary.

Examples of the metal salt include lithium salts such as lithium chloride, lithium bromide, and lithium iodide; sodium salts such as sodium chloride, sodium bromide, and sodium iodide; potassium salts such as potassium chloride, potassium bromide, and potassium iodide; calcium salts such as calcium chloride, calcium bromide, and calcium iodide; and magnesium salts such as magnesium chloride, magnesium bromide, and magnesium iodide.

When the halogenation with the metal salt, lithium halide compound, is carried out after the tosylation, the reaction may be carried out with, for example, lithium salts such as lithium chloride, lithium bromide, and lithium iodide.

The metal salt may be used alone or in combination thereof, if necessary. The metal salt may be a commercially available one.

When the metal salt is used, the amount of the metal salt used, per mol of the 4,6,8,10-tetramethyltridecanol (7), is preferably more than 0 up to 30.0 mol, and more preferably more than 0 up to 5.0 mol. By using said preferred amount and said more preferred amount, a preferred reactivity and a more preferred reactivity may be ensured.

Although the metal salt increases the concentration of halide ions in the reaction system to thereby enhance the reactivity, it is preferred not to incorporate the metal salt in the reaction. By not incorporating the metal salt, preferred economy and/or environmental protection may be ensured.

A solvent may be incorporated in the halogenation reaction, if necessary.

Examples of the solvent include general solvents such as, for example, ether solvents such as tetrahydrofuran (THF), 2-methyltetrahydrofuran (2-MeTHF), diethyl ether, dibutyl ether, 4-methyltetrahydropyran (MTHP), cyclopentylmethylether, and 1,4-dioxane; hydrocarbon solvents such as hexane, heptane, benzene, toluene, xylene, and cumene; polar solvents such as *N,N-*dimethylformamide (DMF), *N,N*-dimethylacetamide (DMAC), *N*-methylpyrrolidone (NMP), dimethyl sulfoxide (DMSO), γ-butyrolactone (GBL), acetonitrile, acetone, *N,N*'-dimethylpropylene urea (DMPU), hexamethylphosphoric triamide (HMPA), dichloromethane, and chloroform; and ester solvents such as methyl acetate, ethyl acetate, n-propyl acetate, and n-butyl acetate. 2-Methyltetrahydrofuran, 4-methyltetrahydropyran, dichloromethane, chloroform, γ-butyrolactone, *N*-methylpyrrolidone, *N,N-*dimethylformamide, *N,N*-dimethylacetamide, and acetonitrile are preferred. By using said 2-methyltetrahydrofuran, 4-methyltetrahydropyran, dichloromethane, chloroform, γ-butyrolactone, *N-*methylpyrrolidone, *N,N*-dimethylformamide, *N,N*-dimethylacetamide, and acetonitrile, a preferred reactivity may be ensured. 2-Methyltetrahydrofuran, γ-butyrolactone, and acetonitrile are particularly preferred. By using said 2-methyltetrahydrofuran, γ-butyrolactone, and acetonitrile, particularly preferred safety may be ensured.

The solvent may be used alone or in combination thereof, if necessary. The solvent may be a commercially available one.

When the solvent is incorporated in the halogenation reaction, the amount of the solvent used, per mol of the 4,6,8,10-tetramethyltridecanol (7), is preferably more than 0 up to 3,000 g, and more preferably more than 0 up to 800 g.

The solvent may occupy part of the reactor space, which reduces the space for the starting materials, and reduces productivity. Therefore, the reaction may be carried out without a solvent, or with the base as the solvent.

The reaction temperature of the halogenation reaction varies, depending on the halogenating agent used, and is preferably 5 to 180°C, and more preferably 20 to 120°C. By using said preferred reaction temperature and said more preferred reaction temperature, a preferred reactivity and a more preferred reactivity may be ensured.

The reaction time of the halogenation reaction varies, depending on the halogenating agent used and/or production scale, and is preferably 0.5 to 100 hours. By using said preferred reaction time, a preferred reactivity may be ensured.

### (iv) Nucleophilic reagent, 4,6,8,10-tetramethyltridecyl compound (9), and process for preparing nucleophilic reagent, 4,6,8,10-tetramethyltridecyl compound (9)

A process for preparing the aforesaid nucleophilic reagent, 4,6,8,10-tetramethyltridecyl compound (9), will be explained in detail below.
(a) The nucleophilic reagent, 4,6,8,10-tetramethyltridecyl compound (9), will be described in detail below.

The nucleophilic reagent, 4,6,8,10-tetramethyltridecyl compound (9), is represented by the following general formula (9):

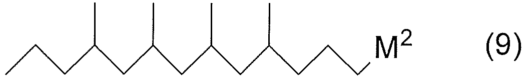

In the general formula (9) above, M² represents Li, MgZ², CuZ², or CuLiZ², and Z² represents a halogen atom or a 4,6,8,10-tetramethyltridecyl group. Examples of the halogen atom Z² include a chlorine atom, a bromine atom, and an iodine atom.

Specific examples of the nucleophilic reagent, 4,6,8,10-tetramethyltridecyl compound (9), include 4,6,8,10-tetramethyltridecyllithium; 4,6,8,10-tetramethyltridecylmagnesium halide reagents (when M² = MgZ²) such as 4,6,8,10-tetramethyltridecylmagnesium chloride, 4,6,8,10-tetramethyltridecylmagnesium bromide, and 4,6,8,10-tetramethyltridecylmagnesium iodide (i.e., Grignard reagents); bis[4,6,8,10-tetramethyltridecyl]cuprate (when M² = CuZ²); and Gilman reagents (when M² = CuLiZ²) such as lithium bis[4,6,8,10-tetramethyltridecyl]cuprate (i.e., Gilman reagents). Grignard reagents such as 4,6,8,10-tetramethyltridecylmagnesium halide reagents are preferred. By using said Grignard reagents such as 4,6,8,10-tetramethyltridecylmagnesium halide reagents, a preferred reactivity may be ensured.

(b) A process for preparing the aforesaid nucleophilic reagent, 4,6,8,10-tetramethyltridecyl compound (9), will be described in detail below.

The nucleophilic reagent, 4,6,8,10-tetramethyltridecyl compound (9), may be prepared in a conventional method or a process described below.

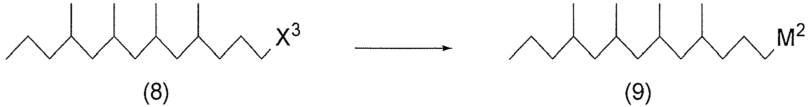

A process for preparing the nucleophilic reagent, 4,6,8,10-tetramethyltridecyl compound (9), when M² is MgZ², that is, the 4,6,8,10-tetramethyltridecylmagnesium halide reagent (9: M² = MgZ²) (i.e., a Grignard reagent) will be described in detail as an example.

The 4,6,8,10-tetramethyltridecylmagnesium halide reagent (9: M² = MgZ², Z² = halogen atom) may be prepared, for example, by reacting the aforesaid 1-halo-4,6,8,10-tetramethyltridecane compound (8) with magnesium in a solvent, as shown in the following chemical reaction formula:

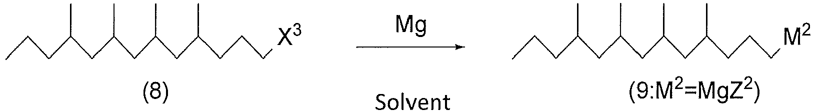

The amount of magnesium used, per mol of the 1-halo-4,6,8,10-tetramethyltridecane compound (8), is preferably 1.0 to 2.0 gram atoms. By using said preferred amount, a preferred completion of the reaction may be ensured.

Examples of the solvent include general solvents such as, for example, ether solvents such as tetrahydrofuran (THF), 2-methyltetrahydrofuran (2-MeTHF), diethyl ether, dibutyl ether, 4-methyltetrahydropyran (MTHP), cyclopentylmethylether, and 1,4-dioxane; and hydrocarbon solvents such as hexane, heptane, benzene, toluene, xylene, and cumene. Hydrocarbon solvents such as toluene and xylene; and ether solvents such as tetrahydrofuran, 2-methyltetrahydrofuran, and 4-methyltetrahydropyran are preferred. By using said hydrocarbon solvents such as toluene and xylene; and ether solvents such Solvent as tetrahydrofuran, 2-methyltetrahydrofuran, and 4-methyltetrahydropyran, a preferred reaction rate of forming the aforesaid Grignard reagent may be ensured. Tetrahydrofuran, 2-methyltetrahydrofuran, and 4-methyltetrahydropyran are more preferred. By using said tetrahydrofuran, 2-methyltetrahydrofuran, and 4-methyltetrahydropyran, a more preferred reaction rate of forming the aforesaid Grignard reagent may be ensured.

The solvent may be used alone or in combination thereof, if necessary. The solvent may be a commercially available one.

The amount of the solvent used, per mol of the 1-halo-4,6,8,10-tetramethyltridecane compound (8), is preferably 30 to 5,000 g, and more preferably 50 to 3,000 g. By using said preferred amount and said more preferred amount, a preferred reactivity and a more preferred reactivity may be ensured.

The reaction temperature varies, depending on the solvent used, and is preferably 30 to 120°C. By using said preferred reaction temperature, a preferred reactivity may be ensured.

The reaction time varies, depending on the solvent used and/or production scale, and is preferably 0.5 to 100 hours. By using said preferred reaction time, a preferred reactivity may be ensured.

### (v) 4,6,8,10,16-Pentamethyldocosane (11) and process for preparing 4,6,8,10,16-pentamethyldocosane (11)

(a) The 4,6,8,10,16-pentamethyldocosane (11) will be described in detail below.
   The 4,6,8,10,16-pentamethyldocosane (11) is represented by the following general formula (11): 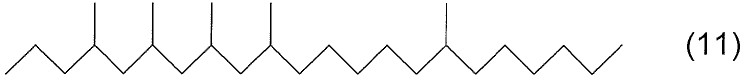
(b) A process for preparing the aforesaid 4,6,8,10,16-pentamethyldocosane (11) will be explained in detail below.

The 4,6,8,10,16-pentamethyldocosane (11) may be prepared, for example, from a nucleophilic reagent, 4,6,8,10-tetramethyltridecyl compound, of the following general formula (9) and a 1-halo-3-methylnonane compound of the following general formula (10), as shown in the following chemical reaction formula:

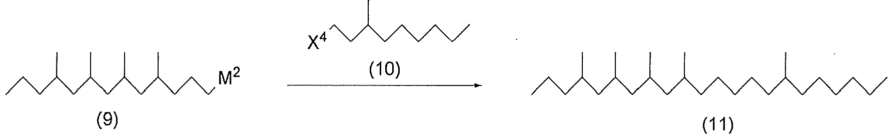

The preparation process includes a step of subjecting the nucleophilic reagent, 4,6,8,10-tetramethyltridecyl compound (9), to a coupling reaction with the 1-halo-3-methylnonane compound (10).

The aforesaid 1-halo-3-methylnonane compound (10) will be described in detail below.

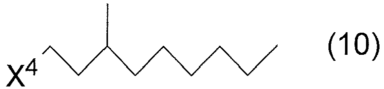

In the general formula (10) above, X⁴ represents a halogen atom. Examples of the halogen atom X⁴ include a chlorine atom, a bromine atom, and an iodine atom. A bromine atom and an iodine atom are preferred. By using said bromine atom and iodine atom, a preferred reactivity may be ensured. A bromine atom is particularly preferred. By using said bromine atom, a particularly preferred reactivity may be ensured.

Specific examples of the 1-halo-3-methylnonane compound (10) include 1-chloro-3-methylnonane, 1-bromo-3-methylnonane, and 1-iodo-3-methylnonane. 1-Bromo-3-methylnonane and 1-iodo-3-methylnonane are preferred. By using said 1-bromo-3-methylnonane and 1-iodo-3-methylnonane, a preferred yield may be ensured.

The 1-halo-3-methylnonane compound (10) may be used alone or in combination thereof, if necessary. The 1-halo-3-methylnonane compound (10) may be a commercially available one, or may be synthesized in house.

The 1-halo-3-methylnonane compound (10) may be prepared, for example, by subjecting 3-methylnonanol to a halogenation reaction.

The coupling reaction of the nucleophilic reagent, 4,6,8,10-tetramethyltridecyl compound (9), with the 1-halo-3-methylnonane compound (10) will be described in detail below.

In the coupling reaction, the amount of the nucleophilic reagent, 4,6,8,10-tetramethyltridecyl compound (9), used, per mol of the 1-halo-3-methylnonane compound (10), is preferably 0.8 to 1.4 mol. By using said preferred amount, preferred economy may be ensured.

A solvent may be incorporated in the coupling reaction, if necessary.

Examples of the solvent include general solvents such as, for example, ether solvents such as tetrahydrofuran (THF), 2-methyltetrahydrofuran (2-MeTHF), diethyl ether, dibutyl ether, 4-methyltetrahydropyran (MTHP), cyclopentylmethylether, and 1,4-dioxane; hydrocarbon solvents such as hexane, heptane, benzene, toluene, xylene, and cumene; and polar solvents such as *N,N*-dimethylformamide (DMF), *N,N-*dimethylacetamide (DMAC), *N*-methylpyrrolidone (NMP), dimethyl sulfoxide (DMSO), γ-butyrolactone (GBL), acetonitrile, *N,N*'-dimethylpropylene urea (DMPU), hexamethylphosphoric triamide (HMPA), dichloromethane, and chloroform. Hydrocarbon solvents such as toluene and xylene; ether solvents such as tetrahydrofuran, 2-methyltetrahydrofuran, and 4-methyltetrahydropyran; and acetonitrile are preferred. By using said hydrocarbon solvents such as toluene and xylene; ether solvents such as tetrahydrofuran, 2-methyltetrahydrofuran, and 4-methyltetrahydropyran; and acetonitrile, a preferred reactivity may be ensured. Tetrahydrofuran, 2-methyltetrahydrofuran, toluene, and xylene are more preferred. By using said tetrahydrofuran, 2-methyltetrahydrofuran, toluene, and xylene, a more preferred reactivity may be ensured.

The solvent may be used alone or in combination thereof, if necessary. The solvent may be a commercially available one.

The amount of the solvent used, per mol of the 1-halo-3-methylnonane compound (10), is preferably 30 to 5,000 g, and more preferably 50 to 3,000 g. By using said preferred amount and said more preferred amount, a preferred reactivity and a more preferred reactivity may be ensured.

The coupling reaction may be carried out in the presence of a catalyst, if necessary. Examples of the catalyst include cuprous halides such as cuprous chloride, cuprous bromide, and cuprous iodide; and cupric halides such as cupric chloride, cupric bromide, and cupric iodide. Cuprous halides are preferred. By using said cuprous halides, a preferred reactivity may be ensured. Cuprous chloride is more preferred. By using said cuprous chloride, a more preferred reactivity may be ensured.

The catalyst may be used alone or in combination thereof, if necessary. The catalyst may be a commercially available one.

The amount of the catalyst used, per mol of the 1-halo-3-methylnonane compound (10), is preferably 0.0003 to 0.3 mol, and more preferably 0.001 to 0.1 mol. By using said preferred amount and said more preferred amount, a preferred reaction rate and/or post-treatment and a more preferred reaction rate and/or post-treatment may be ensured.

When the coupling reaction is carried out in the presence of the catalyst, a co-catalyst may be added, if necessary. Examples of the co-catalyst include phosphorus compounds such as trialkyl phosphite compounds having 3 to 9 carbon atoms such as triethyl phosphite; and triarylphosphine compounds having 18 to 21 carbon atoms such as triphenylphosphine. Trialkyl phosphite compounds are preferred. By using said trialkyl phosphite compounds, a preferred reactivity may be ensured.

The co-catalyst may be used alone or in combination thereof, if necessary. The co-catalyst may be a commercially available one.

The amount of the co-catalyst used, per mol of the 1-halo-3-methylnonane compound (10), is preferably 0.001 to 0.500 mol, and more preferably 0.005 to 0.200 mol. By using said preferred amount and said more preferred amount, a preferred reactivity and a more preferred reactivity may be ensured.

When the coupling reaction is carried out in the presence of the catalyst, a lithium halide may be added, if necessary.

Examples of the lithium halide include lithium chloride, lithium bromide, and lithium iodide. Lithium chloride is preferred. By using said lithium chloride, a preferred reactivity may be ensured.

The lithium halide may be used alone or in combination thereof, if necessary. The lithium halide may be a commercially available one.

The amount of lithium halide used in the coupling reaction, per mol of the 1-halo-3-methylnonane compound (10), is preferably 0.005 to 0.250 mol. By using said preferred amount, a preferred reactivity may be ensured.

The reaction temperature of the coupling reaction varies, depending on the nucleophilic reagent, 4,6,8,10-tetramethyltridecyl compound (9), used, and is preferably -78 to 70°C, more preferably -20 to 50°C, and most preferably 5 to 35°C. By using said preferred reaction temperature, said more preferred reaction temperature, and said most preferred reaction temperature, a preferred reactivity, a more preferred reactivity, and a most preferred reactivity may be ensured.

The reaction time of the coupling reaction varies, depending on the solvent used and/or production scale, and is preferably 0.5 to 100 hours. By using said preferred reaction time, a preferred reactivity may be ensured.

Thus, according to the present invention, the cuticular hydrocarbon of *Antitragus parvulus,* 4,6,8,10,16-pentamethyldocosane (11), may be efficiently prepared from the synthetic intermediate, haloalkyl alkoxymethyl ether compound (2), with high ease of preparation and in fewer steps.

### EXAMPLES

The present invention will be described with reference to the following Examples. It should be noted that the present invention is not limited to or by the Examples.

The term "purity" as used herein means an area percentage in gas chromatography (GC), unless otherwise specified. The term "product ratio" means a ratio of area percentages in GC. The term "yield" is calculated from the area percentages determined by GC.

In the Examples, monitoring of the reactions and calculation of the yields were carried out in the following GC conditions.

GC conditions: GC: Capillary gas chromatograph GC-2014 (Shimadzu Corporation); column: DB-5, 0.25 µm x 0.25 mmϕ x 30 m; carrier gas: He (1.55 mL/min), detector: FID; column temperature: 150°C, elevated in a rate of 5°C/min, and up to 230°C; column: DB-WAX, 0.25 µm x 0.25 mmϕ x 30 m; carrier gas: He (1.55 mL/min), detector: FID; column temperature: 150°C, elevated in a rate of 5°C/min, and up to 230°C.

The yield was calculated according to the following equation in consideration of purities (% GC) of a starting material and a product. 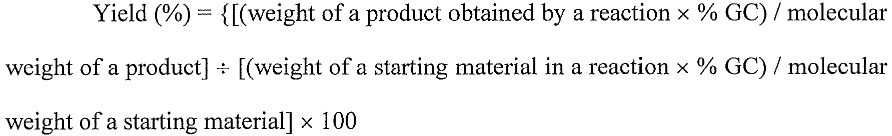 Yield (%) = {[(weight of a product obtained by a reaction × % GC)/molecular weight of a product] ÷ [(weight of a starting material in a reaction × % GC)/molecular weight of a starting material] × 100

THF represents tetrahydrofuran, GBL represents γ-butyrolactone, PO represents propylene oxide, Ph represents a phenyl group, and Et represents an ethyl group.

### Example 1: Preparation of 8-hydroxy-4,6-dimethylnonyl methoxymethyl ether (4: n+1 = 3 and R¹ = H)

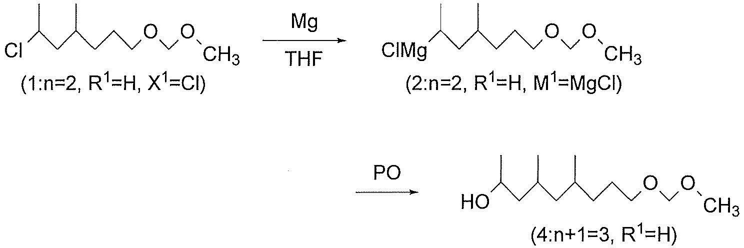

Magnesium (22.66 g, 0.98 gram atoms) and tetrahydrofuran (279.72 g) were placed in a reactor at a room temperature and stirred for 31 minutes at 60 to 65°C. 6-Chloro-4-methylheptyl methoxymethyl ether (1: *n =* 2, R¹ = H, X¹ = Cl) (220.00 g, 0.93 mol, purity 88.46%) was then added dropwise to the reactor at 60 to 75°C. After the completion of the dropwise addition, the reaction mixture was stirred for 2 hours at 75 to 80°C to obtain 6-methoxymethoxy-1,3-dimethylhexylmagnesium chloride (2: *n* = 2, R¹ = H, M¹ = MgCl).

Next, the internal temperature of the reactor was cooled to 0 to 10°C, and then cuprous chloride (0.19 g, 0.0019 mol) was added to the reactor, and propylene oxide was added dropwise at 5 to 30°C. After the completion of the dropwise addition, the reaction mixture was stirred for 3 hours at 15 to 25°C. An aqueous solution of acetic acid (prepared from acetic acid (127.25 g) and water (381.78 g)) and hexane (90.49 g) were then added to the reaction mixture, followed by phase separation. The organic layer thus obtained was then concentrated at a reduced pressure, and the resulting concentrate was distilled at a reduced pressure to obtain 8-hydroxy-4,6-dimethylnonyl methoxymethyl ether (4: *n*+1 = 3, R¹ = H) (182.15 g, 0.75 mol, purity 95.28%, b.p. = 120.0 to 127.8°C/0.36 kPa (2.7 mmHg)) with a yield of 80.11%.

The following is the spectrum data of 8-hydroxy-4,6-dimethylnonyl methoxymethyl ether (4: *n*+1 = 3, R¹ = H) thus prepared.

Nuclear magnetic resonance spectrum: ¹H-NMR (500 MHz, CDCl₃): δ = 0.80-0.90 (6H, m), 0.90-1.14 (2H, m), 1.14-1.20 (4H, m), 1.20-1.75 (8H, m), 3.34 (3H, s), 3.49 (2H, t-like, J = 6.9 Hz), 3.83-3.92 (1H, m), 4.60 (2H, s); ¹³C-NMR (125 MHz, CDCl₃): δ = 19.26, 19.31, 19.91, 20.05, 20.14, 20.68, 23.57, 23.78, 24.25, 26.70, 26.87, 27.10, 27.24, 27.26, 29.72, 32.81, 44.55, 44.94, 45.23, 45.60, 46.65, 47.67, 47.82, 55.04, 65.59, 65.98, 66.14, 68.13, 68.21, 96.33.

Mass spectrum: EI-mass spectrum (70 eV): m/z 231 (M⁺-1), 201, 139, 97, 85, 69, 57, 45, 29.

Infrared absorption spectrum: (D-ATR): v = 2957, 2927, 1461, 1378, 1304, 1214, 1153, 1111, 1045, 920.

### Example 2: Preparation of 8-chloro-4,6-dimethylnonyl methoxymethyl ether (1': n+1 = 3, R¹ = H, X¹ = Cl)

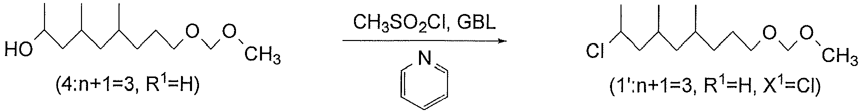

The 8-hydroxy-4,6-dimethylnonyl methoxymethyl ether (4: *n*+1 = 3, R¹ = H) (85.00 g, 0.35 mol, purity 95.28%) obtained in Example 1, pyridine (41.35 g, 0.52 mol), and GBL (100.00 g) were placed in a reactor and stirred for 26 minutes at 10°C.

Methanesulfonyl chloride (CH₃SO₂Cl) (47.90 g, 0.42 mol) was then added dropwise at 5 to 15°C. After the completion of the dropwise addition, the reaction mixture was heated to 60 to 65°C and stirred for 9 hours. After the completion of the stirring, water (87.13 g) and hexane (52.28 g) were added to the reaction mixture, followed by phase separation. The aqueous layer was removed to obtain the organic layer. The organic layer thus obtained was washed with an aqueous solution of acetic acid (prepared from acetic acid (3.67 g) and water (45.85 g)), and then washed with an aqueous solution of sodium bicarbonate (prepared from sodium bicarbonate (1.83 g) and water (45.85 g)). The organic layer thus obtained was concentrated at a reduced pressure, and the resulting concentrate was purified by silica gel column chromatography (hexane:ethyl acetate = 100:0 to 9:1) to obtain 8-chloro-4,6-dimethylnonyl methoxymethyl ether (1': *n*+1 = 3, R¹ = H, X¹ = Cl) (73.37 g, 0.27 mol, purity 93.15%) with a yield of 78.19%.

The following is the spectrum data of 8-chloro-4,6-dimethylnonyl methoxymethyl ether (1': *n*+1 = 3, R¹ = H, X¹ = Cl) thus prepared.

Nuclear magnetic resonance spectrum: ¹H-NMR (500 MHz, CDCl₃): δ = 0.83-0 (6H, m), 0.90-1.46 (4H, m), 1.46-1.90 (9H, m), 3.35 (3H, s), 3.50 (2H, t, J = 6.9 Hz), 4.06-4.14 (1H, m), 4.61 (2H, s); ¹³C-NMR (125 MHz, CDCl₃): δ = 18.60, 19.11, 19.38, 19.80, 20.26, 25.46, 26.01, 27.58, 27.69, 29.78, 29.86, 32.67, 33.36, 33.97, 34.43, 44.36, 44.87, 55.07, 68.15, 68.17, 96.37.

Mass spectrum: EI-mass spectrum (70 eV): m/z 249 (M⁺-1), 236, 219, 205, 188, 151, 137, 123, 109, 83, 69, 45.

Infrared absorption spectrum: (D-ATR): v = 2953, 2928, 1461, 1380, 1214, 1153, 1111, 1045, 920, 677, 616.

The 8-chloro-4,6-dimethylnonyl methoxymethyl ether (1': *n*+1 = 3, R¹ = H, X¹ = Cl) thus obtained is the same as the haloalkyl alkoxymethyl ether compound (1: *n =* 3, R¹ = H, X¹ = Cl) described in the present specification. Although 8-chloro-4,6-dimethylnonyl methoxymethyl ether (1': *n*+1 = 3, R¹ = H, X¹ = Cl) is used as the starting material in Examples 3 and 7 below, this is equivalent to using a haloalkyl alkoxymethyl ether compound (1: *n =* 3, R¹ = H, X¹ = Cl) as the starting material.

### Example 3: Preparation of 10-hydroxy-4,6,8-trimethylundecyl methoxymethyl ether (4: n+1 = 4, R¹ = H)

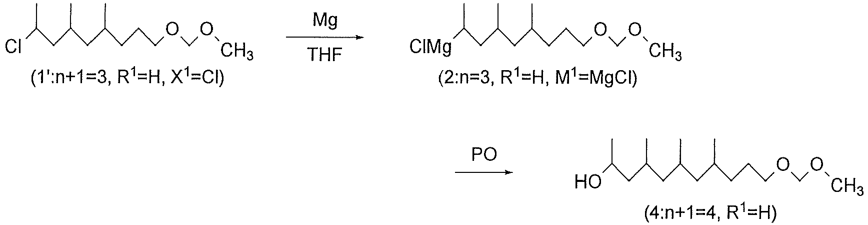

Magnesium (0.75 g, 0.031 gram atoms) and tetrahydrofuran (8.88 g) were placed in a reactor at a room temperature and stirred for 19 minutes at 60 to 65°C. The 8-chloro-4,6-dimethylnonyl methoxymethyl ether (1': *n*+1 = 3, R¹ = H, X¹ = Cl) (7.97 g, 0.030 mol, purity 93.15%) obtained in Example 2 was then added dropwise to the reactor at 60 to 75°C. After the completion of the dropwise addition, the reaction mixture was stirred for 3 hours at 75 to 80°C to obtain 8-methoxymethoxy-1,3,5-trimethyloctylmagnesium chloride (2a: *n =* 3, R¹ = H, M¹ = MgCl).

Next, the internal temperature of the reactor was cooled to 0 to 10°C, and then cuprous chloride (0.0062 g, 0.000059 mol) was added to the reactor, and propylene oxide (1.98 g, 0.034 mol) was added dropwise at 5 to 30°C. After the completion of the dropwise addition, the reaction mixture was stirred for 3.5 hours at 15 to 25°C. An aqueous solution of acetic acid (prepared from acetic acid (4.04 g) and water (32.12 g)) and hexane (2.87 g) were then added to the reaction mixture, followed by phase separation. The organic layer thus obtained was concentrated at a reduced pressure, and the resulting concentrate was purified by silica gel column chromatography (hexane:ethyl acetate = 9:1 to 2:1) to obtain 10-hydroxy-4,6,8-trimethylundecyl methoxymethyl ether (4: *n*+1 *=* 4, R¹ = H) (6.94 g, 0.025 mol, purity 100%) with a yield of 85.45%.

The following is the spectrum data of 10-hydroxy-4,6,8-trimethylundecyl methoxymethyl ether (4: *n*+1 = 4, R¹ = H) thus prepared.

Nuclear magnetic resonance spectrum: ¹H-NMR (500 MHz, CDCl₃): δ = 0.77-1.76 (26H, m), 3.35 (3H, s), 3.49 (2H, t, J = 6.9 Hz), 3.84-3.93 (1H, m), 4.61 (2H, s); ¹³C-NMR (125 MHz, CDCl₃): δ = 19.26, 20.05, 20.18, 20.48, 23.50, 27.11, 27.15, 27.19, 27.22, 27.30, 29.79, 29.85, 29.88, 34.45, 44.32, 45.54, 46.87, 47.09, 55.05, 66.30, 68.16, 68.18, 68.22, 96.33.

Mass spectrum: EI-mass spectrum (70 eV): m/z 273 (M⁺-1), 227, 200, 139, 111, 69, 45.

Infrared absorption spectrum: (D-ATR): v = 3440, 2957, 2925, 1461, 1378, 1214, 1153, 1112, 1045, 920.

### Example 4: Preparation of 10-chloro-4,6,8-trimethylundecyl methoxymethyl ether (1': n+1 = 4, R¹ = H, X¹ = Cl)

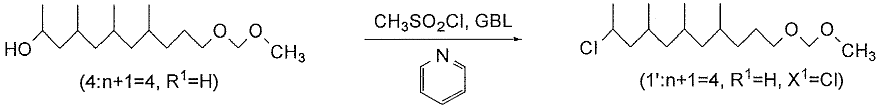

The 10-hydroxy-4,6,8-trimethylundecyl methoxymethyl ether (4: *n*+1 = 4, R¹ = H) (5.64 g, 0.021 mol, purity 100%) obtained in Example 3, pyridine (2.44 g, 0.031 mol), and GBL (15.00 g) were placed in a reactor and stirred for 12 minutes at 10°C.

Methanesulfonyl chloride (CH₃SO₂Cl) (2.83 g, 0.025 mol) was then added dropwise at 5 to 15°C. After the completion of the dropwise addition, the reaction mixture was heated to 60 to 65°C and stirred for 8 hours. After the completion of the stirring, water (15.15 g) and hexane (13.09 g) were added to the reaction mixture, followed by phase separation. The aqueous layer was removed to obtain the organic layer. The organic layered thus obtained was washed with an aqueous solution of acetic acid (prepared from acetic acid (0.22 g) and water (15.00 g)), and then washed with an aqueous solution of sodium bicarbonate (prepared from sodium bicarbonate (0.11 g) and water (15.00 g)). The organic layer thus obtained was concentrated at a reduced pressure, and the resulting concentrate was purified by silica gel column chromatography (hexane:ethyl acetate = 80:0 to 5:1) to obtain 10-chloro-4,6,8-trimethylundecyl methoxymethyl ether *(1': n*+1 = 4, R¹ = H, X¹ = Cl) (5.56 g, 0.017 mol, purity 90.04%) with a yield of 83.03%.

The following is the spectrum data of 10-chloro-4,6,8-trimethylundecyl methoxymethyl ether (1': *n*+1 = 4, R¹ = H, X¹ = Cl) thus prepared.

Nuclear magnetic resonance spectrum: ¹H-NMR (500 MHz, CDCl₃): δ = 0.75-1.90 (25H, m), 3.36 (3H, s), 3.50 (2H, t, J = 6.9 Hz), 4.06-4.15 (1H, m), 4.61 (2H, s); ¹³C-NMR (125 MHz, CDCl₃): δ = 19.35, 19.78, 20.24, 20.46, 26.27, 27.10, 27.11, 27.22, 27.25, 27.31, 27.52, 27.77, 29.79, 29.86, 29.91, 32.98, 45.28, 55.06, 68.18, 68.22, 96.37.

Mass spectrum: EI-mass spectrum (70 eV): m/z 291 (M⁺-1), 247, 146, 111, 69, 45.

Infrared absorption spectrum: (D-ATR): v = 2854, 2927, 1460, 1380, 1214, 1153, 1111, 1047, 968, 920, 677, 617.

### Example 5: Preparation of 8-chloro-4,6-dimethylnonyl butyloxymethyl ether (1': n+1 = 3, R¹ = CH₂CH₂CH₃, X¹ = Cl)

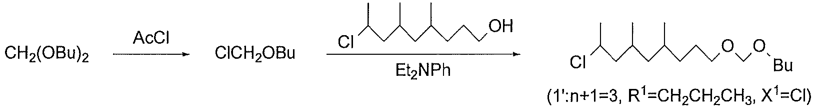

Dibutoxymethane (3.02 g, 0.019 mol) and zinc chloride (0.02 g, 0.00018 mol) were placed in a reactor at a room temperature and stirred for 4 minutes at 15 to 25°C. Acetyl chloride (AcCl) (1.31 g, 0.017 mol) was then added dropwise to the reactor at 30 to 40°C. After the completion of the dropwise addition, the reaction mixture was stirred for 4 hours at 38 to 42°C to obtain chloromethyl butyl ether.

A mixed solution of N,Ndiethylaniline (2.49 g, 0.017 mol) and 8-chloro-4,6-dimethylnonanol (3.02 g, 0.015 mol, purity 99.26%) was then added dropwise to the reactor at 20 to 30°C. After the completion of the dropwise addition, the reaction mixture was stirred for 19 hours at 20 to 30°C. An aqueous solution (2.52 g) of 25% by mass sodium hydroxide and water (1.60 g) were then added to the reaction mixture, followed by phase separation. The organic layer thus obtained was concentrated at a reduced pressure, and the resulting concentrate was purified by silica gel column chromatography (hexane:ethyl acetate = 50:0 to 9:1) to obtain 8-chloro-4,6-dimethylnonyl butyloxymethyl ether (1': *n*+1 = 3, R¹ = CH₂CH₂CH₃, X¹ = Cl) (4.03 g, 0.014 mol, purity 99.99%) with a yield of 94.90%.

The following is the spectrum data of 8-chloro-4,6-dimethylnonyl butyloxymethyl ether (1': *n*+1 = 3, R¹ = CH₂CH₂CH₃, X¹ = Cl) thus prepared.

Nuclear magnetic resonance spectrum: ¹H-NMR (500 MHz, CDCl₃): δ = 0.83-0.89 (6H, m), 0.92 (3H, t, J = 7.3 Hz), 1.00-1.90 (17H, m), 3.50 (2H, t, J = 6.9 Hz), 3.52 (2H, t, J = 6.9 Hz), 4.05-4.14 (1H, m), 4.66 (2H, s); ¹³C-NMR (125 MHz, CDCl₃): δ = 13.87, 19.12, 19.37, 19.58, 19.60, 19.80, 25.47, 26.22, 27.17, 27.30, 27.68, 27.80, 29.68, 29.79, 29.86, 31.80, 33.39, 34.00, 34.45, 43.62, 44.84, 45.17, 47.34, 48.44, 48.78, 56.63, 67.51, 68.08, 68.13, 95.25.

Mass spectrum: EI-mass spectrum (70 eV): m/z 291 (M⁺-1), 257, 205, 151, 111, 87, 57.

Infrared absorption spectrum: (D-ATR): v = 2958, 2929, 2872, 1459, 1380, 1145, 1115, 1071, 1046, 616.

### Example 6: Preparation of 8-chloro-4,6-dimethylnonyl benzyloxymethyl ether (1': n+1 = 3, R¹ = Ph, X¹ = Cl)

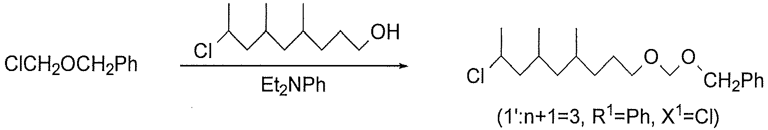

Benzyl chloromethyl ether (2.25 g, 0.013 mol, purity 90%) and toluene (4.0 g) were placed in a reactor at a room temperature and stirred for 19 minutes at 15 to 25°C. A mixture of 8-chloro-4,6-dimethylnonanol (2.95 g, 0.011 mol, purity 78.77%) and *N,N-*diethylaniline (1.93 g, 0.013 mol) was then added dropwise to the reactor at 20 to 30°C. After the completion of the dropwise addition, the reaction mixture was stirred for 20 hours at 20 to 30°C. An aqueous solution of 25% sodium hydroxide (2.54 g; 0.016 mol of sodium hydroxide) and water (1.61 g) were then added to the reaction mixture, followed by phase separation. The reaction mixture was washed three times with 20% hydrochloric acid (0.88 g; 0.0048 mol of hydrogen chloride). The organic layer thus obtained was concentrated, and the resulting concentrate was purified by silica gel column chromatography (hexane:ethyl acetate = 50:1 to 9:1) to obtain 8-chloro-4,6-dimethylnonyl benzyloxymethyl ether (1': *n*+1 = 3, R¹ = Ph, X¹ = Cl) (4.31 g, 0.011 mol, purity 79.80%) with a yield of 93.60%.

The following is the spectrum data of 8-chloro-4,6-dimethylnonyl benzyloxymethyl ether (1': *n*+1 = 3, R¹ = Ph, X¹ = Cl) thus prepared.

Nuclear magnetic resonance spectrum: ¹H-NMR (500 MHz, CDCl₃): δ = 0.80-1.90 (19H, m), 3.58 (2H, t, J = 6.9 Hz), 4.07-4.16 (1H, m), 4.61 (2H, s), 4.77 (2H, s), 7.34-7.38 (5H, m); ¹³C-NMR (125 MHz, CDCl₃): δ = 20.27, 20.29, 25.21, 26.02, 26.94, 27.18, 27.58, 27.69, 27.93, 29.70, 29.79, 32.68, 44.34, 45.17, 47.34, 48.16, 56.70, 68.41, 69.23, 69.25, 69.49, 94.58, 94.60, 127.62, 127.85, 127.93, 128.38, 128.42, 137.97.

Mass spectrum: EI-mass spectrum (70 eV): m/z 326 (M⁺), 151, 120, 91, 55.

Infrared absorption spectrum: (D-ATR): v = 3031, 2953, 2927, 2872, 1497, 1455, 1379, 1208, 1169, 1113, 1048, 1028, 967, 735, 697, 614.

### Example 7: Preparation of 4,6,8,10-tetramethyltridecyl methoxymethyl ether (6: R¹ = H)

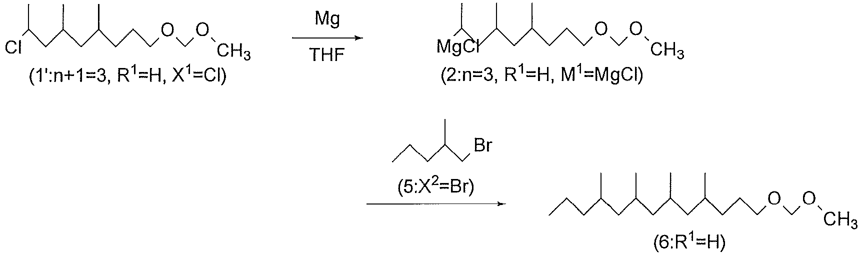

Magnesium (3.49 g, 0.14 gram atoms) and tetrahydrofuran (75.13 g) were placed in a reactor at a room temperature and stirred for 29 minutes at 60 to 65°C. The 8-chloro-4,6-dimethylnonyl methoxymethyl ether (1': *n*+1 = 3, R¹ = H, X¹ = Cl) (36.78 g, 0.14 mol, purity 93.15%) obtained in Example 2 was then added dropwise to the reactor at 60 to 75°C. After the completion of the dropwise addition, the reaction mixture was stirred for 5 hours at 75 to 80°C to obtain 8-methoxymethoxy-1,3,5-trimethyloctylmagnesium chloride (2: *n =* 3, R¹ = H, M¹ = MgCl).

Cuprous iodide (0.26 g, 0.0014 mol), triethyl phosphite (0.54 g, 0.0033 mol), tetrahydrofuran (162.56 g), and 1-bromo-2-methylpentane (5: X² = Br) (21.85 g, 0.13 mol, purity 98.05%) were then placed in another reactor, and the aforesaid 8-methoxymethoxy-1,3,5-trimethyloctylmagnesium chloride (2: *n* = 3, R¹ = H, M¹ = MgCl) that was prepared was added dropwise at 15 to 25°C. After the completion of the dropwise addition, the reaction mixture was stirred for 2.5 hours at 15 to 25°C. An aqueous solution of acetic acid (prepared from acetic acid (17.07 g) and water (51.23 g)) and an aqueous solution (4.09 g) of 25% by mass sodium hydroxide were then added to the reaction mixture, followed by phase separation. The organic layer thus obtained was concentrated at a reduced pressure, and the resulting concentrate was purified by silica gel column chromatography (hexane:ethyl acetate = 50:1 to 9:1) to obtain 4,6,8,10-tetramethyltridecyl methoxymethyl ether (6: R¹ = H) (36.09 g, 0.10 mol, purity 87.02%) with a yield of 80.50%.

The following is the spectrum data of 4,6,8,10-tetramethyltridecyl methoxymethyl ether (6: R¹ = H) thus prepared.

Nuclear magnetic resonance spectrum: ¹H-NMR (500 MHz, CDCl₃): δ = 0.76-0.90 (16H, m), 0.90-1.43 (11H, m), 1.43-1.70 (6H, m), 1.43-1.70 (6H, m), 3.50 (2H, t, J = 6.9 Hz), 3.03 (3H, s), 4.62 (2H, s); ¹³C-NMR (125 MHz, CDCl₃): δ = 14.36, 14.41, 19.34, 19.93, 19.97, 20.00, 20.02, 20.12, 20.14, 20.28, 20.30, 20.42, 20.66, 21.01, 27.10, 27.20, 27.24, 27.27, 27.31, 27.39, 29.73, 29.76, 29.82, 33.02, 40.56, 46.12, 55.06, 68.22, 68.26.

Mass spectrum: EI-mass spectrum (70 eV): m/z 299 (M⁺-1), 269, 255, 239, 225, 199, 185, 171, 155, 141, 125, 111, 85, 69, 45.

Infrared absorption spectrum: (D-ATR): v = 2956, 2915, 2872, 1461, 1379, 1154, 1112, 1047, 921, 740.

### Example 8: Preparation of 4,6,8,10-tetramethyltridecanol (7)

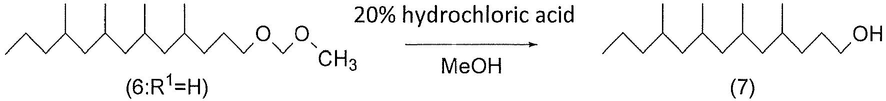

The 4,6,8,10-tetramethyltridecyl methoxymethyl ether (6: R¹ = H) (36.09 g, 0.10 mol, purity 87.02%) obtained in Example 7, methanol (50.50 g, 1.58 mol), 20% hydrochloric acid (5.05 g; 0.028 mol of hydrogen chloride) were placed in a reactor equipped with a distillation tower, and the reaction mixture was heated to 60°C and stirred for 1 hour. After the completion of the stirring, the internal temperature of the reactor was heated to 65 to 70°C to distill off a mixture of by-produced dimethoxymethane and methanol from the distillation tower. The reaction mixture was sampled during the reaction. After the conversion was confirmed to be 100%, water (30.30 g) was added to the reaction mixture, followed by phase separation. The aqueous layer was removed to obtain the organic layer. The organic layer thus obtained was concentrated at a reduced pressure, and the resulting concentrate was purified by silica gel column chromatography (hexane:ethyl acetate = 20:1 to 9:1) to obtain 4,6,8,10-tetramethyltridecanol (7) (27.19 g, 0.10 mol, purity 97.52%) with a yield of 100%.

The following is the spectrum data of 4,6,8,10-tetramethyltridecanol (7) thus prepared.

Nuclear magnetic resonance spectrum: ¹H-NMR (500 MHz, CDCl₃): δ = 0.76-0.90 (16H, m), 0.90-1.42 (12H, m), 1.42-1.66 (6H, m), 1.42-1.67 (6H, m), 3.62 (2H, t, J = 6.9 Hz); ¹³C-NMR (125 MHz, CDCl₃): δ = 14.37, 14.42, 19.25, 19.34, 19.94, 19.97, 20.02, 20.06, 20.12, 20.15, 20.31, 20.35, 20.44, 20.50, 20.64, 20.95, 27.21, 27.24, 27.27, 27.30, 27.39, 29.67, 29.72, 29.76, 29.83, 29.88, 29.98, 30.19, 30.21, 30.37, 32.55, 32.86, 34.11, 38.80, 39.44, 40.56, 44.25, 44.31, 45.08, 45.37, 45.66, 45.89, 46.11, 46.22, 63.43, 63.47.

Mass spectrum: EI-mass spectrum (70 eV): m/z 255 (M⁺-1), 238, 224, 210, 195, 181, 167, 153, 139, 125, 111, 97, 83, 69, 43, 29.

Infrared absorption spectrum: (D-ATR): vmax = 3325, 2857, 2914, 2871, 1460, 1378, 1058, 739.

### Example 9: Preparation of 1-chloro-4,6,8,10-tetramethyltridecane (8: X³ = Cl)

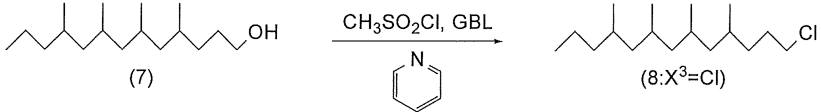

The 4,6,8,10-tetramethyltridecanol (7) (21.89 g, 0.083 mol, purity 97.52%) obtained in Example 8, pyridine (9.88 g, 0.12 mol), and GBL (12.48 g) were placed in a reactor and stirred for 16 minutes at 40°C.

Methanesulfonyl chloride (CH₃SO₂Cl) (11.44 g, 0.10 mol) was then added dropwise at 40 to 60°C. After the completion of the dropwise addition, the reaction mixture was heated to 60 to 65°C and stirred for 10 hours. After the completion of the stirring, water (20.81 g) and hexane (12.49 g) were added to the reaction mixture, followed by phase separation. The aqueous layer was removed to obtain the organic layer. The organic layer thus obtained was washed with an aqueous solution of acetic acid (prepared from acetic acid (0.89 g) and water (10.95 g)), and then washed with an aqueous solution of sodium bicarbonate (prepared from sodium bicarbonate (0.44 g) and water (10.95 g)). The organic layer thus obtained was then concentrated at a reduced pressure, and the resulting concentrate was purified by silica gel column chromatography (hexane:ethyl acetate = 20:1 to 9:1) to obtain 1-chloro-4,6,8,10-tetramethyltridecane (8: X³ = Cl) (21.26 g, 0.076 mol, purity 98.37%) with a yield of 91.40%.

The following is the spectrum data of 1-chloro-4,6,8,10-tetramethyltridecane (8: X³ = Cl) thus prepared.

Nuclear magnetic resonance spectrum: ¹H-NMR (500 MHz, CDCl₃): δ = 0.77-0.90 (16H, m), 0.90-1.65 (15H, m), 1.65-1.86 (2H, m), 3.52 (2H, t, J = 6.9 Hz); ¹³C-NMR (125 MHz, CDCl₃): δ = 14.38, 14.44, 19.26, 19.36, 19.93, 19.95, 19.99, 20.04, 20.11, 20.16, 20.26, 20.38, 20.51, 20.63, 20.94, 21.03, 27.25, 27.29, 27.31, 27.32, 27.41, 29.45, 29.51, 29.55, 29.62, 29.75, 30.14, 30.18, 33.61, 33.79, 34.09, 38.82, 44.34, 44.95, 45.24, 45.51, 45.66.

Mass spectrum: EI-mass spectrum (70 eV): m/z 274 (M⁺), 259, 245, 231, 217, 203, 189, 175, 155, 133, 113, 99, 85, 71, 57, 43, 29.

Infrared absorption spectrum: (D-ATR): vmax = 2957, 2914, 2871, 2844, 1461, 1379, 1158, 970, 726, 656.

### Example 10: Preparation of 4,6,8,10,16-pentamethyldocosane (11)

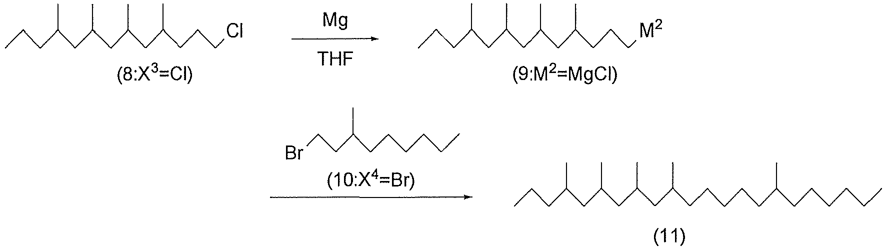

Magnesium (1.94 g, 0.080 gram atoms) and tetrahydrofuran (70.16 g) were placed in a reactor at a room temperature and stirred for 31 minutes at 60 to 65°C. The 1-chloro-4,6,8,10-tetramethyltridecane (8: X³ = Cl) (21.26 g, 0.076 mol, purity 98.37%) obtained in Example 9 was then added dropwise to the reactor at 60 to 75°C. After the completion of the dropwise addition, the reaction mixture was stirred for 5 hours at 75 to 80°C to obtain 4,6,8,10-tetramethyltridecylmagnesium chloride (9: M² = MgCl).

Cuprous chloride (0.085 g, 0.00086 mol), triethyl phosphite (0.85 g, 0.0014 mol), lithium chloride (0.059 g, 0.0014 mol), tetrahydrofuran (132.71 g), and 1-bromo-3-methylnonane (10: X⁴ = Br) (16.32 g, 0.072 mol, purity 97.97%) were then placed in another reactor, and the aforesaid 4,6,8,10-tetramethyltridecylmagnesium chloride (9: M² = MgCl) that was prepared was added dropwise at 15 to 25°C. After the completion of the dropwise addition, the reaction mixture was stirred for 2 hours at 20 to 30°C. An aqueous solution of acetic acid (prepared from acetic acid (0.77 g) and water (20.97 g)), 20% by mass hydrochloric acid (1.59 g; 0.0087 mol of hydrogen chloride), and an aqueous solution of 25% by mass sodium hydroxide (1.59 g; 0.0099 mol of sodium hydroxide) were then added to the reaction mixture, followed by phase separation. The organic layer thus obtained was then distilled at a reduced pressure as such to distil off low-boiling impurities. The resulting concentrate was then purified by silica gel column chromatography (hexane = 100) to obtain the 4,6,8,10,16-pentamethyldocosane (11) (24.29 g, 0.062 mol, purity 96.43%) with a yield of 80.86%.

The following is the spectrum data of 4,6,8,10,16-pentamethyldocosane (11) thus prepared.

Nuclear magnetic resonance spectrum: ¹H-NMR (500 MHz, CDCl₃): δ = 0.78-0.92 (22H, m), 0.92-1.18 (8H, m), 1.18-1.40 (22H, m), 1.40-1.63 (4H, m); ¹³C-NMR (125 MHz, CDCl₃): δ = 14.14, 14.40, 14.45, 19.33, 19.39, 19.45, 19.73, 19.97, 20.01, 20.05, 20.08, 20.18, 20.21, 20.45, 22.72, 27.08, 27.14, 27.26, 27.28, 27.31, 27.35, 29.73, 29.76, 30.37, 30.40, 31.99, 32.77, 37.12, 45.50, 46.21.

Mass spectrum: EI-mass spectrum (70 eV): m/z 380 (M⁺), 337, 295, 253, 197, 155, 113, 71, 41.

Infrared absorption spectrum: (D-ATR): v = 2857, 2925, 2871, 2854, 1463, 1378, 1157, 970, 724.

### Example 11: Preparation of 8-bromo-4,6-dimethylnonyl methoxymethyl ether (1': n+1 = 3, R¹ = H, X¹ = Br)

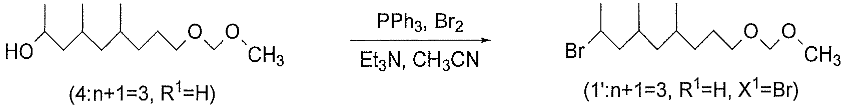

Triphenylphosphine (PPh₃) (5.51 g, 0.021 mol) and acetonitrile (CH₃CN) (15.45 g) were placed in a reactor at a room temperature and stirred for 40 minutes at 0 to 10°C. Bromine (Br₂) (3.21 g, 0.020 mol) was then added dropwise to the reactor at 0 to 10°C. After the completion of the dropwise addition, the reaction mixture was stirred for 3 hours at 0 to 10°C. A mixed solution of the 8-hydroxy-4,6-dimethylnonyl methoxymethyl ether (4: *n*+1 = 3, R¹ = H) (3.69 g, 0.015 mol, purity 95.28%) obtained in Example 1 and triethylamine (2.14 g, 0.021 mol) were then added dropwise at 0 to 10°C. After the completion of the dropwise addition, the reaction mixture was stirred for 20 hours at 15 to 25°C. Water (20.00 g) and hexane (6.60 g) were then added to the reaction mixture, followed by phase separation. The organic layer thus obtained was then concentrated at a reduced pressure, and the resulting concentrate was purified by silica gel column chromatography (hexane:ethyl acetate = 50:1 to 9:1) to obtain 8-bromo-4,6-dimethylnonyl methoxymethyl ether (1': *n*+1 = 3, R¹ = H, X¹ = Br) (3.38 g, 0.0095 mol, purity 82.67%) with a yield of 62.62%.

The following is the spectrum data of 8-bromo-4,6-dimethylnonyl methoxymethyl ether (1': *n*+1 = 3, R¹ = H, X¹ = Br) thus prepared.

Nuclear magnetic resonance spectrum: ¹H-NMR (500 MHz, CDCl₃): δ = 0.82-0.90 (6H, m), 0.90-1.45 (4H, m), 1.45-1.90 (9H, m), 3.35 (3H, s), 3.50 (2H, dt, J = 1.9 Hz, 6.7 Hz), 4.14-4.25 (1H, m), 4.61 (2H, s); ¹³C-NMR (125 MHz, CDCl₃): δ = 18.56, 19.14, 19.42, 19.45, 19.51, 19.81, 20.29, 26.21, 26.50, 27.09, 27.15, 27.19, 27.29, 27.31, 28.81, 28.87, 28.89, 29.66, 29.75, 29.79, 29.84, 32.64, 33.39, 33.94, 34.44, 43.52, 44.25, 44.72, 45.08, 48.09, 49.04, 49.18, 49.54, 49.57, 49.63, 60.25, 50.40, 55.09, 68.16, 96.37.

Mass spectrum: EI-mass spectrum (70 eV): m/z 293 (M⁺-1), 249, 206, 151, 111, 69, 45.

Infrared absorption spectrum: (D-ATR): v = 2953, 2925, 2872, 1457, 1380, 1214, 1152, 1111, 1045, 966, 920, 625, 540.

## Claims

1. A process for preparing a haloalkyl alkoxymethyl ether compound of the following general formula (1'): 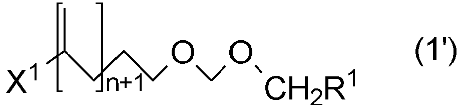
wherein X¹ represents a halogen atom, R¹ represents a hydrogen atom, an n-alkyl group having 1 to 9 carbon atoms, or a phenyl group, and n represents an integer of 2 to 7,
the process comprising the steps of
converting a haloalkyl alkoxymethyl ether compound of the following general formula (1): 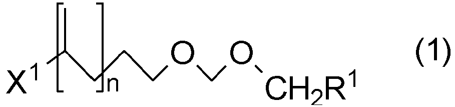
wherein X¹, R¹, and *n* are as defined above,
into a nucleophilic reagent, (2n+2)-alkoxymethoxyalkyl compound, of the following general formula (2): 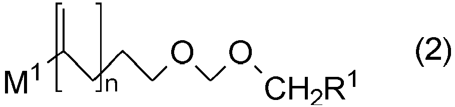
wherein M¹ represents Li, MgZ¹, CuZ¹, or CuLiZ¹, Z¹ represents a halogen atom or Z², and Z² represents: 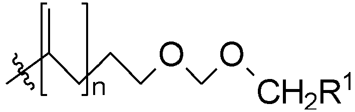
wherein R¹ and *n* are as defined above, and the wavy line indicates that the structures therebeyond are abbreviated,
subsequently subjecting the nucleophilic reagent, (2n+2)-alkoxymethoxyalkyl compound (2), to a nucleophilic addition reaction with propylene oxide of the following formula (3): 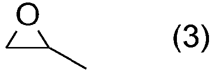
to obtain a hydroxyalkyl alkoxymethyl ether compound of the following general formula (4): 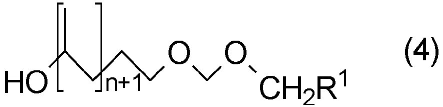
wherein R¹ and *n* are as defined above;
and subjecting the hydroxyalkyl alkoxymethyl ether compound (4) to a halogenation reaction to obtain the aforesaid haloalkyl alkoxymethyl ether compound (1').

2. The process for preparing the haloalkyl alkoxymethyl ether compound (1') according to claim 1, wherein n represents 2 in the haloalkyl alkoxymethyl ether compounds (1) and (1'), resulting the haloalkyl alkoxymethyl ether compound (1') being the haloalkyl alkoxymethyl ether compound (1':n+1=3).

3. A process for preparing 4,6,8,10,16-pentamethyldocosane of the following general formula (11): 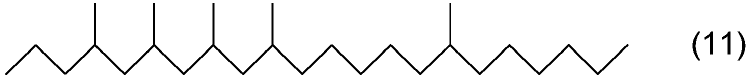
the process comprising the steps of
the process for preparing the aforesaid haloalkyl alkoxymethyl ether compound (1': *n+1* = 3) according to claim 2,
converting the haloalkyl alkoxymethyl ether compound (1': *n+1* = 3) into a nucleophilic reagent, 8-alkoxymethoxy-1,3,5-trimethyloctyl compound, of the following general formula (2: *n =* 3): 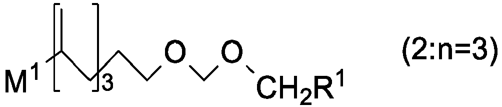
wherein M¹ represents Li, MgZ¹, CuZ¹, or CuLiZ¹, Z¹ represents a halogen atom or a 8-alkoxymethoxy-1,3,5-trimethyloctyl group, and R¹ is as defined above,
subsequently subjecting the nucleophilic reagent, 8-alkoxymethoxy-1,3,5-trimethyloctyl compound (2: *n* = 3), to a coupling reaction with a 1-halo-2-methylpentane compound of the following general formula (5): 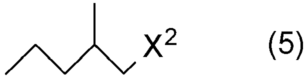
wherein X² represents a halogen atom,
to obtain a 4,6,8,10-tetramethyltridecyl alkoxymethyl ether compound of the following general formula (6): 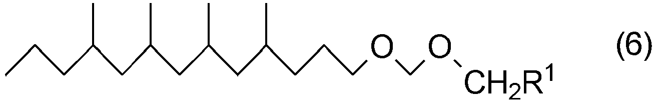
wherein R¹ is as defined above;
subjecting the 4,6,8,10-tetramethyltridecyl alkoxymethyl ether compound (6) to a dealkoxymethylation reaction to obtain 4,6,8,10-tetramethyltridecanol of the following formula (7): 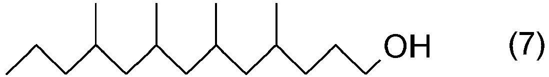
subjecting the 4,6,8,10-tetramethyltridecanol (7) to a halogenation reaction to obtain a 1-halo-4,6,8,10-tetramethyltridecane compound (8) of the following general formula (8): 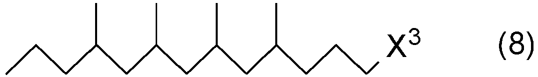
wherein X³ represents a halogen atom,
converting the 1-halo-4,6,8,10-tetramethyltridecane compound (8) into a nucleophilic reagent, 4,6,8,10-tetramethyltridecyl compound, of the following general formula (9): 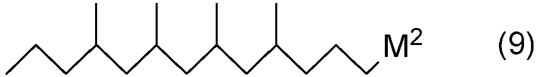
wherein M² represents Li, MgZ¹, CuZ¹, or CuLiZ¹, Z¹ represents a halogen atom or a 4,6,8,10-tetramethyltridecyl group,
and subsequently subjecting the nucleophilic reagent, 4,6,8,10-tetramethyltridecyl compound (9), to a coupling reaction with a 1-halo-3-methylnonane compound of the following general formula (10): 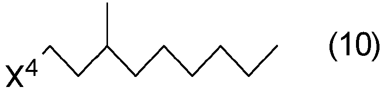
wherein X⁴ represents a halogen atom,
to form the aforesaid 4,6,8,10,16-pentamethyldocosane (11).

4. A process for preparing 4,6,8,10,16-pentamethyldocosane of the following general formula (11): 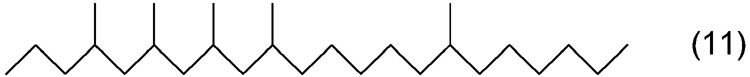
the process comprising the steps of
converting a haloalkyl alkoxymethyl ether compound of the following general formula (1'): 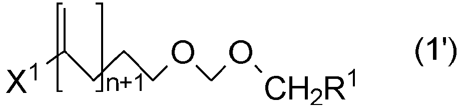
wherein X¹ represents a halogen atom, R¹ represents a hydrogen atom, an n-alkyl group having 1 to 9 carbon atoms, or a phenyl group, and n represents 2 ,
into a nucleophilic reagent, 8-alkoxymethoxy-1,3,5-trimethyloctyl compound, of the following general formula (2: *n* = 3): 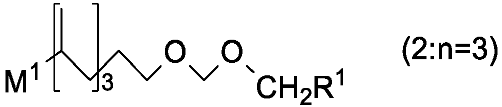
wherein M¹ represents Li, MgZ¹, CuZ¹, or CuLiZ¹, Z¹ represents a halogen atom or a 8-alkoxymethoxy-1,3,5-trimethyloctyl group, and R¹ is as defined above,
subsequently subjecting the nucleophilic reagent, 8-alkoxymethoxy-1,3,5-trimethyloctyl compound (2: *n* = 3), to a coupling reaction with a 1-halo-2-methylpentane compound of the following general formula (5): 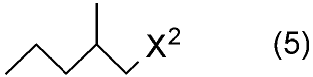
wherein X² represents a halogen atom,
to obtain a 4,6,8,10-tetramethyltridecyl alkoxymethyl ether compound of the following general formula (6): 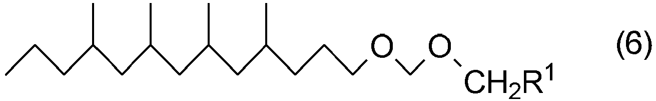
wherein R¹ is as defined above;
subjecting the 4,6,8,10-tetramethyltridecyl alkoxymethyl ether compound (6) to a dealkoxymethylation reaction to obtain 4,6,8,10-tetramethyltridecanol of the following formula (7): 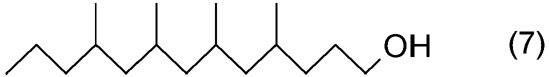
subjecting the 4,6,8,10-tetramethyltridecanol (7) to a halogenation reaction to obtain a 1-halo-4,6,8,10-tetramethyltridecane compound (8) of the following general formula (8): 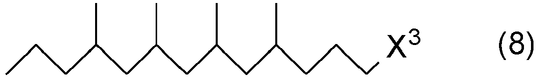
wherein X³ represents a halogen atom;
converting the aforesaid 1-halo-4,6,8,10-tetramethyltridecane compound (8) into a nucleophilic reagent, 4,6,8,10-tetramethyltridecyl compound, of the following general formula (9): 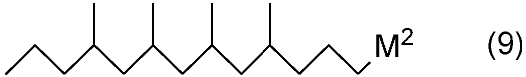
wherein M² represents Li, MgZ¹, CuZ¹, or CuLiZ¹, and Z¹ represents a halogen atom or a 4,6,8,10-tetramethyltridecyl group,
and subsequently subjecting the nucleophilic reagent, 4,6,8,10-tetramethyltridecyl compound (9), to a coupling reaction with a 1-halo-3-methylnonane compound of the following general formula (10): 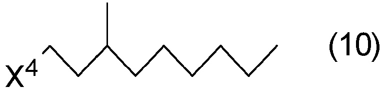
wherein X⁴ represents a halogen atom,
to form the aforesaid 4,6,8,10,16-pentamethyldocosane (11).

5. A haloalkyl alkoxymethyl ether compound of the following general formula (1'): 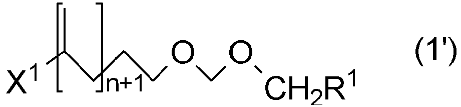 wherein X¹ represents a halogen atom, R¹ represents a hydrogen atom, an n-alkyl group having 1 to 9 carbon atoms, or a phenyl group, and n represents an integer of 2 to 7.

## Patentansprüche

1. Ein Verfahren zur Herstellung einer Halogenalkylalkoxymethyletherverbindung der folgenden allgemeinen Formel (1'): 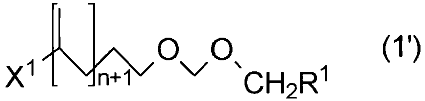
wobei X¹ ein Halogenatom darstellt, R¹ ein Wasserstoffatom, eine n-Alkylgruppe mit 1 bis 9 Kohlenstoffatomen oder eine Phenylgruppe darstellt und n eine ganze Zahl von 2 bis 7 darstellt,
wobei das Verfahren die Schritte umfasst:
Umwandeln einer Halogenalkylalkoxymethyletherverbindung der folgenden allgemeinen Formel (1): 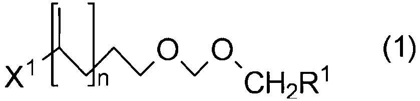
wobei X¹, R¹ und n wie vorstehend definiert sind,
in ein nukleophiles Reagenz, (2n+2)-Alkoxymethoxyalkylverbindung, der folgenden allgemeinen Formel (2): 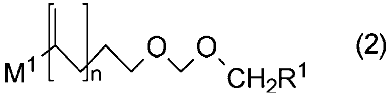
wobei M¹ für Li, MgZ¹, CuZ¹ oder CuLiZ¹ steht, Z¹ ein Halogenatom oder Z² darstellt und Z² 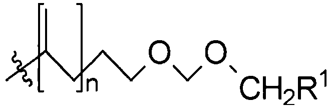
darstellt,
wobei R¹ und *n* wie vorstehend definiert sind, und die Wellenlinie angibt, dass die Strukturen darunter abgekürzt sind,
anschließendes Unterziehen des nukleophilen Reagenz, *(2n+2)-*Alkoxymethoxyalkylverbindung (2), einer nukleophilen Additionsreaktion mit Propylenoxid der folgenden Formel (3): 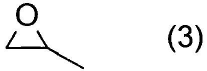
um eine Hydroxyalkylalkoxymethyletherverbindung der folgenden allgemeinen Formel (4) zu erhalten: 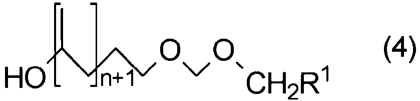
wobei R¹ und *n* wie vorstehend definiert sind;
und Unterziehen der Hydroxyalkylalkoxymethyletherverbindung (4) einer Halogenierungsreaktion, um die vorstehende Halogenalkylalkoxymethyletherverbindung (1') zu erhalten.

2. Das Verfahren zur Herstellung der Halogenalkylalkoxymethyletherverbindung (1') nach Anspruch 1, wobei n für 2 in den Halogenalkylalkoxymethyletherverbindungen (1) und (1') steht, was dazu führt, dass die Halogenalkylalkoxymethyletherverbindung (1') die Halogenalkylalkoxymethyletherverbindung (1': n+1=3) ist.

3. Ein Verfahren zur Herstellung von 4,6,8,10,16-Pentamethyldocosan der folgenden allgemeinen Formel (11): 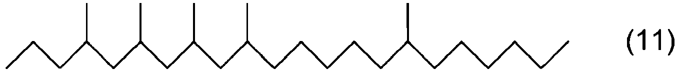
wobei das Verfahren die Schritte umfasst
das Verfahren zur Herstellung der vorstehenden Halogenalkylalkoxymethyletherverbindung (1': n+1=3) nach Anspruch 2,
Umwandeln der Halogenalkylalkoxymethyletherverbindung (1': *n+1=3)* in ein nukleophiles Reagenz, 8-Alkoxymethoxy-1,3,5-trimethyloctylverbindung, der folgenden allgemeinen Formel (2: *n=3):* 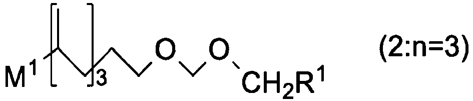
wobei M¹ für Li, MgZ¹, CuZ¹ oder CuLiZ¹ steht, Z¹ ein Halogenatom oder eine 8-Alkoxymethoxy-1,3,5-trimethyloctylgruppe darstellt und R¹ wie vorstehend definiert ist,
anschließendes Unterziehen des nukleophilen Reagenz, 8-Alkoxymethoxy-1,3,5-trimethyloctylverbindung (2: n=3), einer Kupplungsreaktion mit einer 1-Halogen-2-methylpentanverbindung der folgenden allgemeinen Formel (5): 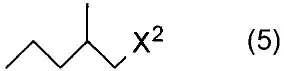
wobei X² ein Halogenatom darstellt,
um eine 4,6,8,10-Tetramethyltridecylalkoxymethyletherverbindung der folgenden allgemeinen Formel (6) zu erhalten: 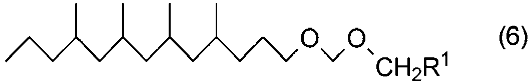
wobei R¹ wie vorstehend definiert ist,
Unterziehen der 4,6,8,10-Tetramethyltridecylalkoxymethyletherverbindung (6) einer Dealkoxymethylierungsreaktion, um 4,6,8,10-Tetramethyltridecanol der folgenden Formel (7) zu erhalten: 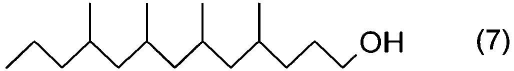
Unterziehen des 4,6,8,10-Tetramethyltridecanols (7) einer Halogenierungsreaktion, um eine 1-Halogen-4,6,8,10-tetramethyltridecanverbindung (8) der folgenden allgemeinen Formel (8) zu erhalten: 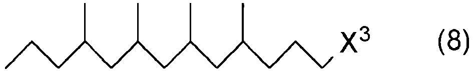
wobei X³ ein Halogenatom darstellt,
Umwandeln der 1-Halogen-4,6,8,10-tetramethyltridecanverbindung (8) in ein nukleophiles Reagenz, 4,6,8,10-Tetramethyltridecylverbindung, der folgenden allgemeinen Formel (9): 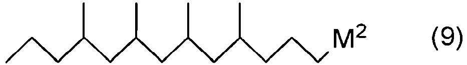
wobei M² für Li, MgZ¹, CuZ¹ oder CuLiZ¹ steht, Z¹ ein Halogenatom oder eine 4,6,8,10-Tetramethyltridecylgruppe darstellt,
und anschließendes Unterziehen des nukleophilen Reagenz, 4,6,8,10-Tetramethyltridecylverbindung (9), einer Kupplungsreaktion mit einer 1-Halogen-3-methylnonanverbindung der folgenden allgemeinen Formel (10): 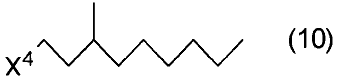
wobei X⁴ ein Halogenatom darstellt,
um das vorstehende 4,6,8,10,16-Pentamethyldocosan (11) zu bilden.

4. Ein Verfahren zur Herstellung von 4,6,8,10,16-Pentamethyldocosan der folgenden allgemeinen Formel (11): 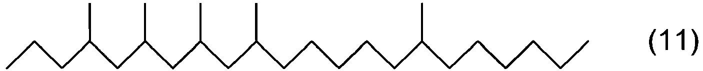
wobei das Verfahren die Schritte umfasst
Umwandeln einer Halogenalkylalkoxymethyletherverbindung der folgenden allgemeinen Formel (1'): 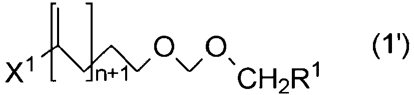
wobei X¹ ein Halogenatom darstellt, R¹ ein Wasserstoffatom, eine n-Alkylgruppe mit 1 bis 9 Kohlenstoffatomen oder eine Phenylgruppe darstellt und *n* 2 darstellt,
in ein nukleophiles Reagenz, 8-Alkoxymethoxy-1,3,5-trimethyloctylverbindung, der folgenden allgemeinen Formel (2: *n=3):* 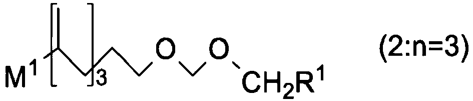
wobei M¹ für Li, MgZ¹, CuZ¹ oder CuLiZ¹ steht, Z¹ ein Halogenatom oder eine 8-Alkoxymethoxy-1,3,5-trimethyloctylgruppe darstellt und R¹ wie vorstehend definiert ist,
anschließendes Unterziehen des nukleophilen Reagenz, 8-Alkoxymethoxy-1,3,5-trimethyloctylverbindung (2: n=3), einer Kupplungsreaktion mit einer 1-Halogen-2-methylpentanverbindung der folgenden allgemeinen Formel (5): 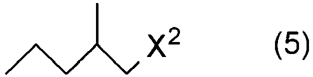
wobei X² ein Halogenatom darstellt,
um eine 4,6,8,10-Tetramethyltridecylalkoxymethyletherverbindung der folgenden allgemeinen Formel (6) zu erhalten: 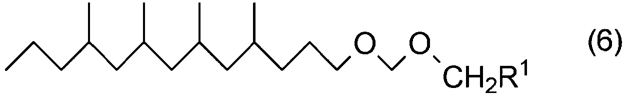
wobei R¹ wie vorstehend definiert ist,
Unterziehen der 4,6,8,10-Tetramethyltridecylalkoxymethyletherverbindung (6) einer Dealkoxymethylierungsreaktion, um 4,6,8,10-Tetramethyltridecanol der folgenden Formel (7) zu erhalten: 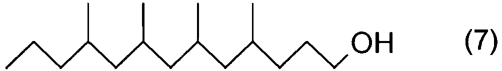
Unterziehen des 4,6,8,10-Tetramethyltridecanols (7) einer Halogenierungsreaktion, um eine 1-Halogen-4,6,8,10-Tetramethyltridecanverbindung (8) der folgenden allgemeinen Formel (8) zu erhalten: 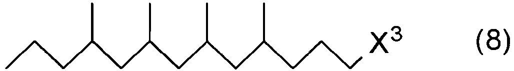
wobei X³ ein Halogenatom darstellt,
Umwandeln der vorstehenden 1-Halogen-4,6,8,10-Tetramethyltridecanverbindung (8) in ein nukleophiles Reagenz, 4,6,8,10-Tetramethyltridecylverbindung, der folgenden allgemeinen Formel (9): 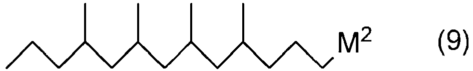
wobei M² für Li, MgZ¹, CuZ¹ oder CuLiZ¹ steht und Z¹ ein Halogenatom oder eine 4,6,8,10-Tetramethyltridecylgruppe darstellt,
und anschließendes Unterziehen des nukleophilen Reagenz, 4,6,8,10-Tetramethyltridecylverbindung (9), einer Kupplungsreaktion mit einer 1-Halogen-3-methylnonanverbindung der folgenden allgemeinen Formel (10): 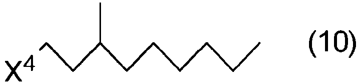
wobei X⁴ ein Halogenatom darstellt,
um das vorstehende 4,6,8,10,16-Pentamethyldocosan (11) zu bilden.

5. Eine Halogenalkylalkoxymethyletherverbindung der folgenden allgemeinen Formel (1'): 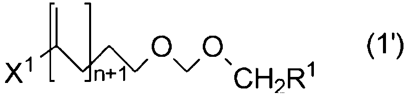 wobei X¹ ein Halogenatom darstellt, R¹ ein Wasserstoffatom, eine n-Alkylgruppe mit 1 bis 9 Kohlenstoffatomen oder eine Phenylgruppe darstellt und n eine ganze Zahl von 2 bis 7 darstellt.

## Revendications

1. Procédé de préparation d'un composé haloalkyl alcoxyméthyl éther de formule générale (1') suivante : 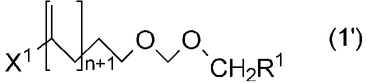
dans laquelle X¹ représente un atome d'halogène, R¹ représente un atome d'hydrogène, un groupe n-alkyle ayant 1 à 9 atomes de carbone, ou un groupe phényle, et n représente un nombre entier de 2 à 7, le procédé comprenant les étapes de
conversion d'un composé haloalkyl alcoxyméthyl éther de formule générale (1) suivante : 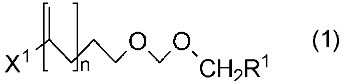
dans laquelle X¹, R¹, et n sont tels que définis ci-dessus, en un réactif nucléophile, composé *(2n+2)-*alcoxyméthoxyalkyle, de formule générale (2) suivante : 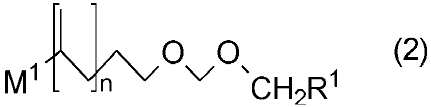
dans laquelle M¹ représente Li, MgZ¹, CuZ¹, ou CuLiZ¹, Z¹ représente un atome d'halogène ou Z², et Z² représente : 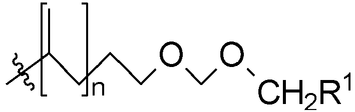
dans laquelle R¹ et n sont tels que définis ci-dessus, et la ligne ondulée indique que les structures au-delà de celle-ci sont abrégées,
soumission ensuite du réactif nucléophile, composé *(2n+2)-*alcoxyméthoxyalkyle (2), à une réaction d'addition nucléophile avec de l'oxyde de propylène de formule (3) suivante : 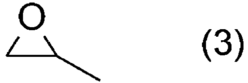
pour obtenir un composé hydroalkyl alcoxyméthyl éther de formule générale (4) suivante : 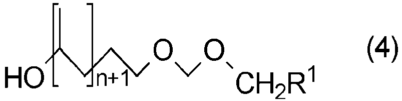
dans laquelle R¹ et n sont tels que définis ci-dessus ;
et soumission du composé hydroxyalkyl alcoxyméthyl éther (4) à une réaction d'halogénation pour obtenir le composé haloalkyl alcoxyméthyl éther (1') mentionné ci-dessus.

2. Procédé de préparation du composé haloalkyl alcoxyméthyl éther (1') selon la revendication 1, dans lequel n représente 2 dans les composés haloalkyl alcoxyméthyl éther (1) et (1'), le composé haloalkyl alcoxyméthyl éther(1') résultant étant le composé haloalkyl alcoxyméthyl éther (1' : *n+1=3).*

3. Procédé de préparation de 4,6,8,10,16-pentaméthyldocosane de formule générale (11) suivante : 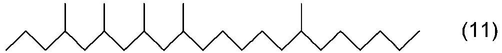
le procédé comprenant les étapes
du procédé de préparation du composé haloalkyl alcoxyméthyl éther (1' : *n+1* = 3) mentionné ci-dessus selon la revendication 2,
de conversion du composé haloalkyl alcoxyméthyl éther (1' : *n*+1 = 3) en un réactif nucléophile, composé 8-alcoxyméthoxy-1,3,5-triméthyloctyle, de formule générale (2 : *n* = 3) suivante : 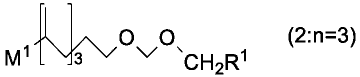
dans laquelle M¹ représente Li, MgZ¹, CuZ¹ ou CuLiZ¹, Z¹ représente un atome d'halogène ou un groupe 8-alcoxyméthoxy-1,3,5-triméthyloctyle, et R¹ est tel que défini ci-dessus,
de soumission ultérieure du réactif nucléophile, composé 8-alcoxyméthoxy-1,3,5-triméthyloctyle (2 : *n* = 3), à une réaction de couplage avec un composé 1-halo-2-méthylpentane de formule générale (5) suivante : 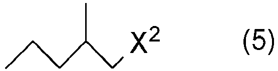
dans laquelle X² représente un atome d'halogène,
pour obtenir un composé 4,6,8,10-tétraméthyltridécyl alcoxyméthyl éther de formule générale (6) suivante : 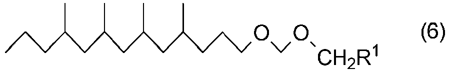
dans laquelle R¹ est tel que défini ci-dessus ;
de soumission du composé 4,6,8,10-tétraméthyltridécyl alcoxyméthyl éther (6) à une réaction de déalcoxyméthylation pour obtenir du 4,6,8,10-tétraméthyltridécanol de formule (7) suivante : 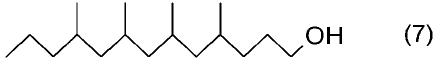
de soumission de 4,6,8,10-tétraméthyltridécanol (7) à une réaction d'halogénation pour obtenir un composé 1-halo-4,6,8,10-tétraméthyltridécane (8) de formule générale (8) suivante : 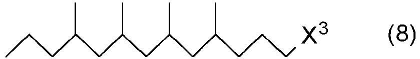
dans laquelle X³ représente un atome d'halogène,
de conversion du composé 1-halo-4,6,8,10-tétraméthyltridécane (8) en un réactif nucléophile, composé 4,6,8,10-tétraméthyltridécyle, de formule générale (9) suivante : 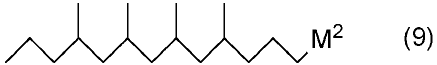
dans laquelle M² représente Li, MgZ¹, CuZ¹, ou CuLiZ¹, et Z¹ représente un atome d'halogène ou un groupe 4,6,8,10-tétraméthyltridécyle,
et de soumission ultérieure du réactif nucléophile, composé 4,6,8,10-tétraméthyltridécyle (9), à une réaction de couplage avec un composé 1-halo-3-méthylnonane de formule générale (10) suivante : 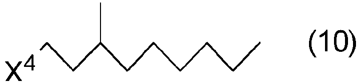
dans laquelle X⁴ représente un atome d'halogène,
pour obtenir le 4,6,8,10,16-pentaméthyldocosane (11) mentionné ci-dessus.

4. Procédé de préparation de 4,6,8,10,16-pentaméthyldocosane de formule générale (11) suivante : 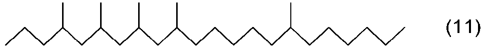
le procédé comprenant les étapes de
conversion d'un composé haloalkyl alcoxyméthyl éther de formule générale (1') suivante : 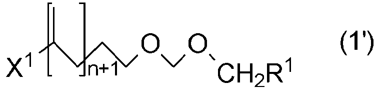
dans laquelle X¹ représente un atome d'halogène, R¹ représente un atome d'hydrogène, un groupe n-alkyle ayant 1 à 9 atomes de carbone, ou un groupe phényle, et n représente 2,
en un réactif nucléophile, composé 8-alcoxyméthoxy-1,3,5-triméthyloctyle, de formule générale suivante (2 : *n* = 3) : 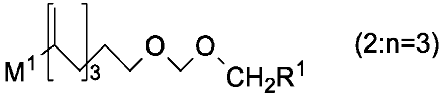
dans laquelle M¹ représente Li, MgZ¹, CuZ¹ ou CuLiZ¹, Z¹ représente un atome d'halogène ou un groupe 8-alcoxyméthoxy-1,3,5-triméthyloctyle, et R¹ est tel que défini ci-dessus,
soumission ultérieure du réactif nucléophile, composé 8-alcoxyméthoxy-1,3,5-triméthyloctyle (2 : *n* = 3), à une réaction de couplage avec un composé 1-halo-2-méthylpentane de formule générale (5) suivante : 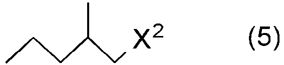
dans laquelle X² représente un atome d'halogène,
pour obtenir un composé 4,6,8,10-tétraméthyltridécyl alcoxyméthyl éther de formule générale (6) suivante : 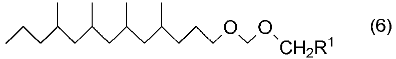
dans laquelle R¹ est tel que défini ci-dessus ;
soumission du composé 4,6,8,10-tétraméthyltridécyl alcoxyméthyl éther (6) à une réaction de déalcoxyméthylation pour obtenir du 4,6,8,10-tétraméthyltridécanol de formule (7) suivante : 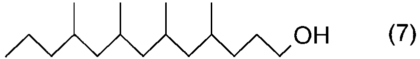
de soumission de 4,6,8,10-tétraméthyltridécanol (7) à une réaction d'halogénation pour obtenir un composé 1-halo-4,6,8,10-tétraméthyltridécane (8) de formule générale (8) suivante : 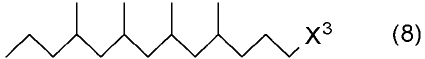
dans laquelle X³ représente un atome d'halogène ;
conversion du composé 1-halo-4,6,8,10-tétraméthyltridécane (8) mentionné ci-dessus en un réactif nucléophile, composé 4,6,8,10-tétraméthyltridécyle, de formule générale (9) suivante : 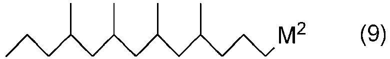
dans laquelle M² représente Li, MgZ¹, CuZ¹, ou CuLiZ¹, et Z¹ représente un atome d'halogène ou un groupe 4,6,8,10-tétraméthyltridécyle,
et soumission ultérieure du réactif nucléophile, composé 4,6,8,10-tétraméthyltridécyle (9), à une réaction de couplage avec un composé 1-halo-3-méthylnonane de formule générale (10) suivante : 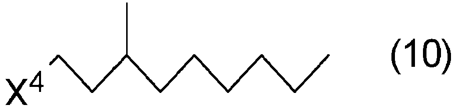
dans laquelle X⁴ représente un atome d'halogène,
pour former le 4,6,8,10,16-pentaméthyldocosane (11) mentionné ci-dessus.

5. Composé haloalkyl alcoxyméthyl éther de formule générale (1) suivante : 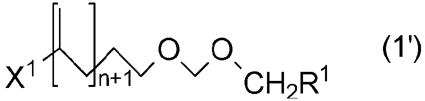 dans laquelle X¹ représente un atome d'halogène, R¹ représente un atome d'hydrogène, un groupe n-alkyle ayant 1 à 9 atomes de carbone, ou un groupe phényle, et n représente un nombre entier de 2 à 7.
